# EUROPEAN PATENT SPECIFICATION

(11) **EP 3 688 142 B1**
(45) Date of publication and mention of the grant of the patent: **09.04.2025**
(21) Application number: 18782605.2
(22) Date of filing: 17.09.2018
(51) Int. Cl.: C12N 5/0783, C12N 5/0784, A61K 35/17, A61K 39/00, C07K 14/47

(54) **METHODS OF ISOLATING T CELLS HAVING ANTIGENIC SPECIFICITY FOR A P53 CANCER-SPECIFIC MUTATION**
VERFAHREN ZUR ISOLIERUNG VON T-ZELLEN MIT ANTIGENSPEZIFIZITÄT FÜR EINE KREBSSPEZIFISCHE P53-MUTATION
PROCÉDÉS D'ISOLEMENT DE CELLULES T AYANT UNE SPÉCIFICITÉ ANTIGÉNIQUE POUR UNE MUTATION SPÉCIFIQUE DU CANCER P53

(30) Priority: 29.09.2017 US 201762565464 P
(43) Date of publication of application: 05.08.2020
(60) Divisional application: 24215129.8
(73) Proprietor: The United States of America, as represented by the Secretary, Department of Health and Human Services, Bethesda, MD 20892-7788 (US)
(72) Inventor: DENIGER, Drew C., Kensington, Maryland 20895 (US); ROSENBERG, Steven A., Potomac, Maryland 20854 (US); MALEKZADEH, Parisa, Washington, District of Columbia (US)
(74) Representative: Maiwald GmbH
(86) International application number: PCT/US2018/051280
(87) International publication number: WO 2019/067242

(56) References cited:
- KATERINA SHAMALOV ET AL: "The mutational status of p53 can influence its recognition by human T-cells", ONCOIMMUNOLOGY, vol. 6, no. 4, 31 January 2017 (2017-01-31), pages e1285990, XP055523658, DOI: 10.1080/2162402X.2017.1285990
- INGE MARIE SVANE ET AL: "Vaccination with p53 peptide-pulsed dendritic cells is associated with disease stabilization in patients with p53 expressing advanced breast cancer; monitoring of serum YKL-40 and IL-6 as response biomarkers", CANCER IMMUNOLOGY, IMMUNOTHERAPY, SPRINGER, BERLIN, DE, vol. 56, no. 9, 7 February 2007 (2007-02-07), pages 1485 - 1499, XP019514194, ISSN: 1432-0851, DOI: 10.1007/S00262-007-0293-4
- ALENA GROS ET AL: "Prospective identification of neoantigen-specific lymphocytes in the peripheral blood of melanoma patients", NATURE MEDICINE, vol. 22, no. 4, 22 February 2016 (2016-02-22), New York, pages 433 - 438, XP055460267, ISSN: 1078-8956, DOI: 10.1038/nm.4051
- CYRILLE J. COHEN ET AL: "Isolation of neoantigen-specific T cells from tumor and peripheral lymphocytes", JOURNAL OF CLINICAL INVESTIGATION, vol. 125, no. 10, 21 September 2015 (2015-09-21), pages 3981 - 3991, XP055365967, ISSN: 0021-9738, DOI: 10.1172/JCI82416
- YONG-CHEN LU ET AL: "Cancer immunotherapy targeting neoantigens", SEMINARS IN IMMUNOLOGY., vol. 28, no. 1, 30 November 2015 (2015-11-30), US, pages 22 - 27, XP055364718, ISSN: 1044-5323, DOI: 10.1016/j.smim.2015.11.002
- DREW C. DENIGER ET AL: "T-cell Responses to TP53 "Hotspot" Mutations and Unique Neoantigens Expressed by Human Ovarian Cancers", CLINICAL CANCER RESEARCH, 31 May 2018 (2018-05-31), US, XP055523675, ISSN: 1078-0432, DOI: 10.1158/1078-0432.CCR-18-0573

## Description

### CROSS-REFERENCE TO RELATED APPLICATION

This patent application claims the benefit of U.S. Provisional Patent Application No. 62/565,464, filed September 29, 2017.

### STATEMENT REGARDING FEDERALLY SPONSORED RESEARCH AND

### DEVELOPMENT

This invention was made with Government support under project number ZIABC010984 by the National Institutes of Health, National Cancer Institute. The Government has certain rights in the invention.

### MATERIAL SUBMITTED ELECTRONICALLY

Submitted herewith is a computer-readable nucleotide/amino acid sequence listing submitted concurrently herewith and identified as follows: One 687,616 Byte ASCII (Text) file named "740175_ST25.txt," dated September 14, 2018.

### BACKGROUND OF THE INVENTION

Adoptive cell therapy (ACT) can produce positive clinical responses in some cancer patients. Nevertheless, obstacles to the successful use of ACT for the widespread treatment of cancer and other diseases remain. For example, T cells that specifically recognize cancer antigens may be difficult to identify and/or isolate from a patient. Accordingly, there is a need for improved methods of obtaining cancer-reactive T cells.

### BRIEF SUMMARY OF THE INVENTION

An embodiment of the invention provides a method of isolating T cells having antigenic specificity for a mutated human p53 amino acid sequence encoded by a cancer-specific p53 mutation, the method comprising: inducing autologous antigen presenting cells (APCs) of a patient to present at least one mutated human p53 amino acid sequence , wherein inducing APCs of the patient to present the at least one mutated human p53 amino acid sequence comprises (i) or (ii): (i) pulsing the APCs with a peptide comprising the mutated human p53 amino acid sequence or a pool of peptides, each peptide in the pool comprising a different mutated human p53 amino acid sequence; or (ii) introducing a nucleotide sequence encoding the mutated human p53 amino acid sequence into the APCs; co-culturing autologous T cells of the patient with the autologous APCs that present the mutated human p53 amino acid sequence; and selecting the autologous T cells that (a) were co-cultured with the autologous APCs that present the mutated human p53 amino acid sequence and (b) have antigenic specificity for the mutated human p53 amino acid sequence presented in the context of a major histocompatibility complex (MHC) molecule expressed by the patient to provide isolated T cells having antigenic specificity for the mutated human p53 amino acid sequence encoded by the cancer-specific p53 mutation.

Another embodiment of the invention provides a method of isolating a T cell receptor (TCR), or an antigen-binding portion thereof, having antigenic specificity for a mutated human p53 amino acid sequence encoded by a cancer-specific p53 mutation, the method comprising: isolating T cells having antigenic specificity for a mutated human p53 amino acid sequence encoded by a cancer-specific p53 mutation according to any of the methods described herein; and isolating a nucleotide sequence that encodes the TCR, or the antigen-binding portion thereof, from the selected autologous T cells, wherein the TCR, or the antigen-binding portion thereof, has antigenic specificity for the mutated human p53 amino acid sequence encoded by the cancer-specific p53 mutation.

Still another embodiment of the invention provides a method of preparing a population of cells that express a TCR, or an antigen-binding portion thereof, having antigenic specificity for a mutated human p53 amino acid sequence encoded by a cancer-specific p53 mutation, the method comprising: isolating a TCR, or an antigen-binding portion thereof, according to any of the methods described herein, and introducing the nucleotide sequence encoding the isolated TCR, or the antigen-binding portion thereof, into peripheral blood mononuclear cells (PBMC) to obtain cells that express the TCR, or the antigen-binding portion thereof.

Another embodiment of the invention provides method of preparing a population of T cells that have antigenic specificity for a mutated human p53 amino acid sequence encoded by a cancer-specific p53 mutation, the method comprising: isolating T cells according to any of the methods described herein, and expanding the numbers of selected autologous T cells to obtain a population of T cells that have antigenic specificity for the mutated human p53 amino acid sequence encoded by the cancer-specific p53 mutation.

### BRIEF DESCRIPTION OF THE SEVERAL VIEWS OF THE DRAWINGS

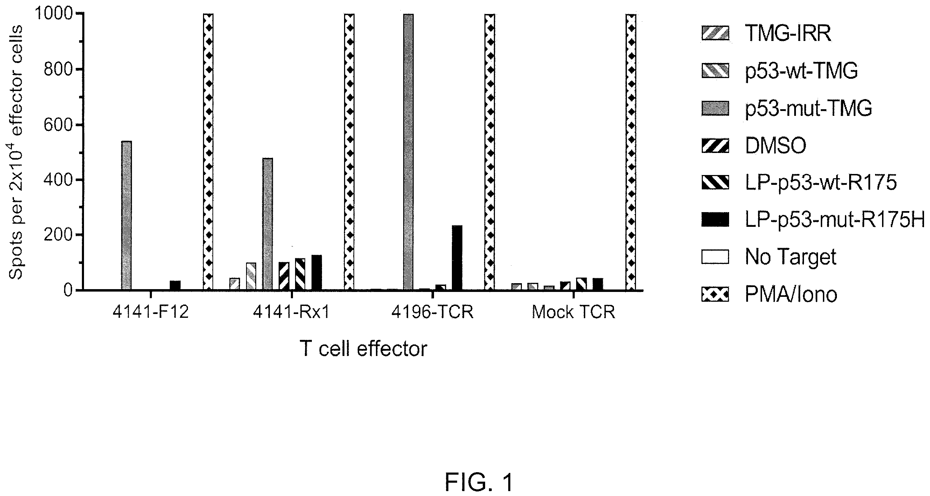

Figure 1 is a graph showing the number of IFN-γ positive spots per 2x10⁴ effector cells measured following co-culture of effector cells and target cells. The effector cells were TIL fragment F12, infusion bag TIL (Rx1) from patient 4141, p53-R175H-specific TCR-transduced cells, or mock transduced T cells from patient 4196. The target cells were HLA-A*02:01 APCs (autologous to patient 4141) that were either (1) electroporated with TMGs composed of irrelevant (IRR; left hatched gray bars), WT p53 (p53wt-TMG; right hatched gray bars) or mutated p53 (p53-mut-TMG; gray bars) sequences or (2) pulsed with peptide vehicle (DMSO; left hatched black bars) or purified (>95% by HPLC) 25 amino acid peptides composed of WT p53-R175 sequence (LP-p53-wt-R175; right hatched black bars) or mutated p53-R175H (LP-p53-mut-R175H; black bars) sequence. T cells only (no target; open bars) was negative control and Phorbol 12-myristate 13-acetate (PMA) and Ionomycin (Iono) was positive control (lattice bars).

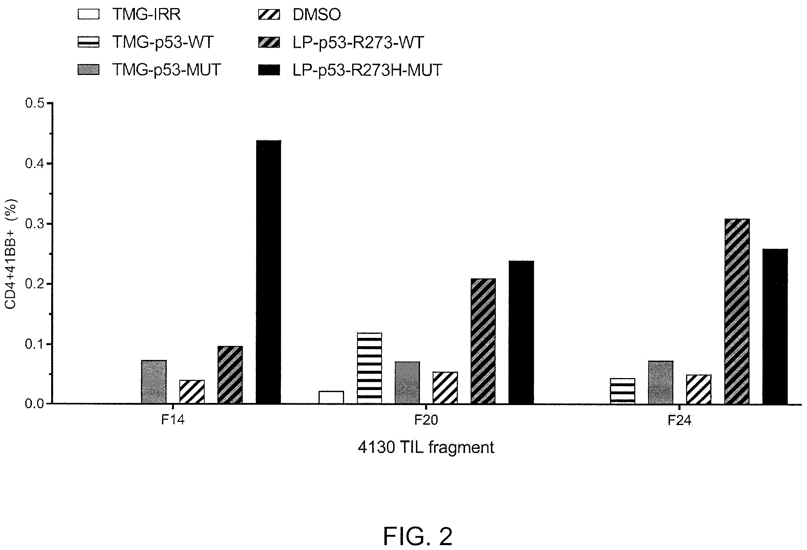

Figure 2 is a graph showing the percentage of 4-1BB+ cells (% of CD4+) detected following co-culture of TIL fragments (F14, F20 or F24) from patient 4130 with autologous APCs (1) electroporated with TMGs composed of irrelevant (IRR; open bars), WT p53 (p53-WT; horizontal black bars) or mutated p53 (p53-MUT; gray bars) sequences or (2) pulsed with peptide vehicle (DMSO; left hatched black bars) or purified (>95% by HPLC) 25 amino acid peptides composed of WT p53-R273 sequence (LP-p53-R273-WT; left hatched gray bars) or mutated p53-R273H (LP-p53-R273H-MUT; black bars) sequence.

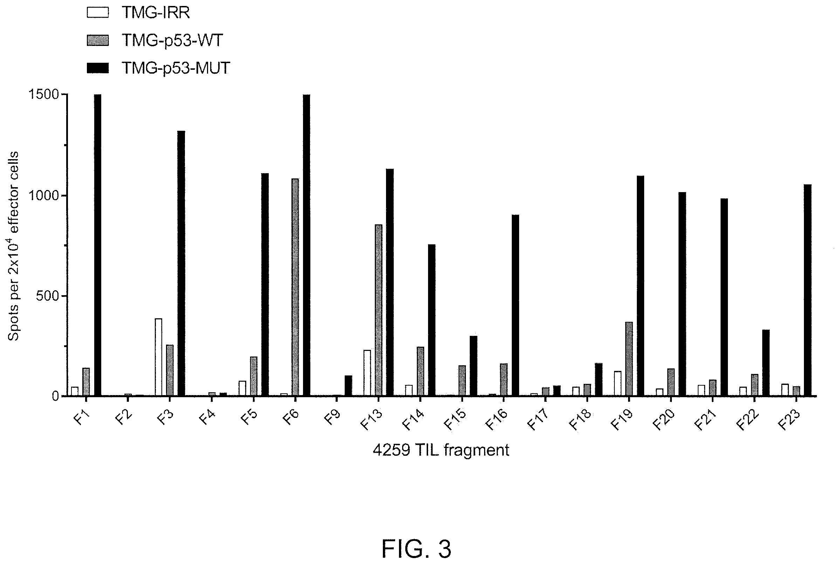

Figure 3 is a graph showing the number of IFN-γ positive spots per 2x10⁴ effector cells measured following co-culture of TIL fragments (F1-6, F9, F13-23, n=18) from patient 4259 with autologous APCs electroporated with TMGs composed of irrelevant (IRR; open bars), WT p53 (p53-WT; gray bars) or mutated p53 (p53-MUT; black bars) sequences.

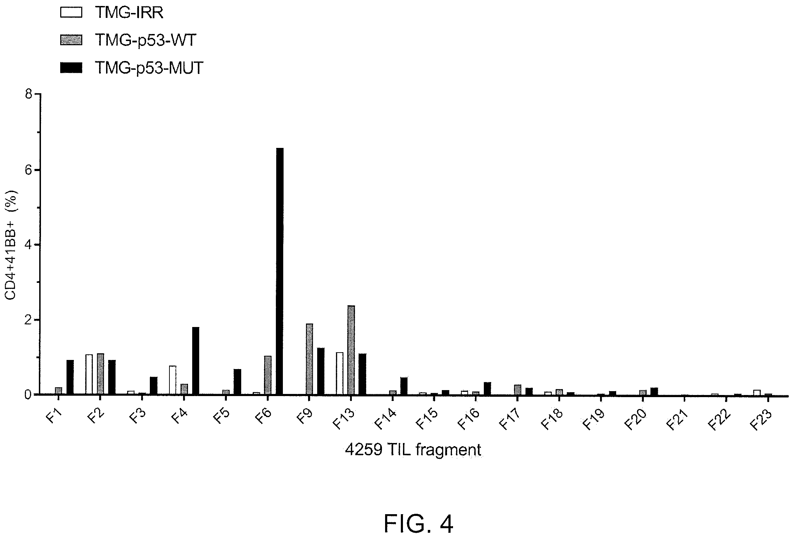

Figure 4 is a graph showing the percentage of 4-1BB+ cells (% of CD4+) detected following co-culture of TIL fragments (F1-F6, F9, F13-F23, n=18) from patient 4259 with autologous APCs electroporated with TMGs composed of irrelevant (IRR; open bars), WT p53 (p53-WT; gray bars) or mutated p53 (p53-MUT; black bars) sequences.

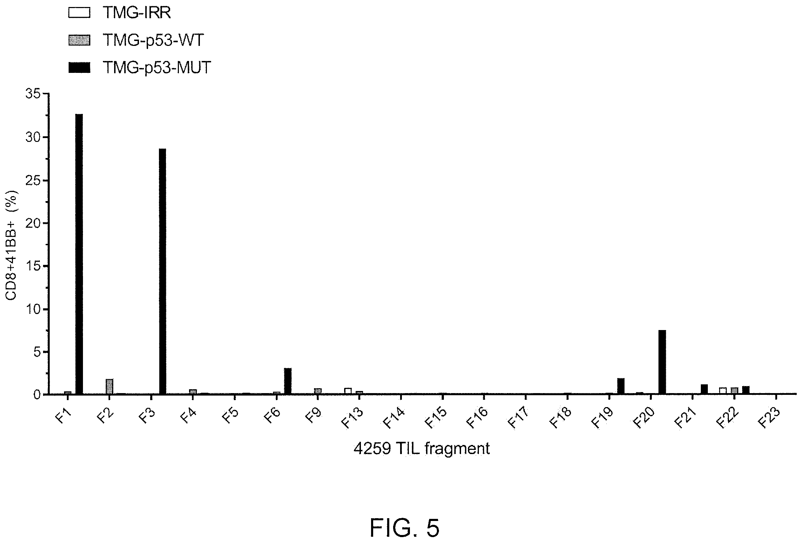

Figure 5 is a graph showing the percentage of 4-1BB+ cells (% of CD8+) detected following co-culture of TIL fragments (F1-F6, F9, F13-F23, n=18) from patient 4259 with autologous APCs electroporated with TMGs composed of irrelevant (IRR; open bars), WT p53 (p53-WT; gray bars) or mutated p53 (p53-MUT; black bars) sequences.

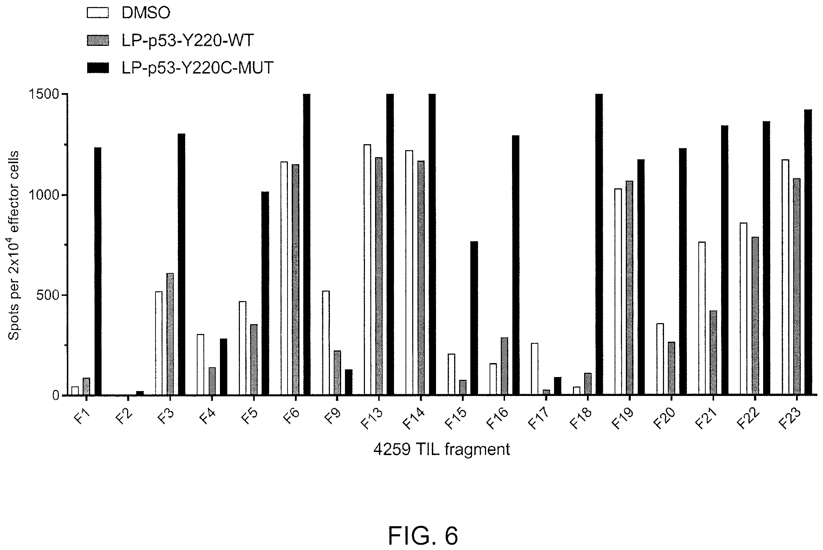

Figure 6 is a graph showing the number of IFN-γ positive spots per 2x10⁴ effector cells measured following co-culture of TIL fragments (F1-F6, F9, F13-23, n=18) from patient 4259 with autologous APCs pulsed with peptide vehicle (DMSO; open bars) or purified (>95% by HPLC) 25 amino acid peptides composed of WT p53-Y220 sequence (LP-p53-Y220-WT; gray bars) or mutated p53-Y220C (LP-p53-Y220C-MUT; black bars) sequence.

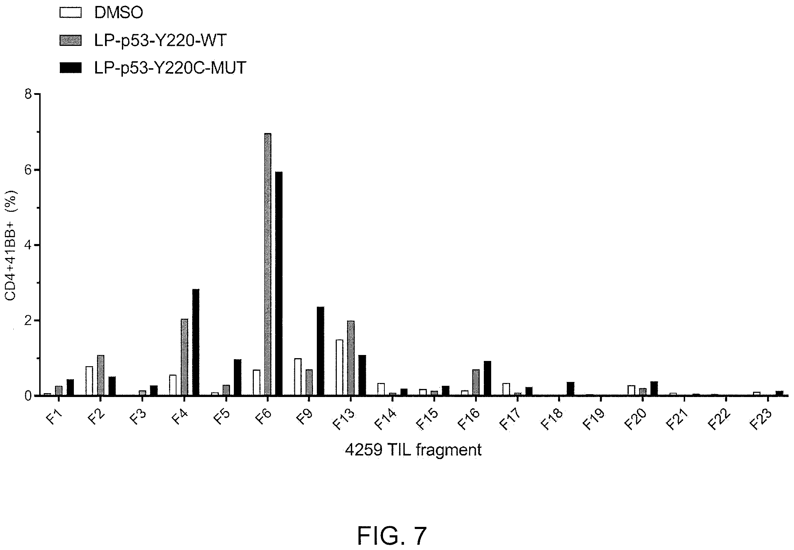

Figure 7 is a graph showing the percentage of 4-1BB+ cells (% of CD4+) detected following co-culture of TIL fragments (F1-F6, F9, F13-F23, n=18) from patient 4259 with autologous APCs pulsed with peptide vehicle (DMSO; open bars) or purified (>95% by HPLC) 25 amino acid peptides composed of WT p53-Y220 sequence (LP-p53-Y220-WT; gray bars) or mutated p53-Y220C (LP-p53-Y220C-MUT; black bars) sequence.

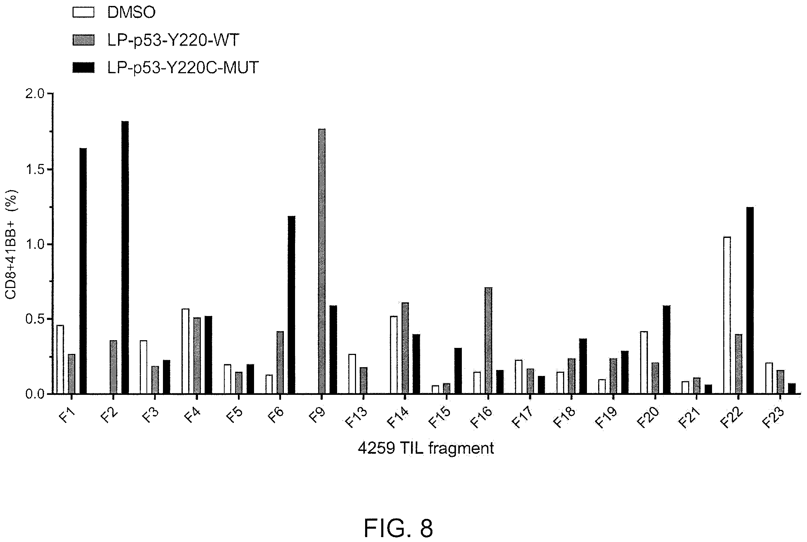

Figure 8 is a graph showing the percentage of 4-1BB+ cells (% of CD8+) detected following co-culture of TIL fragments (F1-F6, F9, F13-F23, n=18) from patient 4259 with autologous APCs pulsed with peptide vehicle (DMSO; open bars) or purified (>95% by HPLC) 25 amino acid peptides composed of WT p53-Y220 sequence (LP-p53-Y220-WT; gray bars) or mutated p53-Y220C (LP-p53-Y220C-MUT; black bars) sequence.

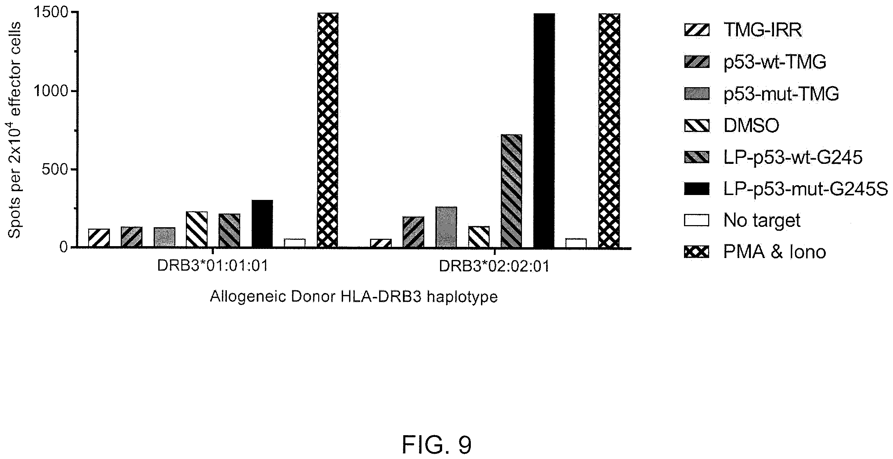

Figure 9 is a graph showing the number of IFN-γ positive spots per 2x10⁴ effector cells measured following co-culture of TIL from patient 4127 with allogeneic (DRB3*01:01:01 or DRB3*02:02:01) APCs which were (1) electroporated with TMGs composed of irrelevant (IRR; left hatched open bars) WT p53 (p53-WT; left hatched gray bars) or mutated p53 (p53-MUT; gray bars) sequences or (2) pulsed with peptide vehicle (DMSO; right hatched open bars) or purified (>95% by HPLC) 25 amino acid peptides composed of WT p53-G245 sequence (LP-p53-wt-G245; right hatched gray bars) or mutated p53-G245S (LP-p53-mut-G245S; black bars) sequence.

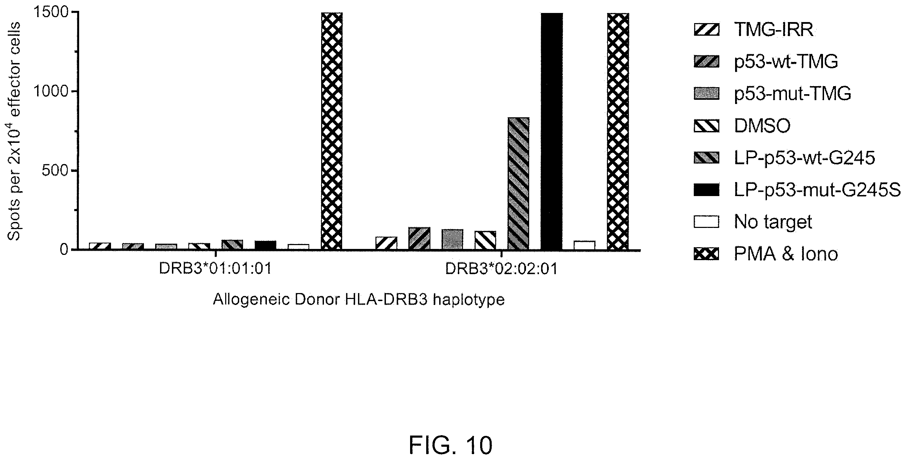

Figure 10 is a graph showing the number of IFN-γ positive spots per 2x10⁴ effector cells measured following co-culture of T cells expressing the 4127-TCR1 with allogeneic (DRB3*01:01:01 or DRB3*02:02:01) APCs which were (1) electroporated with TMGs composed of irrelevant (IRR; left hatched open bars), WT p53 (p53-WT; left hatched gray bars) or mutated p53 (p53-MUT; gray bars) sequences or (2) pulsed with peptide vehicle (DMSO; right hatched open bars) or purified (>95% by HPLC) 25 amino acid peptides composed of WT p53-G245 sequence (LP-p53-wt-G245; right hatched gray bars) or mutated p53-G245S (LP-p53-mut-G245S; black bars) sequence.

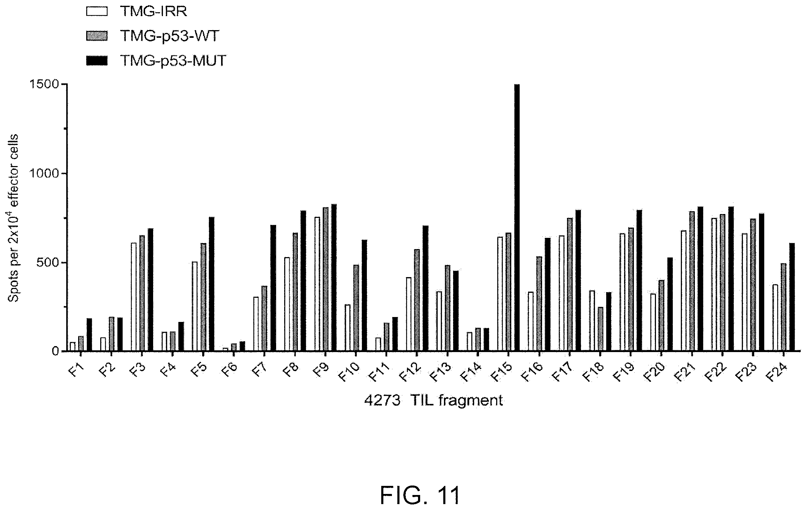

Figure 11 is a graph showing the number of IFN-γ-positive spots per 2x10⁴ effector cells measured following co-culture of TIL fragments (F1-F24, n=24) from patient 4273 with autologous APCs electroporated with TMG (TMG) composed of irrelevant (IRR; open bars) WT p53 (p53-WT; gray bars) or mutated p53 (p53-MUT; black bars) sequence.

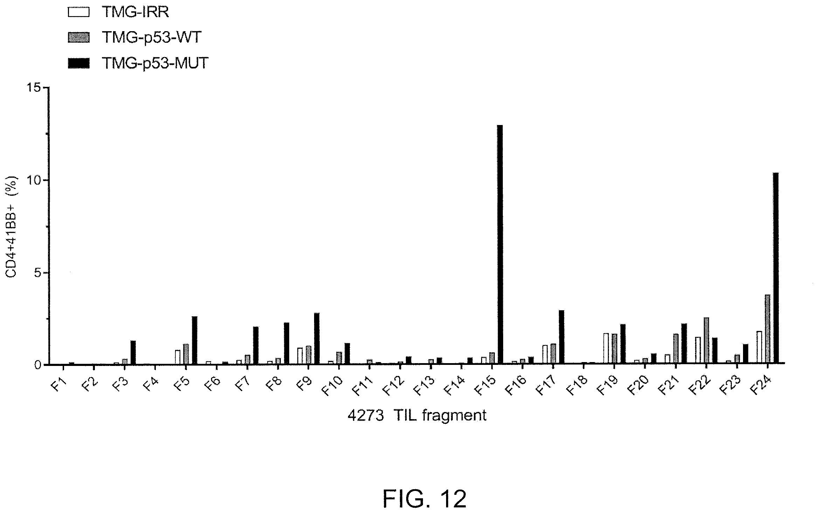

Figure 12 is a graph showing the percentage of 4-1BB+ (% of CD4+) cells detected following co-culture of patient 4273 TIL fragments F1-F24 (n=24) with autologous APCs electroporated with TMG composed of irrelevant (IRR; open bars) WT p53 (p53-WT; gray bars) or mutated p53 (p53-MUT; black bars) sequence.

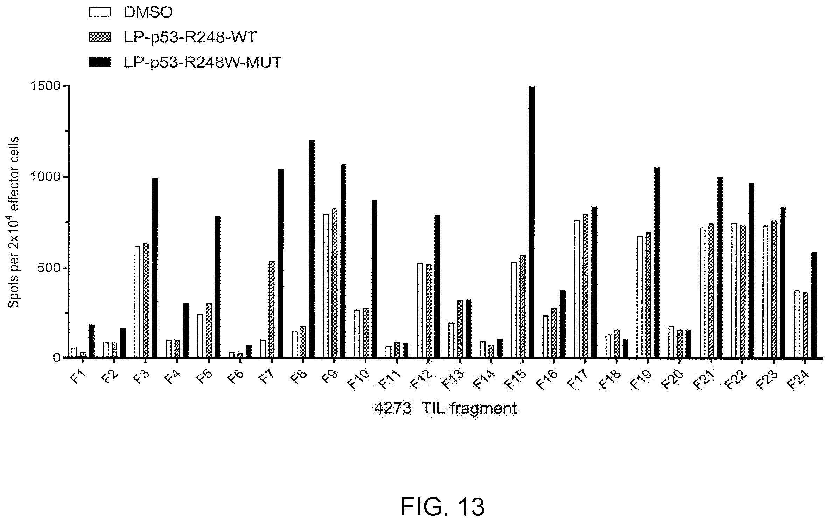

Figure 13 is a graph showing the number of IFN-γ-positive spots per 2x10⁴ effector cells measured following co-culture of TIL fragments (F1-F24, n=24) from patient 4273 with autologous APCs pulsed with peptide vehicle (DMSO; open bars) or purified (>95% by HPLC) 25-amino acid peptides composed of WT p53-R248 sequence (LP-p53-R248-WT; gray bars) or mutated p53-R248W (LP-p53-R248W-MUT; black bars) sequence.

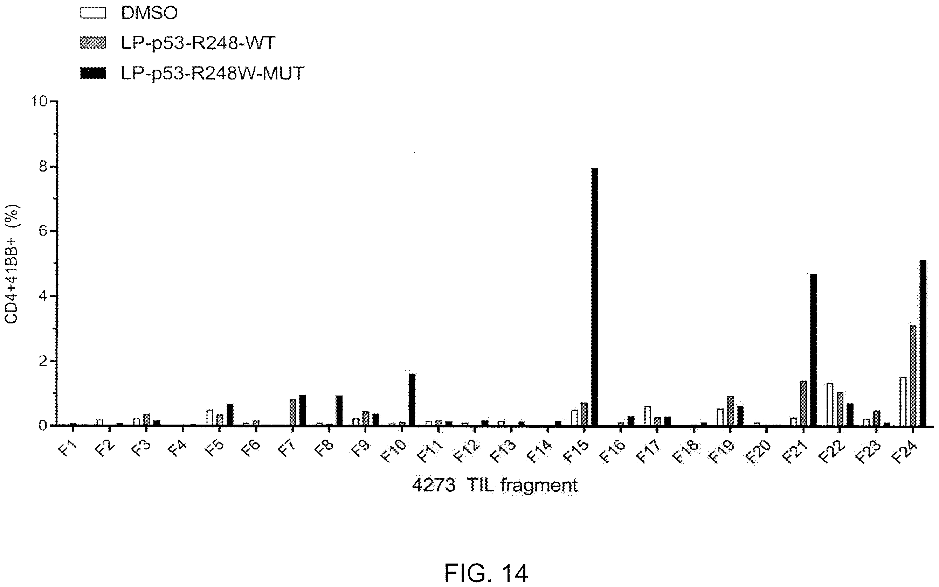

Figure 14 is a graph showing the percentage of 4-1BB+ (% of CD4+) cells detected following co-culture of TIL fragments (F1-F24, n=24) from patient 4273 with autologous APCs pulsed with peptide vehicle (DMSO; open bars) or purified (>95% by HPLC) 25-amino acid peptides composed of WT p53-R248 sequence (LP-p53-R248-WT; gray bars) or mutated p53-R248W (LP-p53-R248W-MUT; black bars) sequence.

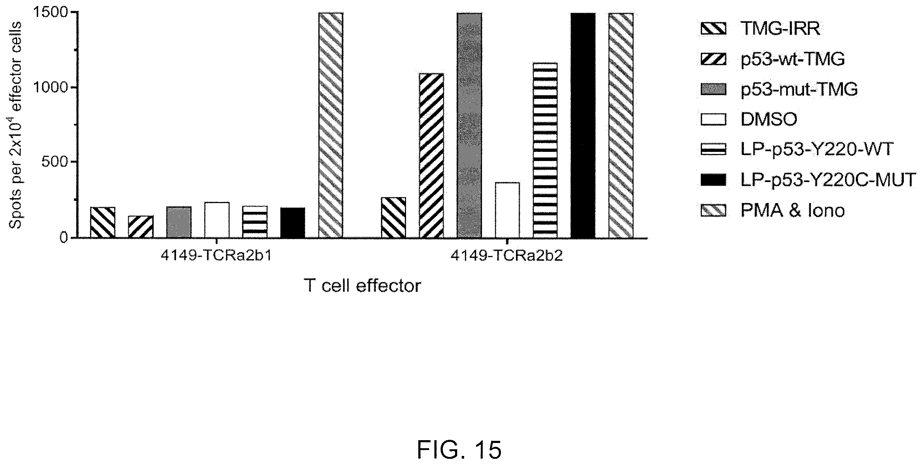

Figure 15 is a graph showing the number of IFN-γ-positive spots per 2x10⁴ effector cells measured following co-culture of autologous PBL from patient 4149 (transposed with 4149-TCRa2b1 or 4149-TCRa2b2) with autologous APCs which were (1) electroporated with TMG composed of irrelevant (IRR; right hatched black bars), WT p53 (p53-wt-TMG; left hatched black bars) or mutated p53 (p53-mut-TMG; gray bars) sequence or (2) pulsed with peptide vehicle (DMSO; open bars) or purified (>95% by HPLC) 25-amino acid peptides composed of WT p53-Y220 sequence (LP-p53-Y220-WT; horizontal hatched black bars) or mutated p53-Y220C (LP-p53-Y220C-MUT; black bars) sequence. Phorbol 12-myristate 13-acetate (PMA) and Ionomycin (Iono) was positive control (gray bars).

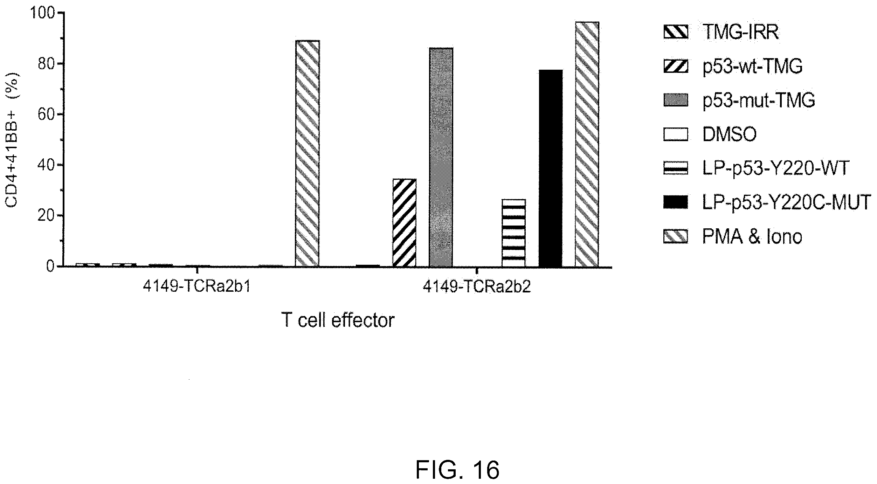

Figure 16 is a graph showing the percentage of 4-1BB+ (% of CD4+) cells detected following co-culture of autologous PBL from patient 4149 that were transposed with a TCR (4149-TCRa2b1 or 4149-TCRa2b2) with autologous APCs which were (1) electroporated with TMG composed of irrelevant (IRR; right hatched black bars), WT p53 (p53-wt-TMG; left hatched black bars), or mutated p53 (p53-mut-TMG; gray bars) sequence or (2) pulsed with peptide vehicle (DMSO; open bars) or purified (>95% by HPLC) 25-amino acid peptides composed of WT p53-Y220 sequence (LP-p53-Y220-WT; horizontal hatched black bars) or mutated p53-Y220C (LP-p53-Y220C-MUT; black bars) sequence. PMA and Iono was positive control (gray bars).

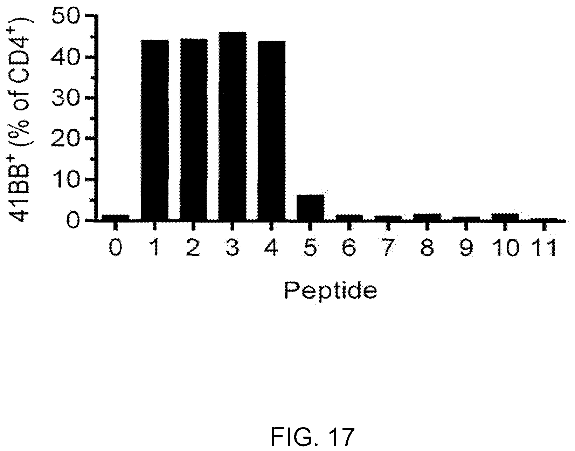

Figure 17 is a graph showing the percentage of 4-1BB+ cells (% of CD4+ T cells) detected following co-culture of TIL from patient 4149 with autologous DCs pulsed with one of the peptides of Table 10.

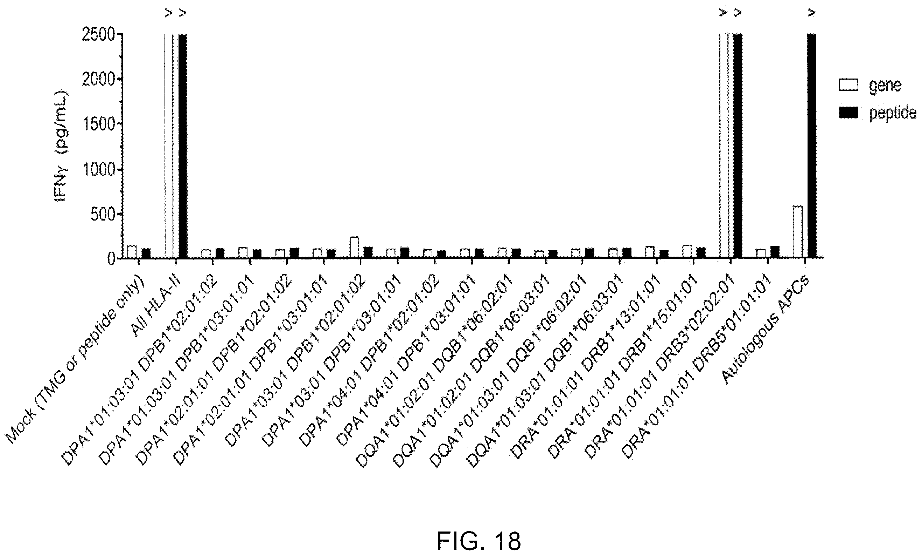

Figure 18 is a graph showing IFN-γ secretion (pg/mL) following co-culture of 4149-TCRa2b2 transposed T cells with Cos7 cells co-transfected with individual HLA alleles +/- TMGs. Cells not transfected with TMG were pulsed with p53Y220C 15-mer peptide. Pulsed target cells are indicated by shaded bars. Target cells transfected with TMG are indicated by unshaded bars.

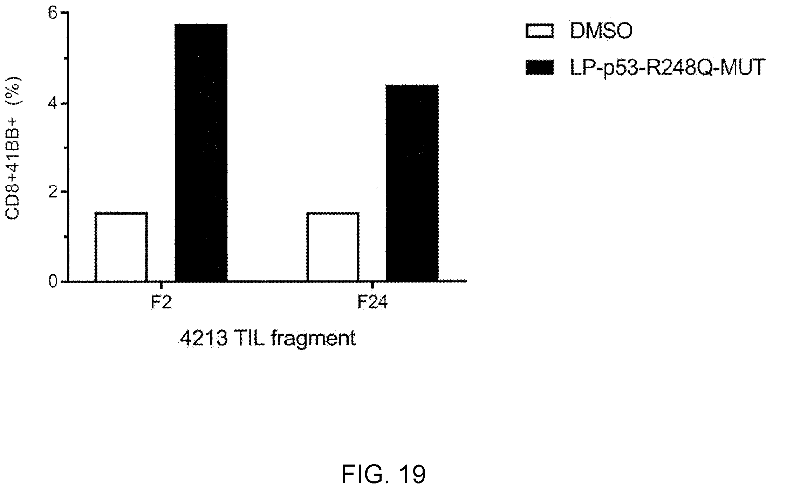

Figure 19 is a graph showing the percentage of 4-1BB+ (% of CD8+) cells detected following co-culture of TIL fragments (F2 and F24) from patient 4213 with autologous APCs pulsed with peptide vehicle (DMSO; open bars) or purified (>95% by HPLC) 25-amino acid peptides composed of the mutated p53-R248Q (LP-p53-R248Q-MUT; black bars) sequence.

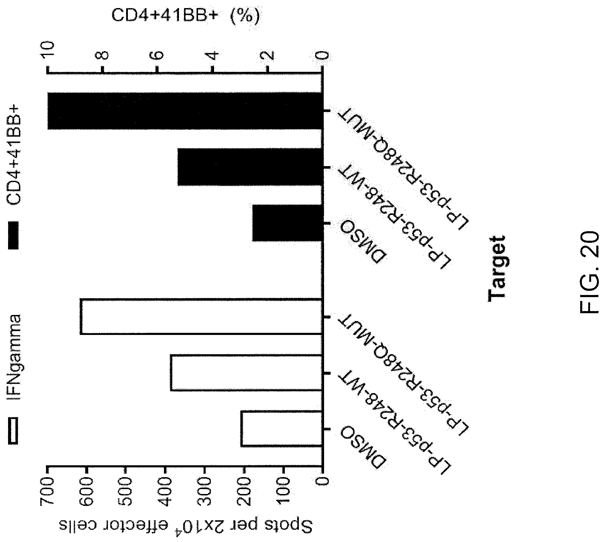

Figure 20 is a graph showing the number of IFN-γ-positive spots per 2x10⁴ effector cells measured following co-culture of CD4+ T cells from patient 4213 with autologous APCs pulsed with peptide vehicle (DMSO; open bars) or purified (>95% by HPLC) 25-amino acid peptides composed of the mutated p53-R248Q (LP-p53-R248Q-MUT; black bars) sequence. Secretion of IFN-γ is shown by open bars. Expression of 4-1BB is shown by closed bars.

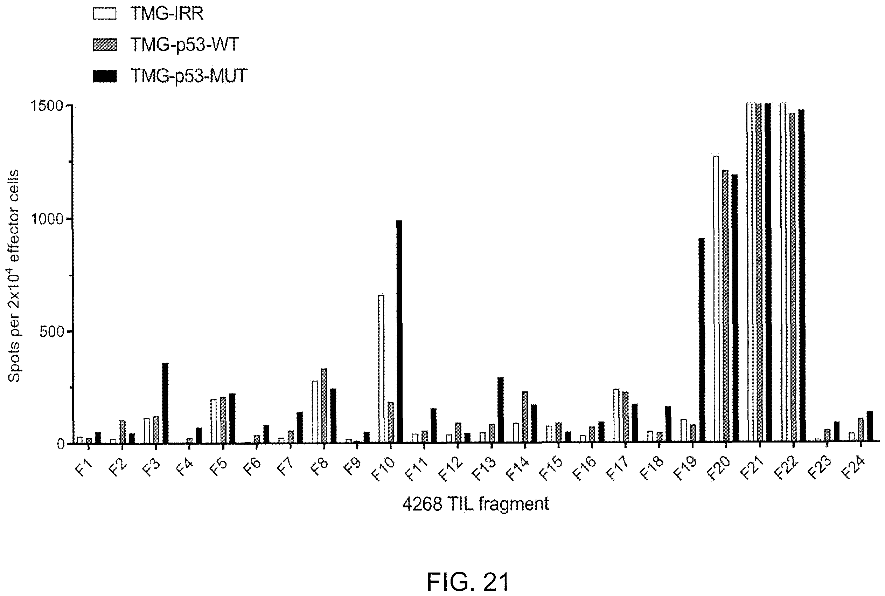

Figure 21 is a graph showing the number of IFN-γ-positive spots per 2x10⁴ effector cells measured following co-culture of TIL fragments (F1-F24, n=24) from patient 4268 with autologous APCs electroporated with TMG composed of irrelevant (IRR; open bars), WT p53 (p53-WT; gray bars), or mutated p53 (p53-MUT; black bars) sequence.

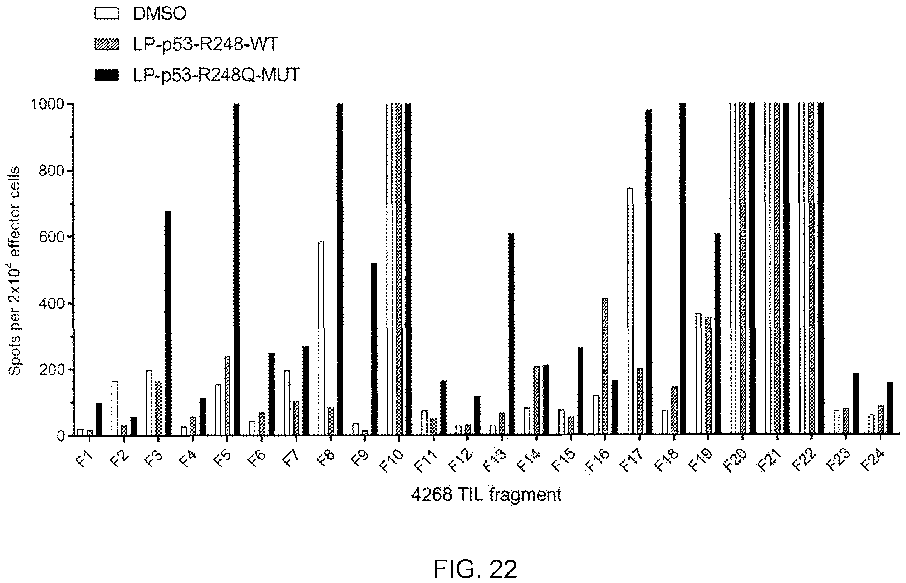

Figure 22 is a graph showing the number of IFN-γ-positive spots per 2x10⁴ effector cells measured following co-culture of TIL fragments (F1-F24, n=24) from patient 4268 with autologous APCs pulsed with peptide vehicle (DMSO; open bars) or purified (>95% by HPLC) 25-amino acid peptides composed of WT p53-R248 sequence (LP-p53-R248-WT; gray bars) or mutated p53-R248Q (LP-p53-R248Q-MUT; black bars) sequence.

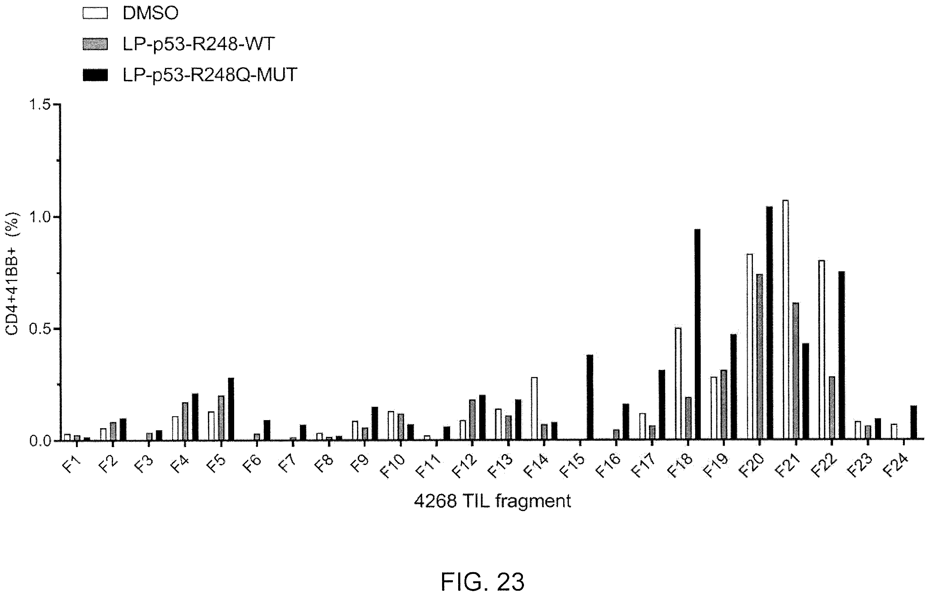

Figure 23 is graph showing the percentage of 4-1BB+ cells (% of CD4+) detected following co-culture of TIL fragments (F1-F24, n=24) from patient 4268 with autologous APCs pulsed with peptide vehicle (DMSO; open bars) or purified (>95% by HPLC) 25-amino acid peptides composed of WT p53-R248 sequence (LP-p53-R248-WT; gray bars) or mutated p53-R248Q (LP-p53-R248Q-MUT; black bars) sequence.

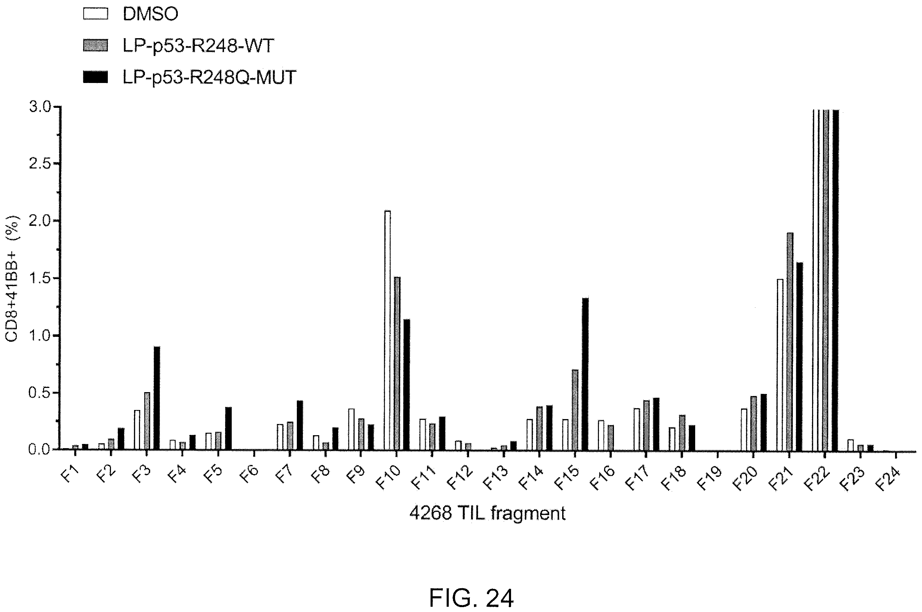

Figure 24 is a graph showing the percentage of 4-1BB+ cells (% of CD8+) detected following co-culture of TIL fragments (F1-F24, n=24) from patient 4268 with autologous APCs pulsed with peptide vehicle (DMSO; open bars) or purified (>95% by HPLC) 25-amino acid peptides composed of WT p53-R248 sequence (LP-p53-R248-WT; gray bars) or mutated p53-R248Q (LP-p53-R248Q-MUT; black bars) sequence.

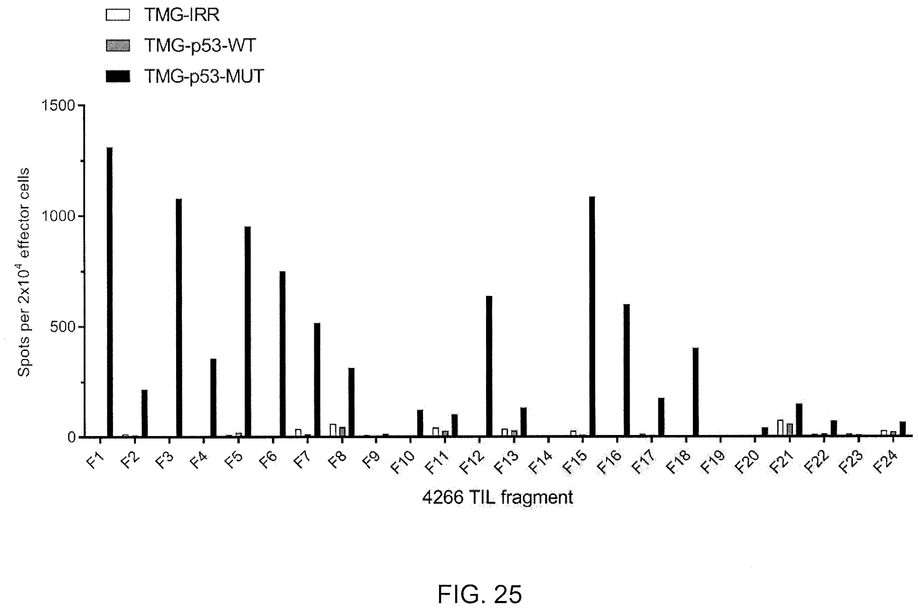

Figure 25 is a graph showing the number of IFN-γ-positive spots per 2x10⁴ effector cells measured following co-culture of TIL fragments (F1-F24, n=24) from patient 4266 with autologous APCs electroporated with TMG composed of irrelevant (IRR; open bars), WT p53 (p53-WT; gray bars), or mutated p53 (p53-MUT; black bars) sequence.

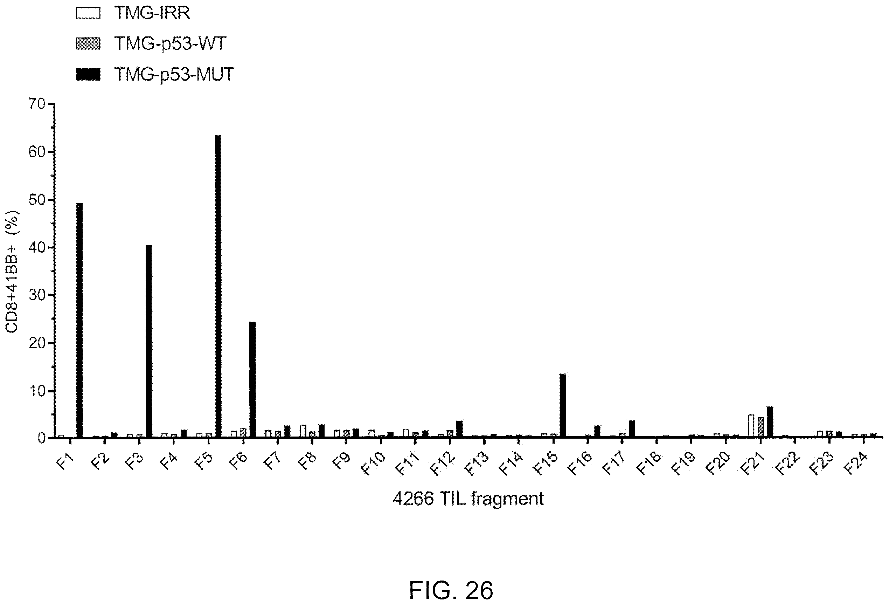

Figure 26 is a graph showing the percentage of 4-1BB+ cells (% of CD8+) detected following co-culture of TIL fragments (F1-F24, n=24) from patient 4266 with autologous APCs electroporated with TMG composed of irrelevant (IRR; open bars), WT p53 (p53-WT; gray bars), or mutated p53 (p53-MUT; black bars) sequence.

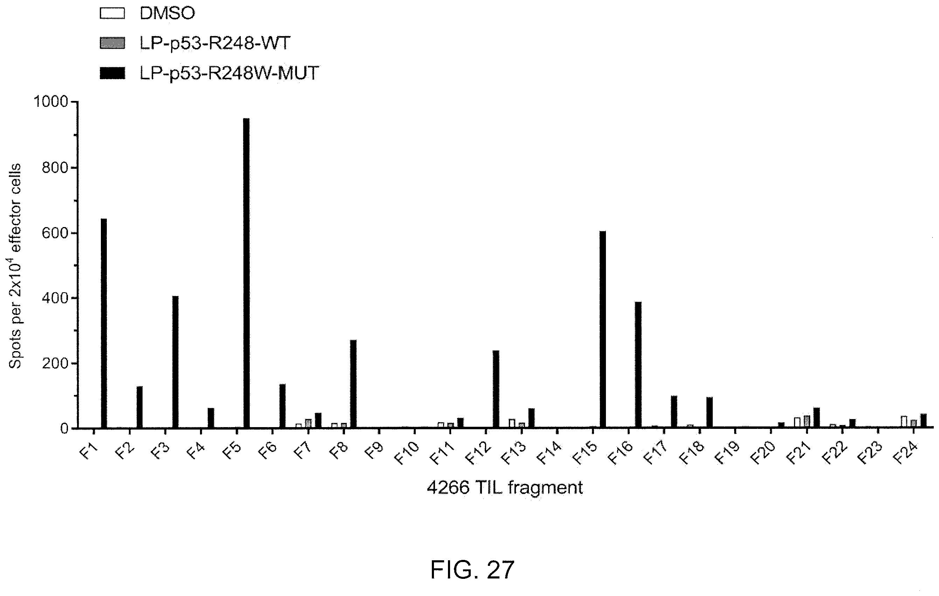

Figure 27 is a graph showing the number of IFN-γ-positive spots per 2x10⁴ effector cells measured following co-culture of TIL fragments (F1-F24, n=24) from patient 4266 with autologous APCs pulsed with peptide vehicle (DMSO; open bars) or purified (>95% by HPLC) 25-amino acid peptides composed of WT p53-R248 sequence (LP-p53-R248-WT; gray bars) or mutated p53-R248W (LP-p53-R248W-MUT; black bars) sequence.

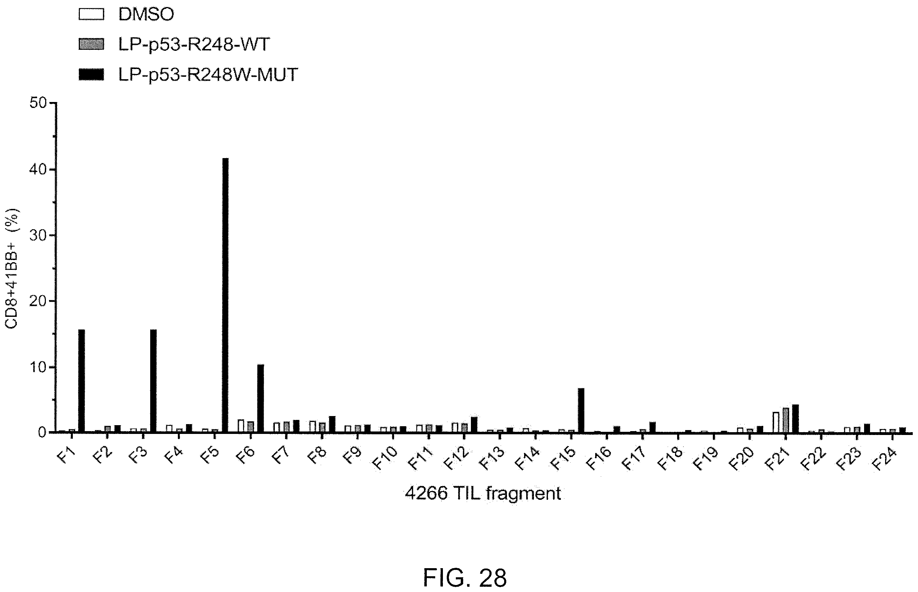

Figure 28 is a graph showing the percentage of 4-1BB+ cells (% of CD8+) detected following co-culture of TIL fragments (F1-F24, n=24) from patient 4266 with autologous APCs pulsed with peptide vehicle (DMSO; open bars) or purified (>95% by HPLC) 25-amino acid peptides composed of WT p53-R248 sequence (LP-p53-R248-WT; gray bars) or mutated p53-R248W (LP-p53-R248W-MUT; black bars) sequence.

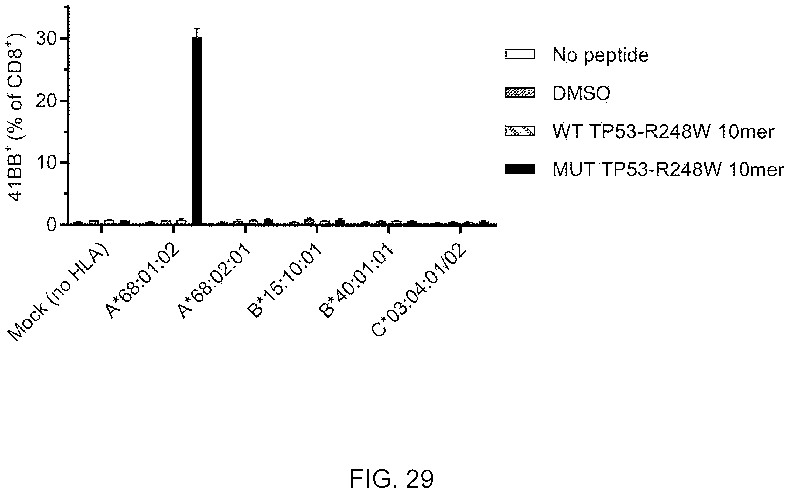

Figure 29 is a graph showing the percentage of 4-1BB+ cells (% of CD8+) detected following co-culture of TIL from patient 4266 with Cos7 cells which were co-transfected with individual HLA alleles from patient 4266 and pulsed with no peptide (open bars), DMSO (peptide vehicle; gray bars), WT p53-R248 peptide (gray hatched bars) or mutated p53-R248W peptide (black bars). Data are mean ± SEM (n=3).

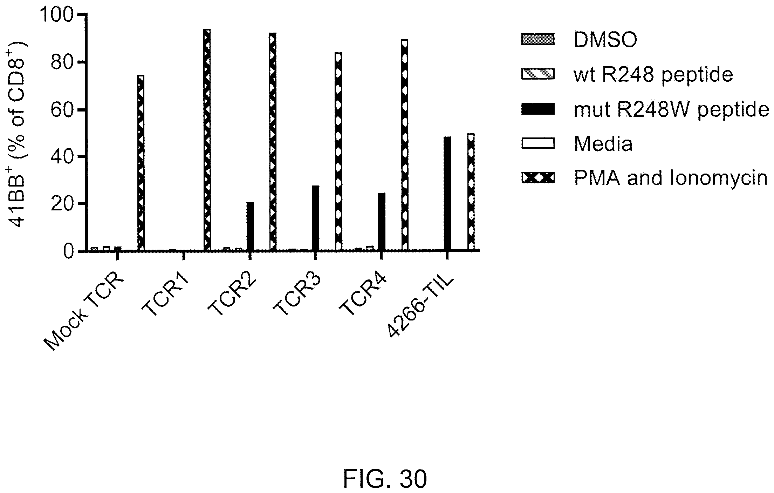

Figure 30 is a graph showing the percentage of 4-1BB+ cells (% of CD8+) detected following co-culture of T cells expressing mock (no TCR), 4266-TCR1, 4266-TCR2, 4266-TCR3 or 4266-TCR4 with autologous APCs which were pulsed with peptide vehicle (DMSO; gray bars) or purified (>95% by HPLC) 25 amino acid peptides composed of WT p53-R248 sequence (hatched gray bars) or mutated p53-R248W (black bars) sequence. Media alone (open bars) and PMA and Ionomycin (lattice bars) were negative and positive controls, respectively.

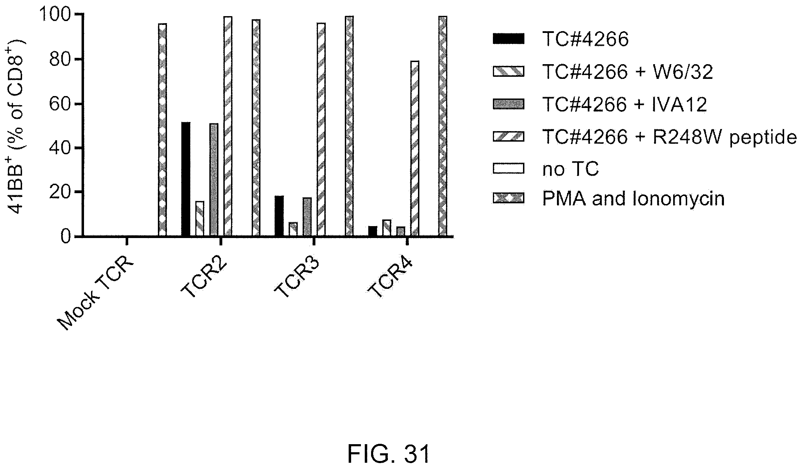

Figure 31 is a graph showing the percentage of 4-1BB+ cells (% of CD8+) detected following co-culture of T cells expressing mock (no TCR) or p53-R248W-specific TCRs (4266-TCR2, 4266-TCR3 or 4266-TCR4) with tumor cell (TC) line established from xenografted tumor fragment resected from Patient 4266 then serially passaged through immunocompromised mice (TC#4266). The TC#4266 cells were either incubated with nothing (black bars), W6/32 pan-HLA Class-I specific blocking antibody (right gray hatched bars), IVA12 pan-HLA Class-II specific blocking antibody (gray bars) or mutated p53-R248W peptide (left gray hatched bars). Media alone (no TC; open bars) and PMA and Ionomycin (gray lattice bars) were negative and positive controls, respectively.

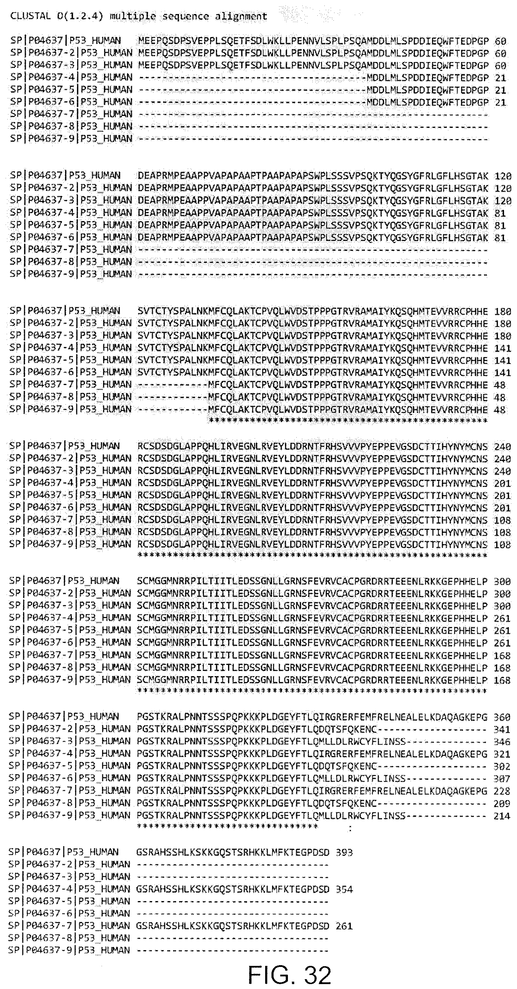

Figure 32 shows an alignment of the amino acid sequences of the nine p53 splice variants. SP|P04637|P53_HUMAN (SEQ ID NO: 1); SP|P04637-2|P53_HUMAN (SEQ ID NO: 535); SP|P04637-3|P53_HUMAN (SEQ ID NO: 536); SP|P04637-4|P53_HUMAN (SEQ ID NO: 537); SP|P04637-5|P53_HUMAN (SEQ ID NO: 538); SP|P04637-6|P53_HUMAN (SEQ ID NO: 539); SP|P04637-7|P53_HUMAN (SEQ ID NO: 540); SP|P04637-8|P53_HUMAN (SEQ ID NO: 541); and SP|P04637-9|P53_HUMAN (SEQ ID NO: 542).

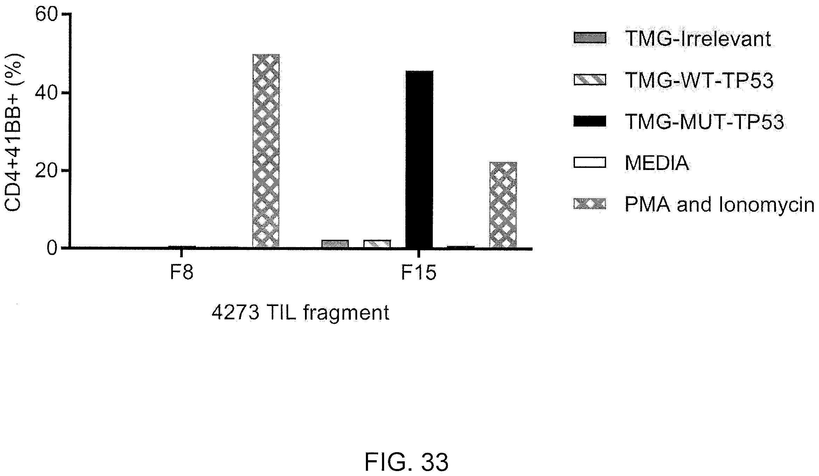

Figure 33 is a graph showing the percentage of 4-1BB+ cells (% of CD8+) detected following co-culture of TIL from patient 4273 with autologous APCs which were transfected with TMG encoding irrelevant mutations (gray bars), WT p53 sequences (gray hatched bars) or mutated p53 sequences including p53-R248W (black bars). Media alone (open bars) and PMA and Ionomycin (lattice bars) were negative and positive controls, respectively.

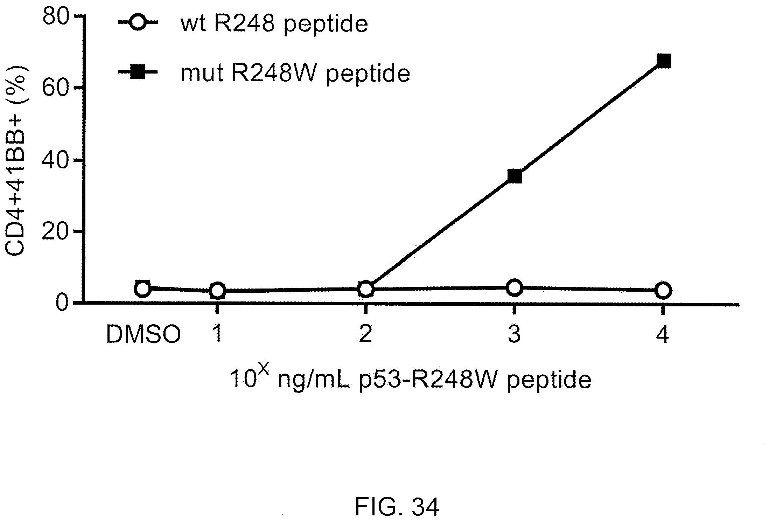

Figure 34 is a graph showing the percentage of 4-1BB+ cells (% of CD4+) detected following co-culture of TIL from patient 4273 with autologous APCs which were pulsed with 25 amino acid peptides corresponding to the WT (open circles) or mutated (closed squares) from the p53-R248W neoepitope. DMSO was peptide vehicle.

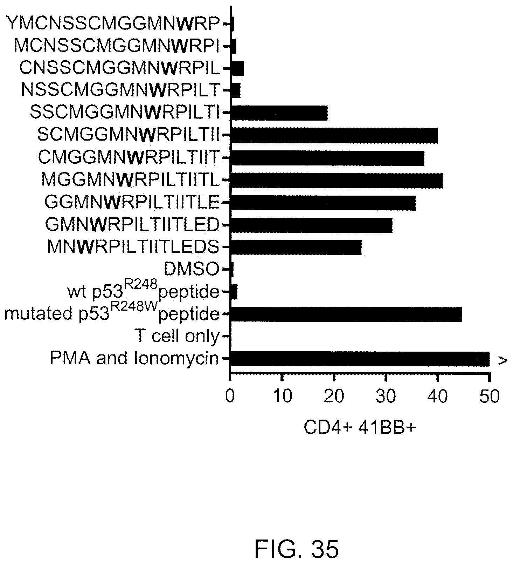

Figure 35 is a graph showing the percentage of 4-1BB+ cells (% of CD4+) detected following co-culture of TIL from patient 4273 with autologous APCs pulsed with 15 amino acid peptides from the p53-R248W neoepitope (amino acid substitution in bold) overlapping 14 amino acids. DMSO was peptide vehicle, media alone (T cells only) and PMA and ionomycin were controls. The 25 amino acid peptides (wt p53-R248 and mutated p53-R248W) were additional controls for the 15 amino acid peptides. The peptides are: YMCNSSCMGGMNWRP (SEQ ID NO: 592); MCNSSCMGGMNWRPI (SEQ ID NO: 593); CNSSCMGGMNWRPIL (SEQ ID NO: 594);
   NSSCMGGMNWRPILT (SEQ ID NO: 595); SSCMGGMNWRPILTI (SEQ ID NO: 596); SCMGGMNWRPILTII (SEQ ID NO: 597); CMGGMNWRPILTIIT (SEQ ID NO: 598); MGGMNWRPILTIITL (SEQ ID NO: 599); GGMNWRPILTIITLE (SEQ ID NO: 600); GMN**W**RPILTIITLED (SEQ ID NO: 601); and MNWRPILTIITLEDS (SEQ ID NO: 602).

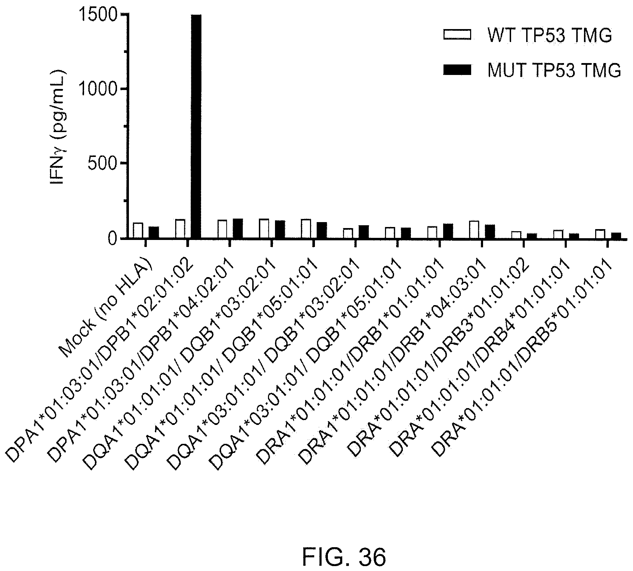

Figure 36 is a graph showing the concentration of IFN-γ (pg/mL) secreted following co-culture of TIL from Patient 4273 with Cos7 cells co-transfected with individual HLA alleles from patient 4273 and either WT (open bars) or mutated (black bars) TP53 TMG with or without the p53-R248W neoantigen, respectively.

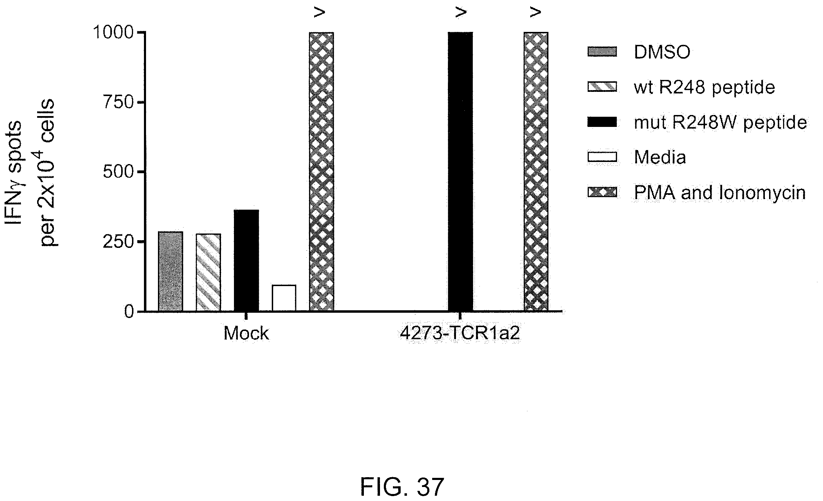

Figure 37 is a graph showing the number of IFN-γ spots per 2x10⁴ cells measured following co-culture of T cells expressing mock (no TCR) or 4273-TCR1a2 with autologous APCs which were pulsed with peptide vehicle (DMSO; gray bars) or purified (>95% by HPLC) 25 amino acid peptides composed of WT p53-R248 sequence (hatched gray bars) or mutated p53-R248W (black bars) sequence. Media alone (open bars) and PMA and Ionomycin (lattice bars) were negative and positive controls, respectively.

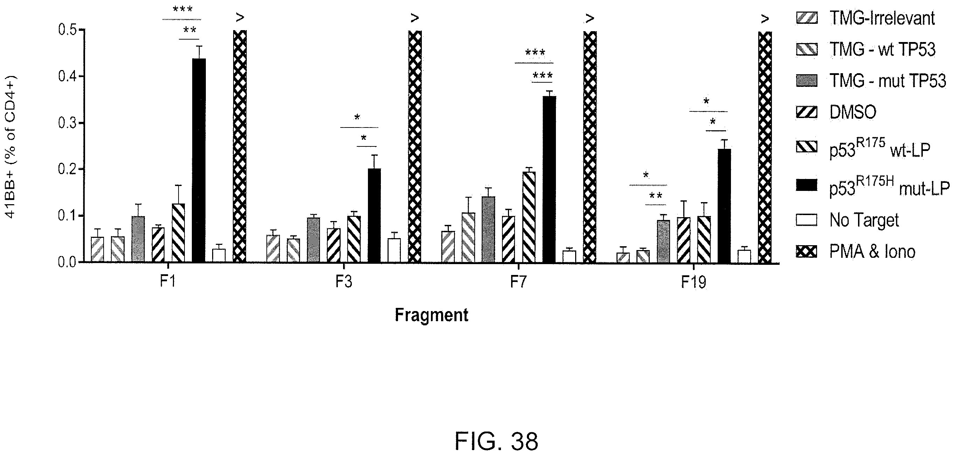

Figure 38 is a graph showing the percentage of 4-1BB+ cells (% of CD4+) detected following co-culture of TIL fragment F1, F3, F7 or F19 from patient 4252 with autologous APCs (immature DCs) that were either (1) electroporated with TMGs composed of irrelevant (IRR; left hatched gray bars), WT p53 (p53wt-TMG; right hatched gray bars) or mutated p53 (p53-mut-TMG; gray bars) sequences or (2) pulsed with peptide vehicle (DMSO; left hatched black bars) or purified (>95% by HPLC) 25 amino acid peptides composed of WT p53-R175 sequence (LP-p53-wt-R175; right hatched black bars) or mutated p53-R175H (LP-p53-mut-R175H; black bars) sequence. T cells only (no target; open bars) was negative control and PMA and Iono was positive control (lattice bars). Data are mean ± SEM (n=3). Student's t-tests were performed between indicated groups by line. *p<0.05, **p<0.01, ***p<0.001.

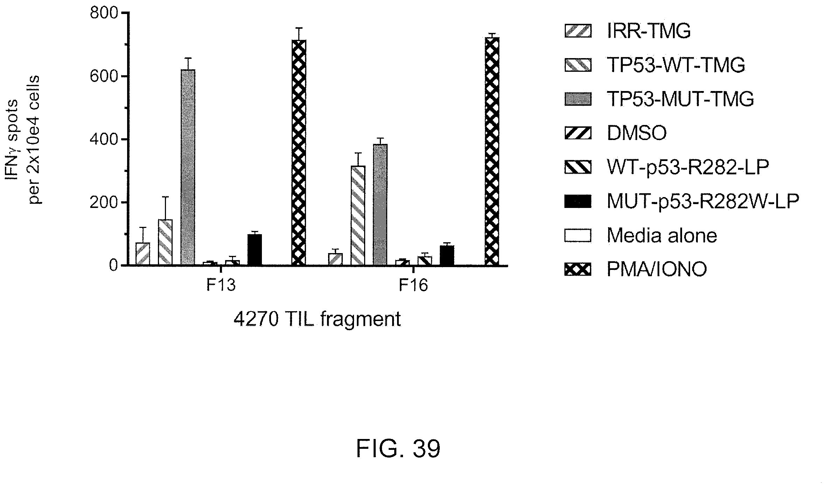

Figure 39 is a graph showing the number of IFN-γ spots per 2x10⁴ cells measured following co-culture of TIL fragment F13 or F16 from patient 4270 with autologous APCs (immature DCs) that were either (1) electroporated with TMGs composed of irrelevant (IRR; left hatched gray bars), WT p53 (p53wt-TMG; right hatched gray bars) or mutated p53 (p53-mut-TMG; gray bars) sequences or (2) pulsed with peptide vehicle (DMSO; left hatched black bars) or purified (>95% by HPLC) 25 amino acid peptides composed of WT p53-R282 sequence (LP-p53-wt-R282; right hatched black bars) or mutated p53-R282W (LP-p53-mut-R282W; black bars) sequence. T cells only (no target; open bars) was negative control and PMA and Iono was positive control (lattice bars). Data are mean ± SEM (n=3).

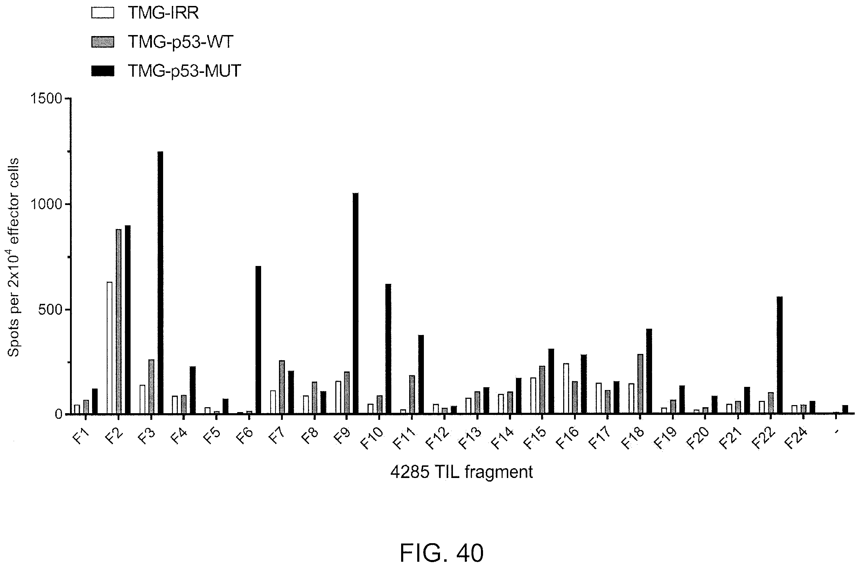

Figure 40 is a graph showing the number of IFN-γ spots per 2x10⁴ cells measured following co-culture of TIL fragments (F1-F24, n=18) from patient 4285 with autologous APCs electroporated with TMGs composed of irrelevant (IRR; open bars), WT p53 (p53-WT; gray bars) or mutated p53 (p53-MUT; black bars) sequences.

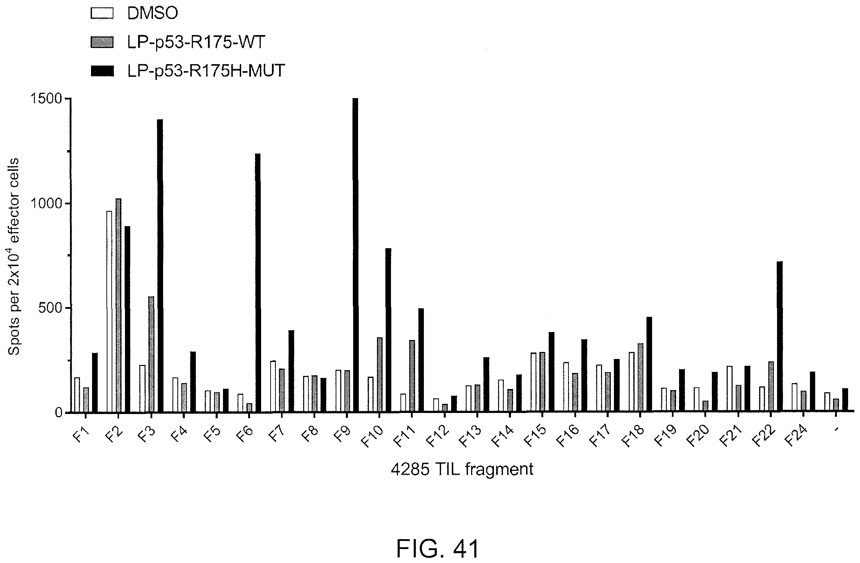

Figure 41 is a graph showing the number of IFN-γ spots per 2x10⁴ cells measured following co-culture of TIL fragments (F1-F24, n=18) from patient 4285 with autologous APCs pulsed with peptide vehicle (DMSO; open bars) or purified (>95% by HPLC) 25 amino acid peptides composed of WT p53-R175 sequence (LP-p53-R175-WT; gray bars) or mutated p53-R175H (LP-p53-R175H-MUT; black bars) sequence.

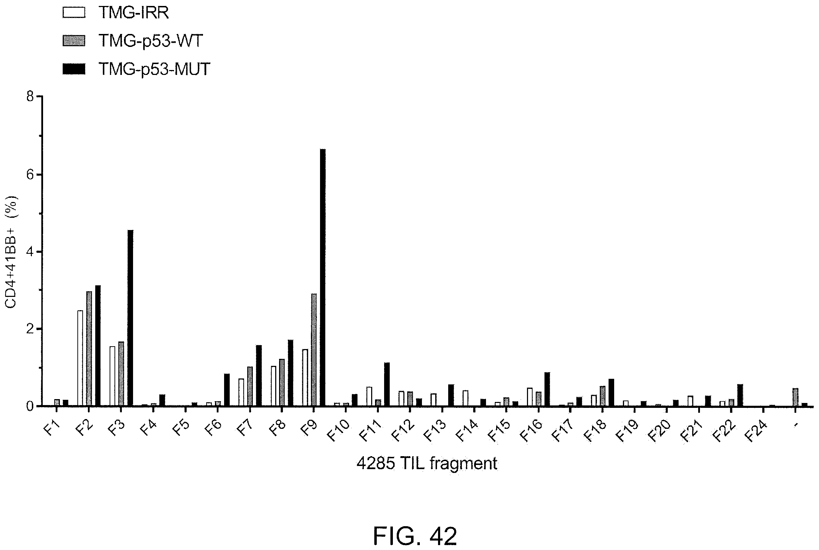

Figure 42 is a graph showing the percentage of 4-1BB+ cells (% of CD4+) detected following co-culture of TIL fragments (F1-F24, n=18) from patient 4285 with autologous APCs electroporated with TMGs composed of irrelevant (IRR; open bars) WT p53 (p53-WT; gray bars) or mutated p53 (p53-MUT; black bars) sequences.

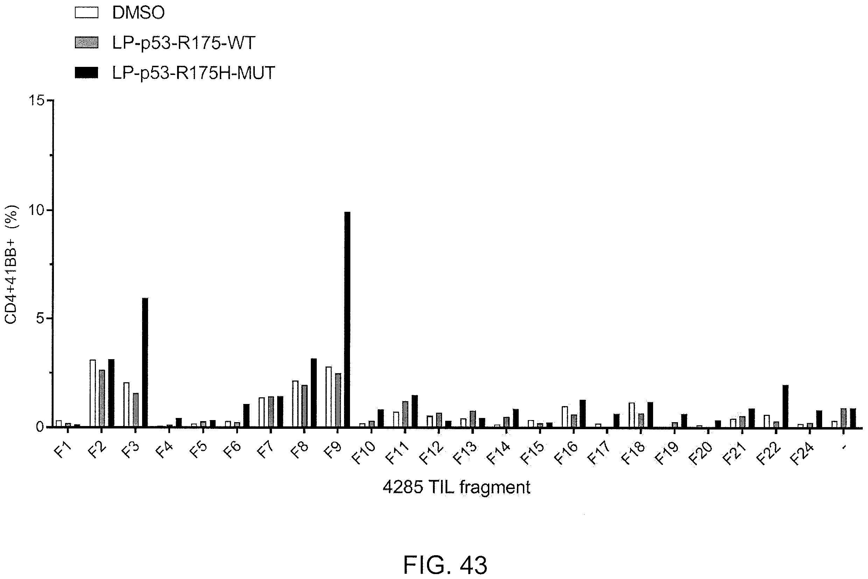

Figure 43 is a graph showing the percentage of 4-1BB+ cells (% of CD4+) detected following co-culture of TIL fragments (F1-F24, n=18) from patient 4285 with autologous APCs pulsed with peptide vehicle (DMSO; open bars) or purified (>95% by HPLC) 25 amino acid peptides composed of WT p53-R175 sequence (LP-p53-R175-WT; gray bars) or mutated p53-R175H (LP-p53-R175H-MUT; black bars) sequence.

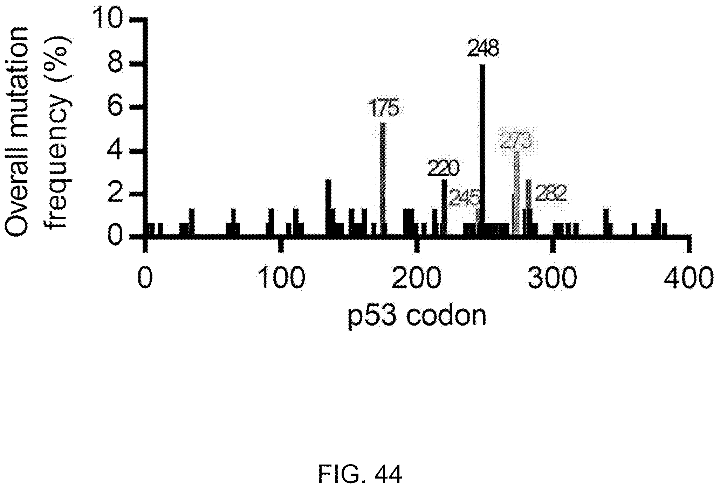

Figure 44 is a graph showing the overall mutation frequency (%) of each missense p53 mutation located at the indicated p53 codons in 141 epithelial tumors sequenced.

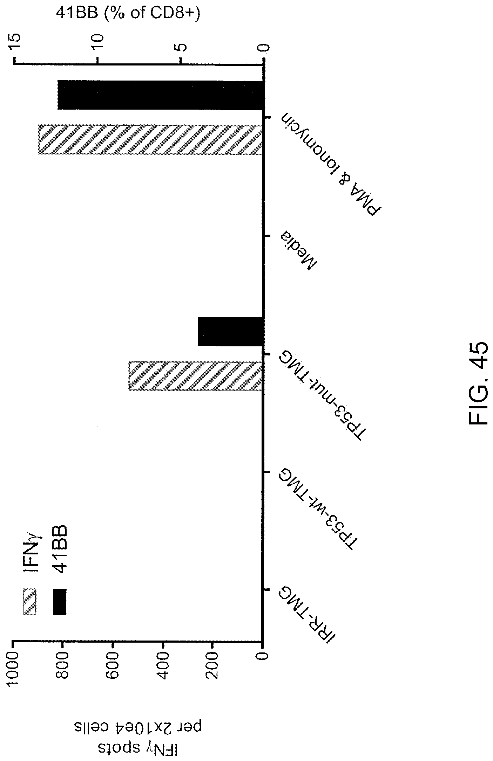

Figure 45 is a graph showing the percentage of 4-1BB positive cells (% of CD8+) (right y-axis; black bars) and IFN-γ (spots per 2x10⁴ cells) (left y-axis; hatched bars) measured following co-culture of TIL from Patient 4141 (fragment culture 12) with autologous APCs transfected with TMG encoding irrelevant mutations (TMG-IRR), WT p53 sequence (TP53-wt-TMG) or mutated p53 sequence including R175H (TP53-mut-TMG). Media alone and PMA and ionomycin were negative and positive controls, respectively.

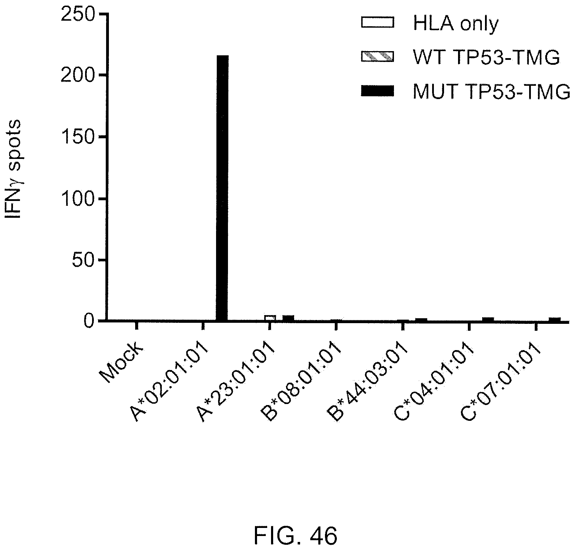

Figure 46 is a graph showing the number of IFN-γ-positive spots per 2x10⁴ effector cells measured following co-culture of TIL from Patient 4141 (fragment culture 12) with Cos7 cells co-transfected with the indicated HLA alleles and either no extra gene (HLA only; open bars), WT TP53 TMG (gray hatched bars), or mutated (black bars) TP53 TMG containing the p53-R175H sequence.

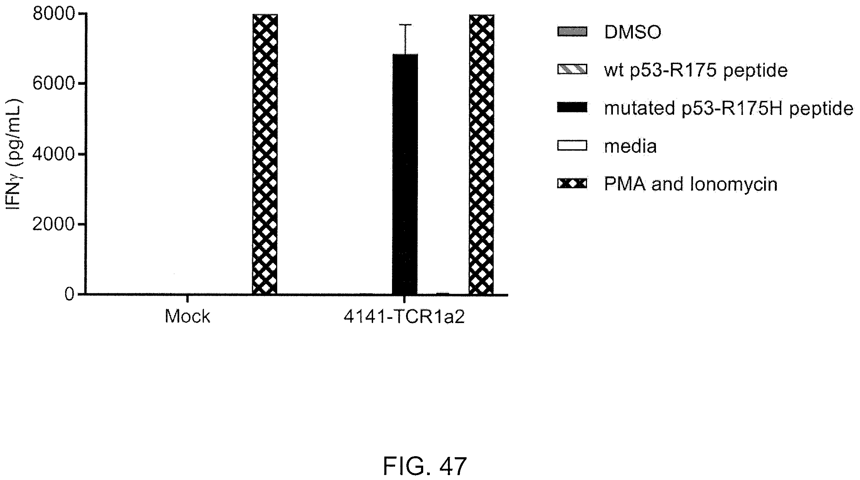

Figure 47 is a graph showing the concentration of IFN-γ (pg/mL) measured following co-culture of T cells expressing mock (no TCR) or 4141-TCR1a2 with T2 tumor cells (expressing HLA-A*02:01). T2 cells were pulsed with peptide vehicle (DMSO; gray bars) or purified (>95% by HPLC) peptides composed of WT p53-R175 peptide (hatched gray bars) or mutated p53-R175H peptide (black bars). Media alone (open bars) and PMA and Ionomycin (lattice bars) were negative and positive controls, respectively. Data are mean ± SEM (n=3).

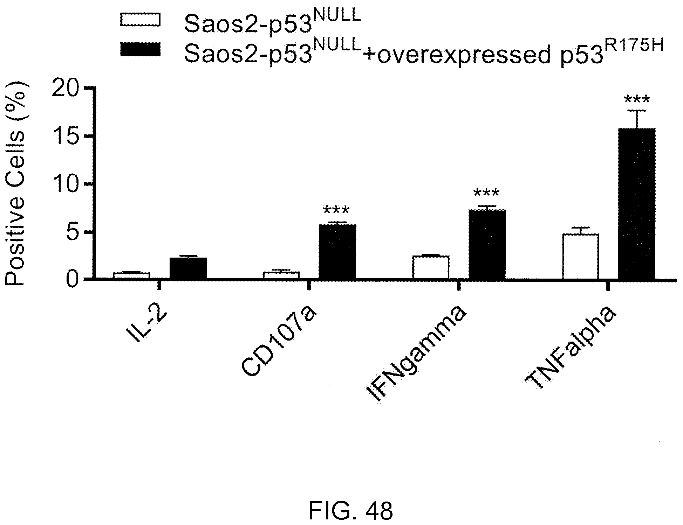

Figure 48 is a graph showing the percentage of cells positive for expression of one of the indicated markers following co-culture of T cells expressing 4141-TCR1a2 with Saos2 cells (p53-NULL and HLA-A*02:01+), which were either unmanipulated (unshaded bars) or made to overexpress full length p53-R175H protein (shaded bars). Data are mean ± SEM (n=3). Student's two-tailed t-tests were performed for each cytokine between the two cell lines for statistical analyses (***p<0.001).

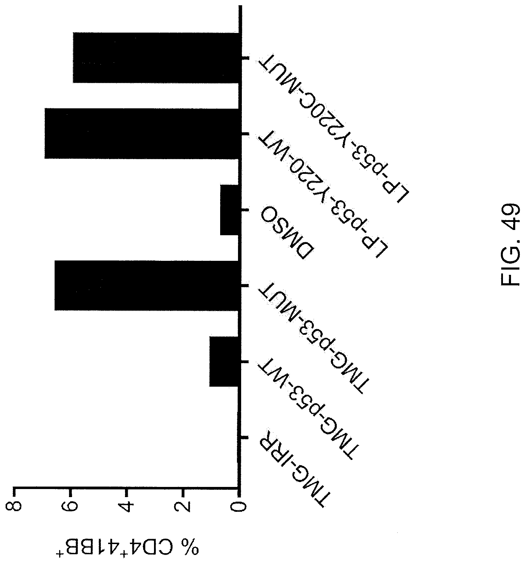

Figure 49 is a graph showing the percentage of CD4+4-1BB positive cells detected following co-culture of TIL (fragment culture 6 from patient 4259) with autologous APCs either (1) electroporated with TMG composed of irrelevant (TMG-IRR), WT p53 (TMG-p53-WT) or mutated p53 (TMG-p53-MUT) sequence or (2) pulsed with peptide vehicle (DMSO) or purified (>95% by HPLC) 25-amino acid peptides composed of WT p53-Y220 sequence (LP-p53-Y220-WT) or mutated p53-Y220C (LP-p53-Y220C-MUT) sequence.

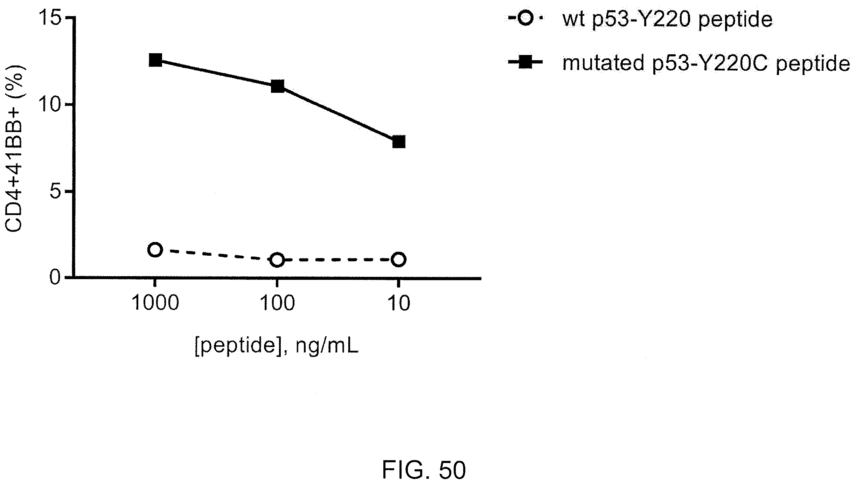

Figure 50 is a graph showing the percentage of CD4+4-1BB positive cells detected following co-culture of TIL fragment culture (no. 6) from patient 4259 with autologous APCs pulsed with decreasing concentrations of 25-amino acid peptides corresponding to the WT p53 sequence (open circles) or mutated p53-Y220C (closed squares) for 2 hours at 37 °C.

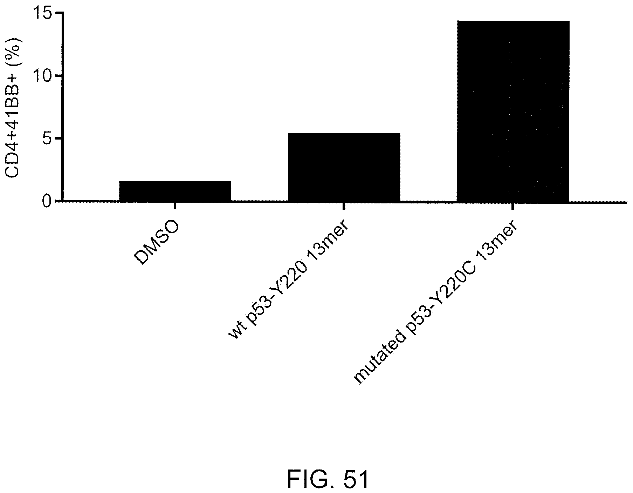

Figure 51 is a graph showing the percentage of CD4+4-1BB positive cells detected following co-culture of TIL from patient 4259 with autologous APCs pulsed with DMSO, WT p53-Y220 peptide, or mutated p53-Y220C peptide.

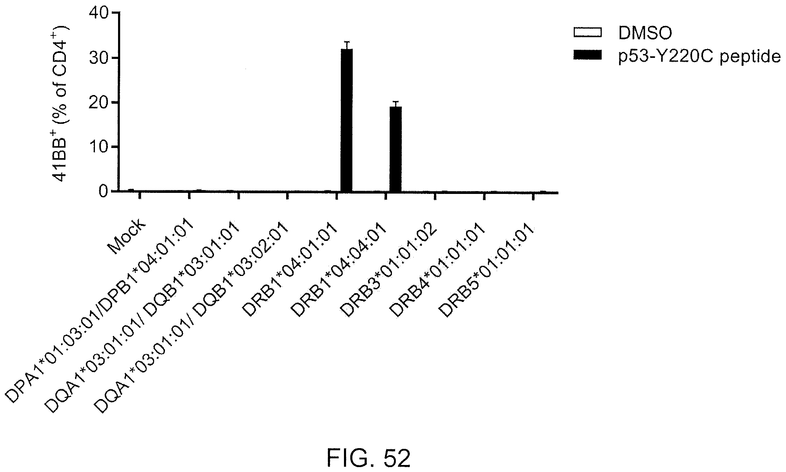

Figure 52 is a graph showing the percentage of 4-1BB positive cells (% of CD4+) detected following co-culture of TIL fragment culture no. 6 from Patient 4259 with Cos7 cells co-transfected with the indicated HLA alleles from patient 4259 and pulsed with DMSO (open bars) or the p53-Y220C peptide (closed bars).

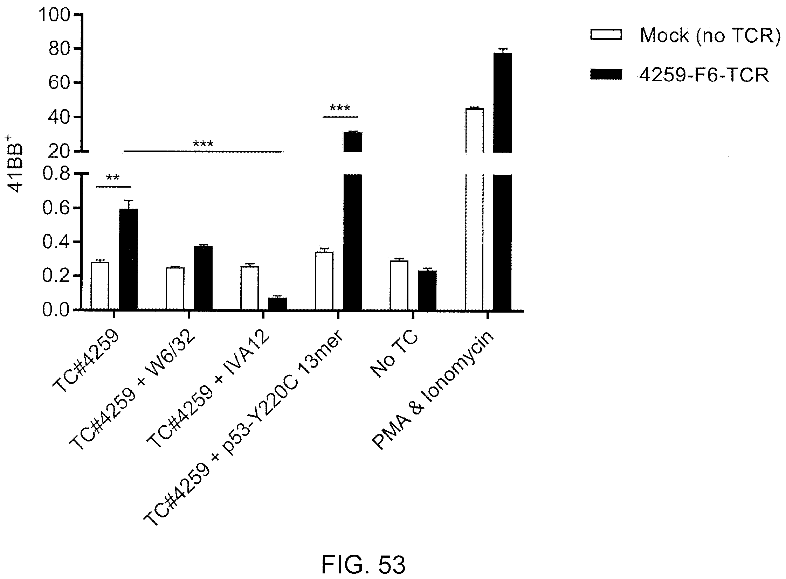

Figure 53 is a graph showing the percentage of 4-1BB+ cells detected following co-culture of TC#4259 target cells (endogenously expressing p53-Y220C and HLA-DRB1*04:01:01) with effector T cells (10⁵) expressing mock (no TCR; open bars) or p53-Y220C-specific TCR (4259-F6-TCR; black bars). The TC#4259 cells were either incubated with nothing, W6/32 pan-HLA Class-I specific blocking antibody, IVA12 pan-HLA Class-II specific blocking antibody or mutated p53-Y220C peptide. Media alone (no TC) and PMA and Ionomycin were negative and positive controls, respectively. Data are mean ± SEM (n=3). Student's two-tailed t-tests were performed between groups as indicated for statistical analyses (**p<0.01 and ***p<0.001).

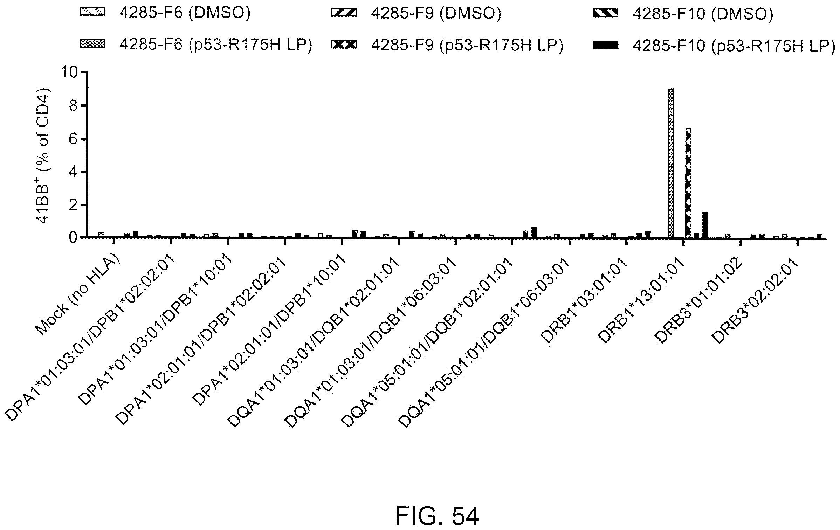

Figure 54 is a graph showing the percentage of 4-1BB+ cells (% of CD4+) detected following co-culture of TIL fragment cultures 4285-F6, 4285-F9 and 4285-F10 with Cos7 cells transfected with the indicated HLA alleles and pulsed with DMSO (peptide vehicle; gray and black hatched bars) or mutated p53-R175H peptide (gray, lattice and black bars).

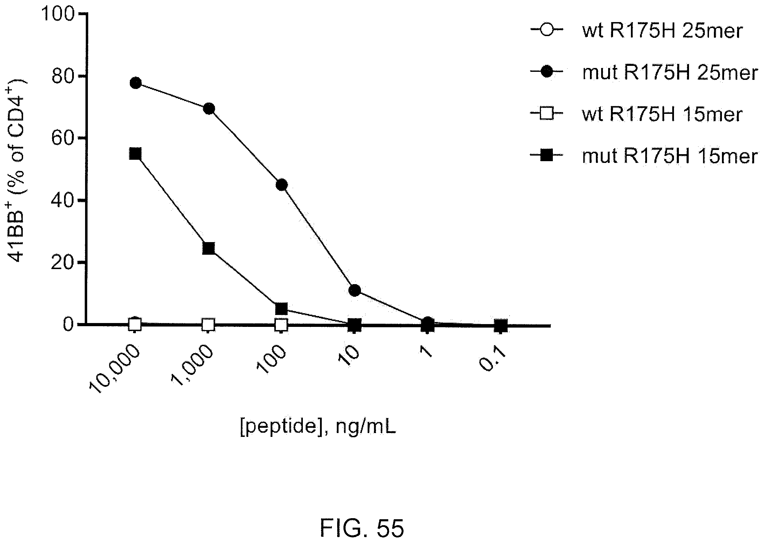

Figure 55 is a graph showing the percentage of 4-1BB+ cells (% of CD4+) detected following co-culture of T cells transposed with 4285-TCR1 with autologous APCs pulsed with decreasing concentrations of 25- or 15-amino acid peptides corresponding to the WT (open circles and squares) or mutated (closed circles and squares) p53-R175H sequence.

### DETAILED DESCRIPTION OF THE INVENTION

Tumor Protein P53 (also referred to as "TP53" or "p53") acts as a tumor suppressor by, for example, regulating cell division. The p53 protein is located in the nucleus of the cell, where it binds directly to DNA. When DNA becomes damaged, the p53 protein is involved in determining whether the DNA will be repaired or the damaged cell will undergo apoptosis. If the DNA can be repaired, p53 activates other genes to fix the damage. If the DNA cannot be repaired, the p53 protein prevents the cell from dividing and signals it to undergo apoptosis. By stopping cells with mutated or damaged DNA from dividing, p53 helps prevent the development of tumors. Wild-type (WT) full-length p53 comprises the amino acid sequence of SEQ ID NO: 1.

Mutations in the p53 protein may reduce or eliminate the p53 protein's tumor suppressor function. Alternatively or additionally, a p53 mutation may be a gain-of-function mutation by interfering with wild type p53 in a dominant negative fashion. Mutated p53 protein may be expressed in any of a variety of human cancers such as, for example, cholangiocarcinoma, melanoma, colon cancer, rectal cancer, ovarian cancer, endometrial cancer, non-small cell lung cancer (NSCLC), glioblastoma, uterine cervical cancer, head and neck cancer, breast cancer, pancreatic cancer, or bladder cancer.

An embodiment of the invention provides a method of isolating T cells having antigenic specificity for a human p53 mutated amino acid sequence encoded by a cancer-specific p53 mutation. Mutations of p53 are defined herein by reference to the amino acid sequence of full-length, WT p53 (SEQ ID NO: 1). Thus, mutations of p53 are described herein by reference to the amino acid residue present at a particular position, followed by the position number, followed by the amino acid with which that residue has been replaced in the particular mutation under discussion. For example, when the positions are as defined by SEQ ID NO: 1, the term "R175" refers to the arginine present at position 175 of SEQ ID NO: 1, "R175H" indicates that the arginine present at position 175 of SEQ ID NO: 1 is replaced by histidine, while "G245S" indicates that the glycine present at position 245 of SEQ ID NO: 1 has been replaced with serine. P53 has nine known splice variants. The p53 mutations described herein are conserved over all nine p53 splice variants. An alignment of the nine p53 splice variants is shown in Figure 32. Accordingly, T cells isolated by the inventive methods may have antigenic specificity for any mutated human p53 amino acid sequence described herein encoded by any of the nine p53 splice variants. When the positions are as defined by SEQ ID NO: 1, then the actual positions of the amino acid sequence of a particular splice variant of p53 are defined relative to the corresponding positions of SEQ ID NO: 1, and the positions as defined by SEQ ID NO: 1 may be different than the actual positions in a particular splice variant. Thus, for example, mutations refer to a replacement of an amino acid residue in the amino acid sequence of a particular splice variant of p53 corresponding to the indicated position of the 393-amino acid sequence of SEQ ID NO: 1 with the understanding that the actual positions in the splice variant may be different.

Embodiments of the invention may provide any one or more of many advantages. Mutated p53 is expressed by cancer cells and is not expressed by normal, noncancerous cells. Without being bound to a particular theory or mechanism, it is believed that T cells isolated by the inventive methods advantageously target the destruction of cancer cells while minimizing or eliminating the destruction of normal, non-cancerous cells, thereby reducing, for example, by minimizing or eliminating, toxicity. Moreover, T cells isolated by the inventive methods may, advantageously, successfully treat or prevent mutated p53-positive cancers that do not respond to other types of treatment such as, for example, chemotherapy, surgery, or radiation. Additionally, T cells isolated by the inventive methods may provide highly avid recognition of mutated p53, which may provide the ability to recognize unmanipulated tumor cells (e.g., tumor cells that have not been treated with interferon (IFN)-γ, transfected with a vector encoding one or both of mutated p53 and the applicable HLA molecule, pulsed with a p53 peptide with the p53 mutation, or a combination thereof). Roughly half of all tumors harbor a mutation in p53, about half of which will be a missense mutation and about 30% of the missense mutations occur at the following "hotspot" residues: R175H, Y220C, G245D, G245S, R248L, R248Q, R248W, R249S, R273C, R273L, R273H and R282W. Moreover, the same "hotspot" mutations in p53 (e.g., R175H, Y220C, G245D, G245S, R248L, R248Q, R248W, R249S, R273H, R273C, R273L, or R282W) occur frequently (cumulatively about 30% of the p53 missense mutations) in tumors of unrelated people. Accordingly, T cells isolated by the inventive methods may increase the number of patients who may be eligible for treatment with immunotherapy.

The inventive methods may be faster, more focused, and more specific as compared to traditional methods of isolating cancer mutation-reactive T cells. Such traditional methods involve identifying gene(s) containing a cancer-specific mutation (e.g., neoantigens) expressed by the patient prior to co-culturing the patient's autologous T cells with the patient's autologous antigen presenting cells which present peptides comprising the cancer-specific mutation encoded by the identified gene(s) as described in, for example, US 2017/0218042 and US 2017/0224800 (hereinafter, "traditional screening methods"). With the inventive methods, peptides and tandem minigenes (TMG) including p53 mutations can be produced, stocked and validated prior to screening a patient so that once a p53 mutation is identified in a patient's tumor, the patient's T cells can be tested independent of the knowledge of the other mutations in the patient's tumor. This may reduce the time required to carry out the method which may be particularly advantageous when evaluating terminally ill patients. For example, the duration of the inventive methods may be, e.g., about 3 to about 6 weeks shorter than traditional screening methods. The inventive methods may also provide a highly-focused study of p53 mutation responses in the absence of other competing mutations. Without being bound to a particular theory or mechanism, it is believed that pools of peptides or competing peptides in patient mutation-derived TMGs (with mutations in proteins other than p53) can interfere with the mutated p53 peptides and could mask a T cell response. Because the inventive methods focus on mutations in only one gene, p53, the inventive methods may provide increased speed and efficiency in the attempt to identify the p53 mutation-specific T cells and TCRs as compared to traditional screening methods, which involve parsing out the neoantigens in the peptide pool or TMG to determine which neoantigen generated the T cell response. The inventive methods may, advantageously, generate T cells and/or TCRs useful for autologous or allogeneic therapy. By focusing on p53 mutations, it may be possible to identify low frequency responses, which may be diluted in the high throughput of traditional screening methods, and may allow for the identification of T cells and TCRs which otherwise would have been lost in the complexity of screening dozens to hundreds of neoantigens. The inventive methods may be useful for testing T cell responses in a patient independent of the knowledge of whether the patient had the corresponding mutation or even a tumor. The inventive methods may be useful for screening patients with a defined HLA haplotype and predicted p53 mutation binding, but are not limited to screening this type of cohort.

The cancer-specific human p53 mutation may be any mutation in the p53 gene which encodes a mutated p53 amino acid sequence (also referred to as a "non-silent mutation") and which is expressed in a cancer cell but not in a normal, noncancerous cell. Non-limiting examples of cancer-specific p53 mutations include missense, nonsense, insertion, deletion, duplication, frameshift, and repeat expansion mutations. In a preferred embodiment, the p53 mutation is a missense mutation.

In an embodiment of the invention, the mutated p53 amino acid sequence comprises a human p53 amino acid sequence with a mutation at position 175, 220, 245, 248, 249, 273, or 282 of SEQ ID NO: 1. The p53 mutation may be any missense mutation. Accordingly, the mutation at position 175, 220, 245, 248, 249, 273, or 282 of SEQ ID NO: 1 may be a substitution of the native (WT) amino acid residue present at position 175, 220, 245, 248, 249, 273, or 282 of SEQ ID NO: 1 with any amino acid residue other than the native (WT) amino acid residue present at the particular position under discussion. In an embodiment of the invention, the mutated p53 amino acid sequence comprises a human p53 amino acid sequence with one of the following human p53 mutations: R175H, Y220C, G245D, G245S, R248L, R248Q, R248W, R249S, R273H, R273C, R273L, or R282W. For example, the mutated p53 amino acid sequence may comprise a mutated p53 amino acid sequence selected from the group consisting of SEQ ID NOs: 2-13.

The method comprises inducing autologous antigen presenting cells (APCs) of a patient to present at least one mutated human p53 amino acid sequence. The APCs may include any cells which present peptide fragments of proteins in association with major histocompatibility complex (MHC) molecules on their cell surface. The APCs may include, for example, any one or more of macrophages, DCs, langerhans cells, B-lymphocytes, and T-cells. Preferably, the APCs are DCs. By using autologous APCs from the patient, the inventive methods may, advantageously, identify T cells that have antigenic specificity for a mutated p53 amino acid sequence encoded by a cancer-specific p53 mutation that is presented in the context of an MHC molecule expressed by the patient. The MHC molecule can be any MHC molecule expressed by the patient including, but not limited to, MHC Class I, MHC Class II, HLA-A, HLA-B, HLA-C, HLA-DM, HLA-DO, HLA-DP, HLA-DQ, and HLA-DR molecules. The inventive methods may, advantageously, identify mutated p53 amino acid sequences presented in the context of any MHC molecule expressed by the patient without using, for example, epitope prediction algorithms to identify MHC molecules or mutated p53 amino acid sequences, which may be useful only for a select few MHC class I alleles and may be constrained by the limited availability of reagents to select p53 mutation-reactive T cells (e.g., an incomplete set of MHC tetramers). Accordingly, in an embodiment of the invention, the inventive methods advantageously induce APCs to present the mutated p53 amino acid sequences in the context of any MHC molecule expressed by the patient and are not limited to any particular MHC molecule. Preferably, the autologous APCs are antigen-negative autologous APCs.

Inducing autologous APCs of the patient to present the at least one mutated human p53 amino acid sequence is carried out using (i) or (ii) below. In an embodiment of the invention, inducing autologous APCs of the patient to present the at least one mutated human p53 amino acid sequence comprises (i) pulsing the autologous APCs with a peptide comprising no more than one mutated human p53 amino acid sequence or a pool of peptides, each peptide in the pool comprising a different mutated human p53 amino acid sequence. In this regard, the autologous APCs may be cultured with a peptide or a pool of peptides comprising the mutated human p53 amino acid sequence in a manner such that the APCs internalize the peptide(s) and display the mutated human p53 amino acid sequence(s), bound to an MHC molecule, on the cell membrane. Methods of pulsing APCs are known in the art and are described in, e.g., Solheim (Ed.), Antigen Processing and Presentation Protocols (Methods in Molecular Biology), Human Press, (2010). The peptide(s) used to pulse the APCs may include the mutated human p53 amino acid(s) encoded by the cancer-specific p53 mutation. The peptide(s) may further comprise any suitable number of contiguous amino acids from the endogenous p53 protein encoded by the p53 gene on each of the carboxyl side and the amino side of the mutated amino acid(s). The number of contiguous amino acids from the endogenous p53 protein flanking each side of the mutation is not limited and may be, for example, about 4, about 5, about 6, about 7, about 8, about 9, about 10, about 11, about 12, about 13, about 14, about 15, about 16, about 17, about 18, about 19, about 20, or a range defined by any two of the foregoing values. Preferably, the peptide(s) comprise(s) about 12 contiguous amino acids from the endogenous p53 protein on each side of the mutated amino acid(s). In an embodiment, the peptide or pool of peptides comprise(s) one or more of the mutated p53 peptides of SEQ ID NOs: 2-13 (Table 1).

In an embodiment of the invention, inducing autologous APCs of the patient to present the mutated human p53 amino acid sequence comprises (ii) introducing a nucleotide sequence encoding the mutated human p53 amino acid sequence into the APCs. The nucleotide sequence is introduced into the APCs so that the APCs express and display the mutated human p53 amino acid sequence, bound to an MHC molecule, on the cell membrane. The nucleotide sequence encoding the mutated human p53 amino acid may be RNA or DNA. Introducing a nucleotide sequence into APCs may be carried out in any of a variety of different ways known in the art as described in, e.g., Solheim et al. *supra.* Non-limiting examples of techniques that are useful for introducing a nucleotide sequence into APCs include transformation, transduction, transfection, and electroporation. In an embodiment, the method may comprise preparing more than one nucleotide sequence, each encoding a different mutated human p53 amino acid sequence, and introducing each nucleotide sequence into a different population of autologous APCs. In this regard, multiple populations of autologous APCs, each population expressing and displaying a different mutated human p53 amino acid sequence, may be obtained.

In an embodiment, the method may comprise introducing a nucleotide sequence encoding the more than one mutated human p53 amino acid sequence. In this regard, in an embodiment of the invention, the nucleotide sequence introduced into the autologous APCs is a tandem minigene (TMG) construct, each minigene comprising a p53 gene, each p53 gene including a cancer-specific p53 mutation that encodes a different mutated human p53 amino acid sequence. Each minigene may encode one p53 mutation flanked on each side of the p53 mutation by any suitable number of contiguous amino acids from the endogenous p53 protein, as described herein with respect to other aspects of the invention. The number of minigenes in the construct is not limited and may include for example, about 5, about 10, about 11, about 12, about 13, about 14, about 15, about 20, about 25, or more, or a range defined by any two of the foregoing values. In an embodiment of the invention, the TMG construct encodes one or more mutated p53 peptides of SEQ ID NOs: 2-13. For example, the TMG construct may encode the amino acid sequence of SEQ ID NO: 14. The APCs express the mutated p53 amino acid sequences encoded by the TMG construct and display the mutated p53 amino acid sequences, bound to an MHC molecule, on the cell membranes. In an embodiment, the method may comprise preparing more than one TMG construct, each construct encoding a different set of mutated human p53 amino acid sequences, and introducing each TMG construct into a different population of autologous APCs. In this regard, multiple populations of autologous APCs, each population expressing and displaying mutated human p53 amino acid sequences encoded by different TMG constructs, may be obtained.

The method comprises culturing autologous T cells of the patient with the autologous APCs that present the mutated human p53 amino acid sequence. The T cells can be obtained from numerous sources in the patient, including but not limited to tumor, blood, bone marrow, lymph node, the thymus, or other tissues or fluids. The T cells can include any type of T cell and can be of any developmental stage, including but not limited to, CD4+/CD8+ double positive T cells, CD4+ helper T cells, e.g., Th1 and Th2 cells, CD8+ T cells (e.g., cytotoxic T cells), tumor infiltrating cells (e.g., tumor infiltrating lymphocytes (TIL)), peripheral blood T cells, memory T cells, naive T cells, and the like. The T cells may be CD8+ T cells, CD4+ T cells, or both CD4+ and CD8+ T cells. The method may comprise co-culturing the autologous T cells and autologous APCs so that the T cells encounter the mutated p53 amino acid sequence presented by the APCs in such a manner that the autologous T cells specifically bind to and immunologically recognize a mutated p53 amino acid sequence presented by the APCs. In an embodiment of the invention, the autologous T cells are co-cultured in direct contact with the autologous APCs.

The method comprises selecting the autologous T cells that (a) were co-cultured with the autologous APCs that present the mutated human p53 amino acid sequence and (b) have antigenic specificity for the mutated human p53 amino acid sequence presented in the context of a MHC molecule expressed by the patient. The phrase "antigenic specificity," as used herein, means that the autologous T cells can specifically bind to and immunologically recognize the mutated human p53 amino acid sequence encoded by the cancer-specific p53 mutation. The selecting may comprise identifying the T cells that have antigenic specificity for the mutated p53 amino acid sequence and separating them from T cells that do not have antigenic specificity for the mutated p53 amino acid sequence. Selecting the autologous T cells having antigenic specificity for the mutated p53 amino acid sequence may be carried out in any suitable manner. In an embodiment of the invention, the method comprises expanding the numbers of autologous T cells, e.g., by co-culturing with a T cell growth factor, such as interleukin (IL)-2 or IL-15, or as described herein with respect to other aspects of the invention, prior to selecting the autologous T cells. In an embodiment of the invention, the method does not comprise expanding the numbers of autologous T cells with a T cell growth factor, such as IL-2 or IL-15 prior to selecting the autologous T cells.

For example, upon co-culture of the autologous T cells with the APCs that present the mutated p53 amino acid sequence, T cells having antigenic specificity for the mutated p53 amino acid sequence may express any one or more of a variety of T cell activation markers which may be used to identify those T cells having antigenic specificity for the mutated p53 amino acid sequence. Such T cell activation markers may include, but are not limited to, programmed cell death 1 (PD-1), lymphocyte-activation gene 3 (LAG-3), T cell immunoglobulin and mucin domain 3 (TIM-3), 4-1BB, OX40, and CD107a. Accordingly, in an embodiment of the invention, selecting the autologous T cells that have antigenic specificity for the mutated p53 amino acid sequence comprises selecting the T cells that express any one or more of PD-1, LAG-3, TIM-3, 4-1BB, OX40, and CD107a. Cells expressing one or more T cell activation markers may be sorted on the basis of expression of the marker using any of a variety of techniques known in the art such as, for example, fluorescence-activated cell sorting (FACS) or magnetic-activated cell sorting (MACS) as described in, e.g., Turcotte et al., Clin. Cancer Res., 20(2): 331-43 (2013) and Gros et al., J. Clin. Invest., 124(5): 2246-59 (2014).

In another embodiment of the invention, selecting the autologous T cells that have antigenic specificity for the mutated p53 amino acid sequence comprises selecting the T cells (i) that secrete a greater amount of one or more cytokines upon co-culture with APCs that present the mutated p53 amino acid sequence as compared to the amount of the one or more cytokines secreted by a negative control or (ii) in which at least twice as many of the numbers of T cells secrete one or more cytokines upon co-culture with APCs that present the mutated p53 amino acid sequence as compared to the numbers of negative control T cells that secrete the one or more cytokines. The one or more cytokines may comprise any cytokine the secretion of which by a T cell is characteristic of T cell activation (e.g., a T cell receptor (TCR) expressed by the T cells specifically binding to and immunologically recognizing the mutated p53 amino acid sequence). Non-limiting examples of cytokines, the secretion of which is characteristic of T cell activation, include IFN-γ, IL-2, and tumor necrosis factor alpha (TNF-α), granulocyte/monocyte colony stimulating factor (GM-CSF), IL-4, IL-5, IL-9, IL-10, IL-17, and IL-22.

For example, the autologous T cells may be considered to have "antigenic specificity" for the mutated p53 amino acid sequence if the T cells secrete at least twice as much IFN-y upon co-culture with (a) antigen-negative APCs pulsed with a concentration of a peptide comprising the mutated p53 amino acid sequence (e.g., about 0.05 ng/mL to about 10 µg/mL, e.g., 0.05 ng/mL, 0.1 ng/mL, 0.5 ng/mL, 1 ng/mL, 5 ng/mL, 100 ng/mL, 1 µg/mL, 5 µg/mL, or 10 µg/mL) or (b) APCs into which a nucleotide sequence encoding the mutated p53 amino acid sequence has been introduced as compared to the amount of IFN-γ secreted by a negative control. The negative control may be, for example, autologous T cells (e.g., derived from peripheral blood mononuclear cells (PBMC)) co-cultured with (a) antigen-negative APCs pulsed with the same concentration of an irrelevant peptide (e.g., the wild-type amino acid sequence, or some other peptide with a different sequence from the mutated p53 amino acid sequence) or (b) APCs into which a nucleotide sequence encoding an irrelevant peptide sequence has been introduced. The autologous T cells may also have "antigenic specificity" for the mutated p53 amino acid sequence if the T cells secrete a greater amount of IFN-y upon co-culture with antigen-negative APCs pulsed with higher concentrations of a peptide comprising the mutated p53 amino acid sequence as compared to a negative control, for example, the negative control described above. IFN-γ secretion may be measured by methods known in the art such as, for example, enzyme-linked immunosorbent assay (ELISA).

Alternatively or additionally, the autologous T cells may be considered to have "antigenic specificity" for the mutated p53 amino acid sequence if at least twice as many of the numbers of T cells secrete IFN-y upon co-culture with (a) antigen-negative APCs pulsed with a concentration of a peptide comprising the mutated p53 amino acid sequence or (b) APCs into which a nucleotide sequence encoding the mutated p53 amino acid sequence has been introduced as compared to the numbers of negative control T cells that secrete IFN-γ. The concentration of peptide and the negative control may be as described herein with respect to other aspects of the invention. The numbers of cells secreting IFN-γ may be measured by methods known in the art such as, for example, ELISPOT.

Alternatively or additionally, the autologous T cells may be considered to have "antigenic specificity" for mutated p53 if at least twice as many spots are detected by ELISPOT for the T cells upon co-culture with (a) antigen-negative, applicable HLA molecule positive target cells pulsed with a low concentration of mutated p53 peptide or (b) antigen-negative, applicable HLA molecule positive target cells into which a nucleotide sequence encoding mutated p53 has been introduced such that the target cell expresses mutated p53 as compared to the number of spots detected by ELISPOT for negative control T cells co-cultured with the same target cells. The concentration of peptide and the negative control may be as described herein with respect to other aspects of the invention.

Alternatively or additionally, the autologous T cells may be considered to have "antigenic specificity" for mutated p53 if greater than about 50 spots are detected by ELISPOT for the T cells expressing the TCR upon co-culture with (a) antigen-negative, applicable HLA molecule positive target cells pulsed with a low concentration of mutated p53 peptide or (b) antigen-negative, applicable HLA molecule positive target cells into which a nucleotide sequence encoding mutated p53 has been introduced such that the target cell expresses mutated p53. The concentration of peptide may be as described herein with respect to other aspects of the invention.

While T cells having antigenic specificity for the mutated p53 amino acid sequence may both (1) express any one or more T cells activation markers described herein and (2) secrete a greater amount of one or more cytokines as described herein, in an embodiment of the invention, T cells having antigenic specificity for the mutated p53 amino acid sequence may express any one or more T cell activation markers without secreting a greater amount of one or more cytokines or may secrete a greater amount of one or more cytokines without expressing any one or more T cell activation markers.

In another embodiment of the invention, selecting the autologous T cells that have antigenic specificity for the mutated p53 amino acid sequence comprises selectively growing the autologous T cells that have antigenic specificity for the mutated p53 amino acid sequence. In this regard, the method may comprise co-culturing the autologous T cells with autologous APCs in such a manner as to favor the growth of the T cells that have antigenic specificity for the mutated p53 amino acid sequence over the T cells that do not have antigenic specificity for the mutated p53 amino acid sequence. Accordingly, a population of T cells is provided that has a higher proportion of T cells that have antigenic specificity for the mutated p53 amino acid sequence as compared to T cells that do not have antigenic specificity for the mutated p53 amino acid sequence.

In an embodiment of the invention, the method further comprises separately co-culturing autologous T cells from each of multiple fragments of a tumor obtained from the patient with the autologous APCs that present the mutated p53 amino acid sequence as described herein with respect to other aspects of the invention, and separately assessing the T cells from each of the multiple fragments for antigenic specificity for the mutated p53 amino acid sequence, as described herein with respect to other aspects of the invention.

In an embodiment of the invention in which T cells are co-cultured with autologous APCs expressing multiple mutated p53 amino acid sequences (e.g., multiple mutated p53 amino acid sequences encoded by a TMG construct or multiple mutated p53 amino acid sequences in a pool of peptides pulsed onto autologous APCs), selecting the autologous T cells may further comprise separately assessing autologous T cells for antigenic specificity for each of the multiple mutated p53 amino acid sequences. For example, the inventive method may further comprise separately inducing autologous APCs of the patient to present each mutated p53 amino acid sequence encoded by the construct (or included in the pool), as described herein with respect to other aspects of the invention (for example, by providing separate APC populations, each presenting a different mutated p53 amino acid sequence encoded by the construct (or included in the pool)). The method may further comprise separately co-culturing autologous T cells of the patient with the different populations of autologous APCs that present each mutated p53 amino acid sequence, as described herein with respect to other aspects of the invention. The method may further comprise separately selecting the autologous T cells that (a) were co-cultured with the autologous APCs that present the mutated p53 amino acid sequence and (b) have antigenic specificity for the mutated p53 amino acid sequence presented in the context of a MHC molecule expressed by the patient, as described herein with respect to other aspects of the invention. In this regard, the method may comprise determining which mutated p53 amino acid sequence encoded by a TMG construct that encodes multiple mutated p53 amino acid sequences (or included in the pool) are immunologically recognized by the autologous T cells (e.g., by process of elimination).

In an embodiment of the invention, the method further comprises expanding the numbers of selected autologous T cells to obtain a population of T cells that have antigenic specificity for the mutated p53 amino acid sequence encoded by the cancer-specific p53 mutation. Expansion of the numbers of selected cells can be accomplished by any of a number of methods as are known in the art as described in, for example, U.S. Patent 8,034,334; U.S. Patent 8,383,099; U.S. Patent Application Publication No. 2012/0244133; Dudley et al., J. Immunother., 26:332-42 (2003); and Riddell et al., J. Immunol. Methods, 128:189-201 (1990). In an embodiment, expansion of the numbers of T cells is carried out by culturing the T cells with OKT3 antibody, IL-2, and feeder PBMC (e.g., irradiated allogeneic PBMC). In this regard, the inventive methods may, advantageously, generate a large number of T cells having antigenic specificity for the mutated p53 amino acid sequence.

The T cells isolated by the inventive methods may be useful for preparing cells for adoptive cell therapies. In this regard, an embodiment of the invention provides a method of preparing a population of T cells that have antigenic specificity for a mutated human p53 amino acid sequence encoded by a cancer-specific p53 mutation, the method comprising isolating T cells as described herein with respect to other aspects of the invention, and expanding the numbers of selected autologous T cells to obtain a population of T cells that have antigenic specificity for the mutated human p53 amino acid sequence encoded by the cancer-specific mutation. Expanding the numbers of selected cells may be carried out as described herein with respect to other aspects of the invention.

Another embodiment of the invention provides a method of isolating a T cell receptor (TCR), or an antigen-binding portion thereof, having antigenic specificity for a mutated human p53 amino acid sequence encoded by a cancer-specific p53 mutation. The method comprises isolating T cells having antigenic specificity for a mutated human p53 amino acid sequence encoded by a cancer-specific p53 mutation as described herein with respect to other aspects of the invention.

The method further comprises isolating a nucleotide sequence that encodes the TCR, or the antigen-binding portion thereof, from the selected T cells, wherein the TCR, or the antigen-binding portion thereof, has antigenic specificity for the mutated human p53 amino acid sequence encoded by the cancer-specific p53 mutation. In an embodiment of the invention, prior to isolating the nucleotide sequence that encodes the TCR, or the antigen-binding portion thereof, the numbers selected T cells that have antigenic specificity for the mutated p53 amino acid sequence may be expanded. Expanding the numbers of selected cells may be carried out as described herein with respect to other aspects of the invention. In another embodiment of the invention, the numbers of selected T cells that have antigenic specificity for the mutated p53 amino acid sequence are not expanded prior to isolating the nucleotide sequence that encodes the TCR, or the antigen-binding portion thereof. For example, the TCR, or antigen binding portion thereof, may be isolated from a single cell.

The "the antigen-binding portion" of the TCR, as used herein, refers to any portion comprising contiguous amino acids of the TCR of which it is a part, provided that the antigen-binding portion specifically binds to the mutated p53 amino acid sequence as described herein with respect to other aspects of the invention. The term "antigen-binding portion" refers to any part or fragment of the TCR, which part or fragment retains the biological activity of the TCR of which it is a part (the parent TCR). Antigen-binding portions encompass, for example, those parts of a TCR that retain the ability to specifically bind to the mutated p53 amino acid sequence, or detect, treat, or prevent cancer, to a similar extent, the same extent, or to a higher extent, as compared to the parent TCR. In reference to the parent TCR, the functional portion can comprise, for instance, about 10%, about 25%, about 30%, about 50%, about 68%, about 80%, about 90%, about 95%, or more, of the parent TCR.

The antigen-binding portion can comprise an antigen-binding portion of either or both of the α and β chains of the TCR, such as a portion comprising one or more of the complementarity determining region (CDR)1, CDR2, and CDR3 of the variable region(s) of the α chain and/or β chain of the TCR. In an embodiment of the invention, the antigen-binding portion can comprise the amino acid sequence of the CDR1 of the α chain (CDR1α), the CDR2 of the α chain (CDR2α), the CDR3 of the α chain (CDR3α), the CDR1 of the β chain (CDR1β), the CDR2 of the β chain (CDR2β), the CDR3 of the β chain (CDR3β), or any combination thereof. Preferably, the antigen-binding portion comprises the amino acid sequences of CDR1α, CDR2α, and CDR3α; the amino acid sequences of CDR1β, CDR2β, and CDR3β; or the amino acid sequences of all of CDR1α, CDR2α, CDR3α, CDR1β, CDR2β, and CDR3β of the TCR.

In an embodiment of the invention, the antigen-binding portion can comprise, for instance, the variable region of the TCR comprising a combination of the CDR regions set forth above. In this regard, the antigen-binding portion can comprise the amino acid sequence of the variable region of the α chain (Vα), the amino acid sequence of the variable region of the β chain (Vβ), or the amino acid sequences of both of the Vα and Vβ of the TCR.

In an embodiment of the invention, the antigen-binding portion may comprise a combination of a variable region and a constant region. In this regard, the antigen-binding portion can comprise the entire length of the α or β chain, or both of the α and β chains, of the TCR.

Isolating the nucleotide sequence that encodes the TCR, or the antigen-binding portion thereof, from the selected T cells may be carried out in any suitable manner known in the art. For example, the method may comprise isolating RNA from the selected T cells and sequencing the TCR, or the antigen-binding portion thereof, using established molecular cloning techniques and reagents such as, for example, 5' Rapid Amplification of cDNA Ends (RACE) polymerase chain reaction (PCR) using TCR-α and -β chain constant primers. Alternatively or additionally, techniques and systems useful for isolating a TCR nucleic acid sequence may include, for example, single-cell reverse transcription polymerase chain reaction (RT-PCR), bioinfomatic reconstruction, limiting dilution cloning, the FLUIDIGM system (Fluidigm Corporation, South San Francisco, CA), deep sequencing of TCR α and TCR β chains with frequency pairing, PAIRSEQ method of identifying TCR α and β chain pairs from Adaptive Biotechnologies (Seattle, WA), and combinations thereof.

In an embodiment of the invention, the method may comprise cloning the nucleotide sequence that encodes the TCR, or the antigen-binding portion thereof, into a recombinant expression vector using established molecular cloning techniques as described in, e.g., Green et al. (Eds.), Molecular Cloning: A Laboratory Manual, Cold Spring Harbor Laboratory Press; 4th Ed. (2012). For purposes herein, the term "recombinant expression vector" means a genetically-modified oligonucleotide or polynucleotide construct that permits the expression of an mRNA, protein, polypeptide, or peptide by a host cell, when the construct comprises a nucleotide sequence encoding the mRNA, protein, polypeptide, or peptide, and the vector is contacted with the cell under conditions sufficient to have the mRNA, protein, polypeptide, or peptide expressed within the cell. The vectors may not be naturally-occurring as a whole. However, parts of the vectors can be naturally-occurring. The recombinant expression vectors can comprise any type of nucleotides, including, but not limited to DNA (e.g., complementary DCA (cDNA)) and RNA, which can be single-stranded or double-stranded, synthesized or obtained in part from natural sources, and which can contain natural, non-natural or altered nucleotides. The recombinant expression vectors can comprise naturally-occurring, non-naturally-occurring internucleotide linkages, or both types of linkages. Preferably, the non-naturally occurring or altered nucleotides or internucleotide linkages does not hinder the transcription or replication of the vector.

The recombinant expression vector can be any suitable recombinant expression vector, and can be used to transform or transfect any suitable host cell. Suitable vectors include those designed for propagation and expansion or for expression or both, such as plasmids and viruses. The vector can be selected from the group consisting of transposon/transposase, the pUC series (Fermentas Life Sciences), the pBluescript series (Stratagene, LaJolla, CA), the pET series (Novagen, Madison, WI), the pGEX series (Pharmacia Biotech, Uppsala, Sweden), and the pEX series (Clontech, Palo Alto, CA). Bacteriophage vectors, such as λGT10, λGT11, λZapII (Stratagene), λEMBL4, and λNM1149, also can be used. Examples of plant expression vectors include pBI01, pBI101.2, pBI101.3, pBI121 and pBIN19 (Clontech). Examples of animal expression vectors include pEUK-Cl, pMAM and pMAMneo (Clontech). Preferably, the recombinant expression vector is a viral vector, e.g., a retroviral vector.

The TCR, or the antigen-binding portion thereof, isolated by the inventive methods may be useful for preparing cells for adoptive cell therapies. In this regard, an embodiment of the invention provides a method of preparing a population of cells that express a TCR, or an antigen-binding portion thereof, having antigenic specificity for a mutated human p53 amino acid sequence encoded by a cancer-specific p53 mutation, the method comprising isolating a TCR, or an antigen-binding portion thereof, as described herein with respect to other aspects of the invention, and introducing the nucleotide sequence encoding the isolated TCR, or the antigen-binding portion thereof, into host cells to obtain cells that express the TCR, or the antigen-binding portion thereof.

Introducing the nucleotide sequence (e.g., a recombinant expression vector) encoding the isolated TCR, or the antigen-binding portion thereof, into host cells may be carried out in any of a variety of different ways known in the art as described in, e.g., Green et al. *supra.* Non-limiting examples of techniques that are useful for introducing a nucleotide sequence into host cells include transformation, transduction, transfection, transposition, and electroporation.

The host cell into which the nucleotide sequence encoding the TCR, or antigen binding portion thereof, is introduced may be any type of cell that can contain the recombinant expression vector. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. Suitable host cells are known in the art and include, for instance, DH5α E. coli cells, Chinese hamster ovarian cells, monkey VERO cells, COS cells, HEK293 cells, and the like. For purposes of amplifying or replicating the recombinant expression vector, the host cell is preferably a prokaryotic cell, e.g., a DH5α cell. For purposes of producing the TCR, or antigen binding portion thereof, the host cell is preferably a mammalian cell. Most preferably, the host cell is a human cell. While the host cell can be of any cell type, can originate from any type of tissue, and can be of any developmental stage, the host cell preferably is a PBL or a PBMC. More preferably, the host cell is a T cell.

In an embodiment of the invention, the PBMC include T cells. The T cells may be any type of T cell. Without being bound to a particular theory or mechanism, it is believed that less differentiated, "younger" T cells may be associated with any one or more of greater *in vivo* persistence, proliferation, and antitumor activity as compared to more differentiated, "older" T cells. Accordingly, the inventive methods may, advantageously, identify and isolate a TCR, or an antigen-binding portion thereof, that has antigenic specificity for the mutated p53 amino acid sequence and introduce the TCR, or an antigen-binding portion thereof, into "younger" T cells that may provide any one or more of greater *in vivo* persistence, proliferation, and antitumor activity as compared to "older" T cells (e.g., effector cells in a patient's tumor).

In an embodiment of the invention, the inventive methods identify and isolate TCRs, or the antigen-binding portions thereof, that have antigenic specificity against a mutated p53 amino acid sequence that is encoded by a recurrent (also referred to as "hot-spot") cancer-specific p53 mutation. In this regard, the method may comprise introducing the nucleotide sequence encoding the isolated TCR, or the antigen-binding portion thereof, into host cells that are allogeneic to the patient. For example, the method may comprise introducing the nucleotide sequence encoding the isolated TCR, or the antigen-binding portion thereof, into the host cells from another patient whose tumors express the same p53 mutation in the context of the same MHC molecule.

In an embodiment of the invention, the method further comprises expanding the numbers of host cells that express the TCR, or the antigen-binding portion thereof. The numbers of host cells may be expanded, for example, as described herein with respect to other aspects of the invention. In this regard, the inventive methods may, advantageously, generate a large number of T cells having antigenic specificity for the mutated p53 amino acid sequence.

Another embodiment that does not form part of the claimed invention provides an isolated population of cells prepared according to any of the methods described herein with respect to other aspects of the invention. The population of cells can be a heterogeneous population comprising the T cells having antigenic specificity for the mutated p53 amino acid sequence encoded by the cancer-specific p53 mutation in addition to at least one other cell, e.g., a PBMC which does not have antigenic specificity for the mutated p53 amino acid sequence encoded by the cancer-specific p53 mutation, or a cell other than a T cell, e.g., a B cell, a macrophage, a neutrophil, an erythrocyte, a hepatocyte, an endothelial cell, an epithelial cells, a muscle cell, a brain cell, etc. Alternatively, the population of cells can be a substantially homogeneous population, in which the population comprises mainly of (e.g., consisting essentially of) T cells having antigenic specificity for the mutated p53 amino acid sequence encoded by the cancer-specific p53 mutation. The population also can be a clonal population of cells, in which all cells of the population are clones of a single T cell, such that all cells of the population have antigenic specificity for the mutated p53 amino acid sequence encoded by the cancer-specific p53 mutation. In one embodiment that does not form part of the claimed invention, the population of cells is a clonal population comprising T cell having antigenic specificity for the mutated p53 amino acid sequence encoded by the cancer-specific p53 mutation, as described herein. In an embodiment that does not form part of the claimed invention, about 1% to about 100%, for example, about 1%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, or about 100%, or a range defined by any two of the foregoing values, of the population of cells comprises T cells that have antigenic specificity for the mutated p53 amino acid sequence. Without being bound to a particular theory or mechanism, it is believed that populations of cells that comprise a high proportion of T cells that have antigenic specificity for the mutated p53 amino acid sequence advantageously may have a lower proportion of irrelevant cells that may hinder the function of the T cell, e.g., the ability of the T cell to target the destruction of cancer cells and/or treat or prevent cancer.

The non-claimed populations of cells can be formulated into a composition, such as a pharmaceutical composition. In this regard, an embodiment that does not form part of the claimed invention provides a pharmaceutical composition comprising any of the non-claimed populations of cells and a pharmaceutically acceptable carrier. The non-claimed pharmaceutical composition can comprise anon-claimed population of cells in combination with another pharmaceutically active agent(s) or drug(s), such as a chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc.

Preferably, the carrier is a pharmaceutically acceptable carrier. With respect to pharmaceutical compositions, the carrier can be any of those conventionally used for the particular non-claimed population of cells under consideration. Such pharmaceutically acceptable carriers are well-known to those skilled in the art and are readily available to the public. It is preferred that the pharmaceutically acceptable carrier be one which has no detrimental side effects or toxicity under the conditions of use.

The choice of carrier will be determined in part by the particular non-claimed population of cells, as well as by the particular method used to administer the non-claimed population of cells. Accordingly, there are a variety of suitable formulations of the non-claimed pharmaceutical composition. Suitable formulations may include any of those for parenteral, subcutaneous, intravenous, intramuscular, intraarterial, intratumoral, intrathecal, or interperitoneal administration. More than one route can be used to administer the non-claimed population of cells, and in certain instances, a particular route can provide a more immediate and more effective response than another route.

Preferably, the non-claimed population of cells is administered by injection, e.g., intravenously. When the non-claimed population of cells is to be administered, the pharmaceutically acceptable carrier for the cells for injection may include any isotonic carrier such as, for example, normal saline (about 0.90% w/v of NaCl in water, about 300 mOsm/L NaCl in water, or about 9.0 g NaCl per liter of water), NORMOSOL R electrolyte solution (Abbott, Chicago, IL), PLASMA-LYTE A (Baxter, Deerfield, IL), about 5% dextrose in water, or Ringer's lactate. In an embodiment that does not form part of the claimed invention, the pharmaceutically acceptable carrier is supplemented with human serum albumin.

It is contemplated that the non-claimed populations of cells and pharmaceutical compositions can be used in methods of treating or preventing cancer. Without being bound to a particular theory or mechanism, the non-claimed T cells are believed to bind specifically to a mutated p53 amino acid sequence encoded by a cancer-specific p53 mutation, such that a TCR expressed by the cell is able to mediate an immune response against a target cell expressing the mutated p53 amino acid sequence. In this regard, an embodiment that does not form part of the claimed invention provides a method of treating or preventing cancer in a mammal, comprising administering to the mammal any of the pharmaceutical compositions or populations of cells described herein, in an amount effective to treat or prevent cancer in the mammal.

The terms "treat," and "prevent" as well as words stemming therefrom, as used herein, do not necessarily imply 100% or complete treatment or prevention. Rather, there are varying degrees of treatment or prevention of which one of ordinary skill in the art recognizes as having a potential benefit or therapeutic effect. In this respect, the non-claimed methods can provide any amount of any level of treatment or prevention of cancer in a mammal. Furthermore, the treatment or prevention provided by the non-claimed method can include treatment or prevention of one or more conditions or symptoms of the cancer being treated or prevented. For example, treatment or prevention can include promoting the regression of a tumor. Also, for purposes herein, "prevention" can encompass delaying the onset of the cancer, or a symptom or condition thereof.

For purposes not forming part of the claimed invention, the amount or dose of the non-claimed population of cells or pharmaceutical composition administered (e.g., numbers of cells when the non-claimed population of cells is administered) should be sufficient to effect, e.g., a therapeutic or prophylactic response, in the mammal over a reasonable time frame. For example, the dose of the non-claimed population of cells or pharmaceutical composition should be sufficient to bind to a mutated p53 amino acid sequence encoded by a cancer-specific p53 mutation, or detect, treat or prevent cancer in a period of from about 2 hours or longer, e.g., 12 to 24 or more hours, from the time of administration. In certain embodiments that do not form part of the claimed invention, the time period could be even longer. The dose will be determined by the efficacy of the particular non-claimed population of cells or pharmaceutical composition administered and the condition of the mammal (e.g., human), as well as the body weight of the mammal (e.g., human) to be treated.

Many assays for determining an administered dose are known in the art. For purposes that do not form part of the claimed invention, an assay, which comprises comparing the extent to which target cells are lysed or IFN-y is secreted by T cells upon administration of a given dose of such T cells to a mammal among a set of mammals of which is each given a different dose of the T cells, could be used to determine a starting dose to be administered to a mammal. The extent to which target cells are lysed or IFN-y is secreted upon administration of a certain dose can be assayed by methods known in the art.

The dose of the non-claimed population of cells or pharmaceutical composition also will be determined by the existence, nature and extent of any adverse side effects that might accompany the administration of a particular non-claimed population of cells or pharmaceutical composition. Typically, the attending physician will decide the dosage of the non-claimed population of cells or pharmaceutical composition with which to treat each individual patient, taking into consideration a variety of factors, such as age, body weight, general health, diet, sex, non-claimed population of cells or pharmaceutical composition to be administered, route of administration, and the severity of the condition being treated.

In an embodiment that does not form part of the claimed invention in which the non-claimed population of cells is to be administered, the number of cells administered per infusion may vary, for example, in the range of one million to 100 billion cells; however, amounts below or above this exemplary range are within the scope of the non-claimed embodiment. For example, the daily dose of non-claimed host cells can be about 1 million to about 150 billion cells (e.g., about 5 million cells, about 25 million cells, about 500 million cells, about 1 billion cells, about 5 billion cells, about 20 billion cells, about 30 billion cells, about 40 billion cells, about 60 billion cells, about 80 billion cells, about 100 billion cells, about 120 billion cells, about 130 billion cells, about 150 billion cells, or a range defined by any two of the foregoing values), preferably about 10 million to about 130 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, about 100 billion cells, about 110 billion cells, about 120 billion cells, about 130 billion cells, or a range defined by any two of the foregoing values), more preferably about 100 million cells to about 130 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells, about 50 billion cells, about 75 billion cells, about 90 billion cells, about 100 billion cells, about 110 billion cells, about 120 billion cells, about 130 billion cells, or a range defined by any two of the foregoing values).

For purposes of the non-claimed methods, wherein populations of cells are administered, the cells can be cells that are allogeneic or autologous to the mammal. Preferably, the cells are autologous to the mammal.

Another embodiment that does not form part of the claimed invention provides any of the isolated population of cells or pharmaceutical compositions described herein for use in treating or preventing cancer in a mammal.

With respect to the inventive methods, the cancer can be any cancer, including any of acute lymphocytic cancer, acute myeloid leukemia, alveolar rhabdomyosarcoma, bone cancer, brain cancer, breast cancer, cancer of the anus, anal canal, or anorectum, cancer of the eye, cancer of the intrahepatic bile duct, cancer of the joints, cancer of the neck, gallbladder, or pleura, cancer of the nose, nasal cavity, or middle ear, cancer of the oral cavity, cancer of the vagina, cancer of the vulva, chronic lymphocytic leukemia, chronic myeloid cancer, colon cancer, colocrectal cancer, endometrial cancer, esophageal cancer, uterine cervical cancer, gastrointestinal carcinoid tumor, glioma, Hodgkin lymphoma, hypopharynx cancer, kidney cancer, larynx cancer, liver cancer, lung cancer, malignant mesothelioma, melanoma, multiple myeloma, nasopharynx cancer, non-Hodgkin lymphoma, cancer of the oropharynx, ovarian cancer, cancer of the penis, pancreatic cancer, peritoneum, omentum, and mesentery cancer, pharynx cancer, prostate cancer, rectal cancer, renal cancer, skin cancer, small intestine cancer, soft tissue cancer, stomach cancer, testicular cancer, thyroid cancer, cancer of the uterus, ureter cancer, and urinary bladder cancer. In a preferred embodiment, the cancer is a cancer which expresses mutated p53. The cancer may express p53 with a mutation at one or more of positions 175, 220, 245, 248, 249, 273, or 282 of SEQ ID NO: 1. The cancer may express p53 with one or more of the following human p53 mutations: R175H, Y220C, G245D, G245S, R248L, R248Q, R248W, R249S, R273H, R273C, R273L, or R282W. Preferably, the cancer is an epithelial cancer or cholangiocarcinoma, melanoma, colon cancer, rectal cancer, ovarian cancer, endometrial cancer, non-small cell lung cancer (NSCLC), glioblastoma, uterine cervical cancer, head and neck cancer, breast cancer, pancreatic cancer, or bladder cancer.

The mammal referred to in the inventive methods can be any mammal. As used herein, the term "mammal" refers to any mammal, including, but not limited to, mammals of the order Rodentia, such as mice and hamsters, and mammals of the order Logomorpha, such as rabbits. It is preferred that the mammals are from the order Carnivora, including Felines (cats) and Canines (dogs). Preferably, the mammals are from the order Artiodactyla, including Bovines (cows) and Swines (pigs) or of the order Perssodactyla, including Equines (horses). Preferably, the mammals are of the order Primates, Ceboids, or Simoids (monkeys) or of the order Anthropoids (humans and apes). A more preferred mammal is the human.

The following examples further illustrate the invention but, of course, should not be construed as in any way limiting its scope.

### EXAMPLES

The amino acid sequences set forth in Tables 1-3 were employed in the experiments described in the following Examples.

**TABLE 1**

| **SEQ ID NO:** | **Name** | **Sequence** |
|---|---|---|
| 2 | LP-p53-R175H-MUT | YKQSQHMTEVVR**H**CPHHERCSDSDG |
| 3 | LP-p53-R273H-MUT | SGNLLGRNSFEV**H**VCACPGRDRRTE |
| 4 | LP-p53-R248L-MUT | YMCNSSCMGGMN**L**RPILTIITLEDS |
| 5 | LP-p53-R282W-MUT | FEVRVCACPGRD**W**RTEEENLRKKGE |
| 6 | LP-p53-R273C-MUT | SGNLLGRNSFEV**C**VCACPGRDRRTE |
| 7 | LP-p53-G245S-MUT | HYNYMCNSSCMG**S**MNRRPELTIITL |
| 8 | LP-p53-R248Q-MUT | YMCNSSCMGGMN**Q**RPILTIITLEDS |
| 9 | LP-p53-G245D-MUT | HYNYMCNSSCMG**D**MNRRPILTIITL |
| 10 | LP-p53-R273L-MUT | SGNLLGRNSFEV**L**VCACPGRDRRTE |
| 11 | LP-p53-R248W-MUT | YMCNSSCMGGMN**W**RPILTIITLEDS |
| 12 | LP-p53-Y220C-MUT | DRNTFRHSVVVP**C**EPPEVGSDCTTI |
| 13 | LP-p53-R249S-MUT | MCNSSCMGGMNR**S**PILTIITLEDSS |

The wild-type versions of the peptides of Table 1 are set forth in Table 2.

**TABLE 2**

| **SEQ ID NO:** | **Name** | **Sequence** |
|---|---|---|
| 15 | LP-p53-R175-WT | YKQSQHMTEVVR**R**CPHHERCSDSDG |
| 16 | LP-p53-R273-WT | SGNLLGRNSFEV**R**VCACPGRDRRTE |
| 17 | LP-p53-R248-WT | YMCNSSCMGGMN**R**RPILTIITLEDS |
| 18 | LP-p53-R282-WT | FEVRVCACPGRD**R**RTEEENLRKKGE |
| 19 | LP-p53-G245-WT | HYNYMCNSSCMG**G**MNRRPILTIITL |
| 20 | LP-p53-Y220-WT | DRNTFRHSVVVP**Y**EPPEVGSDCTTI |
| 21 | LP-p53-R249-WT | MCNSSCMGGMNR**R**PILTIITLEDSS |

**TABLE 3**

| **SEQ ID NO:** | **Name** | **Sequence** |
|---|---|---|
| 14 | TMG-p53-MUT | YKQSQHMTEVVRHCPHHERCSDSDGSNLLGRNSFEV HVCACPGRDRRTEYMCNSSCMGGMNLRPILTIITLEDSF EVRVCACPGRDWRTEEENLRKKGESGNLLGRNSFEVC VCACPGRDRRTEHYNYMCNSSCMGSMNRRPILTIITLY |
| | | MCNSSCMGGMNQRPILTIITLEDSHYNYMCNSSCMGD MNRRPILTIITLSGNLLGRNSFEVLVCACPGRDRRTEYM CNSSCMGGMNWRPILTIITLEDSDRNTFRHSVVVPCEPP EVGSDCTTIMCNSSCMGGMNRSPILTIITLEDSS |
| 22 | TMG-p53-WT | YKQSQHMTEVVRRCPHHERCSDSDGSGNLLGRNSFEV RVCACPGRDRRTEYMCNSSCMGGMNRRPILTIITLEDSF EVRVCACPGRDRRTEEENLRKKGESGNLLGRNSFEVRV CACPGRDRRTEHYNYMCNSSCMGGMNRRPILTIITLYM CNSSCMGGMNRRPILTIITLEDSHYNYMCNSSCMGGMN RRPILTIITLSGNLLGRNSFEVRVCACPGRDRRTEYMCNS SCMGGMNRRPILTIITLEDSDRNTFRHSVVVPYEPPEVGS DCTTIMCNSSCMGGMNRRPILTIITLEDSS |

### EXAMPLE 1

This example demonstrates the identification of anti-mutated p53 T cells in Patient 4141 by co-culturing autologous APCs induced to express mutated p53 within autologous T cells ("p53 hotspot mutation universal screening"). This example also demonstrates the isolation and specific reactivity of a TCR from patient 4141.

Experiments were carried out as described for Figures 1 and 45-48 for Patient 4141. TIL fragment F12 and infusion bag TIL (Rx1) from patient 4141 and p53-R175H-specific TCR or mock transduced T cells from patient 4196 were used as effectors. Co-cultures with T cell effectors and HLA-A*02:01 APCs (autologous to patient 4141) were either (1) electroporated with TMGs composed of irrelevant, WT p53, or mutated p53 sequences or (2) pulsed with peptide vehicle (DMSO) or purified (>95% by HPLC) 25 amino acid peptides composed of WT p53-R175 sequence or mutated p53-R175H sequence. T cells only (no target) was negative control and PMA and Iono was positive control (lattice bars). Co-cultures were performed overnight at 37 °C. Secretion of IFN-γ was evaluated using ELISPOT assay. The results are shown in Figure 1.

Autologous APCs were transfected with TMG encoding irrelevant mutations, WT p53 sequence, or mutated p53 sequence including R175H. Media alone and PMA and ionomycin were negative and positive controls, respectively. The following day, TIL from patient 4141 (fragment culture 12) were co-cultured overnight at 37 °C with TMG-transfected APCs. Secretion of IFN-γ was evaluated by ELISPOT. Expression of 4-1BB was evaluated by flow cytometry after gating for lymphocytes → living cells (PI negative) → CD3+ (T cells) → CD4-CD8+. The results are shown in Figure 45.

Cos7 cells (2.5x10⁴ per well) were plated on wells of flat-bottom 96 well plates. The following day, cells were co-transfected with individual HLA alleles from patient 4141 and either no extra gene, WT TP53 TMG, or mutated TP53 TMG containing the p53-R175H sequence. TIL with specificity to p53-R175H from Patient 4141 (fragment culture 12) were co-cultured the following day with transfected Cos7 cells and were incubated overnight at 37 °C. Secretion of IFN-γ was evaluated by ELISPOT. The results are shown in Figure 46.

T cells expressing mock (no TCR) or 4141-TCR1a2 were co-cultured with T2 tumor cells (expressing HLA-A*02:01). T2 cells were pulsed for 2 hours at 37 °C with peptide vehicle (DMSO) or purified (>95% by HPLC) peptides composed of WT p53-R175 peptide HMTEVVRRC (SEQ ID NO: 532) or mutated p53-R175H peptide HMTEVVRHC (SEQ ID NO: 530). Media alone and PMA and Ionomycin were negative and positive controls, respectively. Co-cultures were performed overnight at 37 °C. Secretion of IFN-γ was evaluated by ELISA. The results are shown in Figure 47.

T cells expressing 4141-TCR1a2 were co-cultured overnight at 37 °C with Saos2 cells (p53-NULL and HLA-A*02:01+), which were either unmanipulated or made to overexpress full length p53-R175H protein. Inhibitors of secretion (monensin and brefeldin A) were added to co-cultures to trap cytokines within T cells. After 6 hours of co-culture, cells were fixed and permeabilized then stained for IL-2, CD107a, IFN-γ and tumor necrosis factor-alpha (TNFα). Flow cytometry was used to analyze co-cultures based on a lymphocyte gate. The results are shown in Figure 48.

The sequence of TCR **4141-TCR1a2,** which was isolated from Patient 4141, is set forth below. Starting from the amino terminus, the first underlined region is the CDR1alpha (SEQ ID NO: 467), the second underlined region is the CDR2alpha (SEQ ID NO: 468), the third underlined region is the CDR3alpha (SEQ ID NO: 469), the fourth underlined region is the CDR1beta (SEQ ID NO: 470), the fifth underlined region is the CDR2beta (SEQ ID NO: 471), and the sixth underlined region is the CDR3beta (SEQ ID NO: 472). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 473) includes the sequence starting from the amino terminus and ending immediately prior to the start of the alpha chain constant region. The beta chain variable region (SEQ ID NO: 474) includes the sequence starting immediately after the linker and ending immediately prior to the start of the beta chain constant region. The full-length alpha chain (SEQ ID NO: 475) includes the sequence starting from the amino terminus and ending immediately prior to the start of the linker. The full-length beta chain (SEQ ID NO: 476) includes the sequence starting immediately after the linker and ending with the carboxyl terminus.

Cancer reactive T cells were identified as described below. The TCR was isolated as described below.
**TCR name: 4141-TCR1a2**
**Recognition of p53 mutation: R175H**
**Screening method: p53 "hotspot" mutation universal screening**
**Co-culture to identify TCR: Co-culture 4141 infusion bag TIL with p53mutTMG and sorted CD8+41BB+ T cells**
**Method to identify TCR: single-cell RT-PCR then TA TOPO cloning kit (Thermo Fisher Scientific, Waltham, MA) for alpha chain**
**TCR orientation: alpha-beta**
**Expression vector: SB transposon**

MASIRAVFIFLWLQLDLVNGENVEQHPSTLSVQEGDSAVIKCTYSDSASNYFPWYKQ
ELGKGPQLIIDIRSNVGEKKDQRIAVTLNKTAKHFSLHITETQPEDSAVYFCAASKSAI
MVVLQTSSSLELALCLLSSQVNIQNPEPAVYQLKDPRSQDSTLCLFTDFDSQINVPKTME
SGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWSSRAKRSGSGATNFSLLKQAGDV
EENPGPMHPQLLGYVVLCLLGAGPLEAQVTQNPRYLITVTGKKLTVTCSQNMNHEY
MSWYRQDPGLGLRQIYYSMNVEVTDKGDVPEGYKVSRKEKRNFPLILESPSPNQTSL
YFCASSIQQGADTQYFGPGTRLTVLEDLRNVTPPKVSLFEPSKAEIANKQKATLVCLARG
FFPDHVELSWWVNGKEVHSGVCTDPQAYKESNYSYCLSSRLRVSATFWHNPRNHFRCQV

QFHGLSEEDKWPEGSPKPVTQNISAEAWGRADCGITSASYQQGVLSATILYEILLGKATLYA
VLVSTLVVMAMVKRKNS (SEQ ID NO: 587)

The statistics for 4141-TCR1a2 from patient 4141 are set forth in Table 4 below.

**TABLE 4**

| **Parameter** | **#** | **Frequency** |
|---|---|---|
| Total wells | 96 | 100% |
| CDR3alpha | Unknown (TA TOPO cloning) | Not applicable |
| CDR3beta | 58 | 60.4% |

### EXAMPLE 2

This example demonstrates the identification of anti-mutated p53 T cells in Patient 4130 by co-culturing autologous APCs induced to express mutated p53 within autologous T cells ("p53 hotspot mutation universal screening").

Experiments were carried out as described for Figure 2 for Patient 4130. TIL fragments (F14, F20 and F24) from patient 4130 were co-cultured with autologous APCs (1) electroporated with TMGs composed of irrelevant, WT p53 or mutated p53 sequences or (2) pulsed with peptide vehicle (DMSO) or purified (>95% by HPLC) 25 amino acid peptides composed of WT p53-R273 sequence or mutated p53-R273H sequence. Co-cultures were performed overnight at 37 °C. Expression of 4-1BB was evaluated by flow cytometry after gating for lymphocytes → living cells (PI negative) → CD3+ (T cells). The results are shown in Figure 2.

### EXAMPLE 3

This example demonstrates the identification of anti-mutated p53 T cells in Patient 4259 by co-culturing autologous APCs induced to express mutated p53 within autologous T cells ("p53 hotspot mutation universal screening"). This example also demonstrates the isolation and specific reactivity of a TCR isolated from patient 4259.

Experiments were carried out as described for Figures 3-8 and 49-53 for Patient 4259. TIL fragments (n=18) from patient 4259 were co-cultured with autologous APCs electroporated with TMG composed of irrelevant, WT p53 or mutated p53 sequences. Co-cultures were performed overnight at 37 °C. Secretion of IFN-γ was evaluated using ELISPOT assay. The results are shown in Figure 3. Expression of 4-1BB was evaluated by flow cytometry after gating for lymphocytes → living cells (PI negative) → CD3+ (T cells). The results are shown in Figures 4-5.

TIL fragments (n=18) from patient 4259 were co-cultured with autologous APCs pulsed with peptide vehicle (DMSO) or purified (>95% by HPLC) 25 amino acid peptides composed of WT p53-Y220 sequence or mutated p53-Y220C sequence. Co-cultures were performed overnight at 37 °C. Secretion of IFN-γ was evaluated using ELISPOT assay. The results are shown in Figure 6. Expression of 4-1BB was evaluated by flow cytometry after gating for lymphocytes → living cells (PI negative) → CD3+ (T cells). The results are shown in Figures 7-8.

TIL fragment culture (no. 6) from patient 4259 was co-cultured with autologous APCs either (1) electroporated with TMG composed of irrelevant, WT p53, or mutated p53 sequence or (2) pulsed with peptide vehicle (DMSO) or purified (>95% by HPLC) 25-amino acid peptides composed of WT p53-Y220 sequence or mutated p53-Y220C sequence. Co-cultures were performed overnight at 37 °C. Expression of 4-1BB was evaluated by flow cytometry after gating for lymphocytes → living cells (PI negative) → CD3+ (T cells). The results are shown in Figure 49.

Autologous APCs were pulsed with decreasing concentrations of 25-amino acid peptides corresponding to the WT p53-Y220 or mutated p53-Y220C sequence for 2 hours at 37 °C. TIL fragment culture (no. 6) from patient 4259 was co-cultured with peptide-pulsed APCs. Expression of 4-1BB was assayed by flow cytometry after gating lymphocytes → living cells (PI negative) → CD3+ (T cells). The results are shown in Figure 50.

Autologous antigen presenting cells were pulsed with DMSO, WT p53-Y220 peptide RNTFRHSVVVPYE (SEQ ID NO: 533) or mutated p53-Y220C peptide RNTFRHSVVVPCE (SEQ ID NO: 534) for 2 hours at 37 °C. Excess peptide was washed away. TIL from patient 4259 (fragment culture 6) with specificity to p53-Y220C was co-cultured overnight at 37 °C with peptide-pulsed APCs. Expression of 4-1BB was evaluated by flow cytometry after gating for lymphocytes → living cells (PI negative) → CD3+ (T cells). The results are shown in Figure 51.

Cos7 cells (2.5x10⁴ per well) were plated on wells of flat-bottom 96 well plates. The following day, cells were co-transfected with individual HLA alleles from patient 4259. The next day, DMSO or the p53-Y220C peptide RNTFRHSVVVPCE (SEQ ID NO: 534) were pulsed for 2 hours on transfected Cos7 cells. Excess peptide was washed away. TIL fragment culture no.6 from Patient 4259 was added (10⁵ cells/well). Co-cultures were incubated overnight at 37 °C. Expression of 4-1BB was assayed by flow cytometry after gating lymphocytes → live → CD3+ (T cells) → CD8-CD4+. The results are shown in Figure 52.

APCs autologous to Patient 4259 were pulsed with 25-amino acid peptides corresponding to the WT p53-Y220 or mutated p53-Y220C sequence for 2 hours at 37 °C. Excess peptide was washed away. T cells expressing 4259-F6-TCR were co-cultured overnight at 37 °C with peptide-pulsed APCs. Expression of 4-1BB was evaluated by flow cytometry after gating for lymphocytes → living cells (PI negative) → CD3+ (T cells). The introduced TCR was measured by mouse TCRbeta (mTCR). The results are shown in Table 5.

**TABLE 5**

| | **WT p53**-**Y220** | **Mut p53-Y220C** |
|---|---|---|
| 4-1BB+/mTCR+ | 0.27 | 5.22 |
| 4-1BB-/mTCR- | 68.5 | 73.7 |
| 4-1BB+/mTCR- | 0.18 | 1.59 |
| 4-1BB-/mTCR+ | 31.0 | 19.5 |

A tumor cell (TC) line was established from a xenografted tumor fragment resected from Patient 4259 and then serially passaged through immunocompromised mice (TC#4259). TC#4259 was co-cultured with T cells (10⁵) expressing mock (no TCR) or p53-Y220C-specific TCR (4259-F6-TCR) overnight at 37 °C. The TC#4259 cells were either incubated with nothing, W6/32 pan-HLA Class-I specific blocking antibody, IVA12 pan-HLA Class-II specific blocking antibody or mutated p53-Y220C peptide RNTFRHSVVVPCE (SEQ ID NO: 534) for 2 hours at 37 °C. The antibodies were kept in the co-culture at 5 µg/mL final concentration. The peptide was incubated at 10 µg/mL and excess peptide was washed after incubation. Media alone (no TC) and PMA and Ionomycin were negative and positive controls, respectively. Expression of 4-1BB was evaluated by flow cytometry after gating for lymphocytes → living cells (PI negative) → CD3+ (T cells). The results are shown in Figure 53.

The sequence of TCR **4259-F6-TCR,** which was isolated from Patient 4259, is set forth below. Starting from the amino terminus, the first underlined region is the CDR1alpha (SEQ ID NO: 477), the second underlined region is the CDR2alpha (SEQ ID NO: 478), the third underlined region is the CDR3alpha (SEQ ID NO: 479), the fourth underlined region is the CDR1beta (SEQ ID NO: 480), the fifth underlined region is the CDR2beta (SEQ ID NO: 481), and the sixth underlined region is the CDR3beta (SEQ ID NO: 482). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 483) includes the sequence starting from the amino terminus and ending immediately prior to the start of the alpha chain constant region. The beta chain variable region (SEQ ID NO: 484) includes the sequence starting immediately after the linker and ending immediately prior to the start of the beta chain constant region. The full-length alpha chain (SEQ ID NO: 485) includes the sequence starting from the amino terminus and ending immediately prior to the start of the linker. The full-length beta chain (SEQ ID NO: 486) includes the sequence starting immediately after the linker and ending with the carboxyl terminus.

Cancer reactive T cells were identified as described below. The TCR was isolated as described below.
TCR name: 4259-F6-TCR
Recognition of p53 mutation: Y220C
Screening method: p53 "hotspot" mutation universal screening
**Co-culture to identify TCR: Co-culture 4259-F6 with p53-Y220C peptide and sorted CD4+41BB+ T cells**
**Method to identify TCR: single-cell RT-PCR**
**Abundance of TCR amongst all paired TCRs: 81.1% (observed 36 times of 44 pairs) TCR orientation: alpha-beta**
**Expression vector: SB transposon**

MASLRVLLVILWLQLSWVWSQQKEVEQNSGPLSVPEGAIASLNCTYSDRGSQSFFW
YRQYSGKSPELIMFIYSNGDKEDGRFTAQLNKASQYVSLLIRDSQPSDSATYLCAWN
SGGSNYKLTFGKGTLLTVNPNIQNPEPAVYQLKDPRSQDSTLCLFTDFDSQINVPKTMES
GTFITDKCVLDMKAMDSKSNGAIAWSNQTSFTCQDIFKETNATYPSSDVPCDATLTEKSFE
TDMNLNFQNLLVIVLRILLLKVAGFNLLMTLRLWSSRAKRSGSGATNFSLLKQAGDVE
ENPGPMHLGLLCCGAFSLLWAGPVNAGVTQTPKFRVLKTGQSMTLLCAQDMNHEY
MYWYRQDPGMGLRLIHYSVGEGTTAKGEVPDGYNVSRLKKQNFLLGLESAAPSQTS
VYFCASSYSQAWGQPQHFGDGTRLSILEDLRNVTPPKVSLFEPSKAEIANKQKATLVCL
ARGFFPDHVELSWWVNGKEVHSGVCTDPQAYKESNYSYCLSSRLRVSATFWHNPRNHFR
CQVQFHGLSEEDKWPEGSPKPVTQNISAEAWGRADCGITSASYQQGVLSATILYEILLGKA
TLYAVLVSTLVVMAMVKRKNS (SEQ ID NO: 588)

The statistics for **4259-F6-TCR** from patient 4259 are set forth in Table 6 below.

**TABLE 6**

| **Parameter** | **#** | **Frequency** |
|---|---|---|
| Total wells | 96 | 100% |
| CDR3alpha | 41 | 42.7% |
| CDR3beta | 47 | 49.0% |
| 4259-F6-TCR pairs | 36 | 37.5% |
| Total paired TCRs | 44 | 45.8% |

### EXAMPLE 4

This example demonstrates the identification of anti-mutated p53 T cells in Patient 4127 by co-culturing autologous APCs induced to express mutated p53 within autologous T cells ("p53 hotspot mutation universal screening").

Experiments were carried out as described for Figures 9-10 for Patient 4127. TIL from patient 4127 were co-cultured with allogeneic (DRB3*01:01:01 or DRB3 *02:02:01) APCs which were (1) electroporated with TMGs composed of irrelevant, WT p53 or mutated p53 sequences or (2) pulsed with peptide vehicle (DMSO) or purified (>95% by HPLC) 25 amino acid peptides composed of WT p53-G245 sequence or mutated p53-G245S sequence. Co-cultures were performed overnight at 37 °C. Secretion of IFN-γ was evaluated using ELISPOT assay. The results are shown in Figure 9.

T cells expressing the 4127-TCR1 specific for p53-G245S were co-cultured with allogeneic (DRB3*01:01:01 or DRB3*02:02:01) APCs which were (1) electroporated with TMGs composed of irrelevant, WT p53 or mutated p53 sequences or (2) pulsed with peptide vehicle (DMSO) or purified (>95% by HPLC) 25 amino acid peptides composed of WT p53-G245 sequence or mutated p53-G245S sequence. Co-cultures were performed overnight at 37 °C. Secretion of IFN-γ was evaluated using ELISPOT assay. The results are shown in Figure 10.

### EXAMPLE 5

T cells from 27 patients were screened by (i) inducing autologous antigen presenting cells (APCs) of a patient to present at least one mutated p53 amino acid sequence; (ii) co-culturing autologous T cells of the patient with the autologous APCs that present the mutated p53 amino acid sequence; and (iii) selecting the autologous T cells that (a) were co-cultured with the autologous APCs that present the mutated p53 amino acid sequence. A summary of responses to p53 "hostpot" mutations by T cells using the screening method is provided in Table 7.

**TABLE 7**

| **Mutation** | **# Pt** | **% Pt** | **# tested** | **# reactive** | **% reactive of screened** |
|---|---|---|---|---|---|
| R175H | 7 | 19% | 6 | 4 | 66.7% |
| Y220C | 3 | 8% | 3 | 2 | 66.7% |
| G245D | 1 | 3% | 1 | 0 | 0.0% |
| G245S | 2 | 6% | 1 | 1 | 100.0% |
| R248L | 0 | 0% | 0 | 0 | Not applicable |
| R248Q | 6 | 17% | 6 | 3 | 50.0% |
| R248W | 6 | 17% | 5 | 3 | 60.0% |
| R249S | 0 | 0% | 0 | 0 | Not applicable |
| R273C | 2 | 6% | 1 | 0 | 0.0% |
| R273H | 5 | 14% | 5 | 1 | 20.0% |
| R273L | 0 | 0% | 0 | 0 | Not applicable |
| R282W | 4 | 11% | 3 | 2 | 66.7% |
| Total | 36 | 100% | 31 | 16 | 51.6% |

### EXAMPLE 6

This example demonstrates the identification of anti-mutated p53 T cells in Patient 4273 by co-culturing autologous APCs induced to express mutated p53 within autologous T cells ("p53 hotspot mutation universal screening"). This example also demonstrates the isolation of two anti-mutated p53 TCRs from patient 4273.

Experiments were carried out as described for Figures 11-14 and 33-37 for Patient 4273. TIL fragments (F1-F24, n=24) from patient 4273 were co-cultured with autologous APCs electroporated with TMG composed of irrelevant, WT p53 or mutated p53 sequence. Co-cultures were performed overnight at 37 °C. Secretion of IFN-γ was evaluated using ELISPOT assay. The results are shown in Figure 11. Expression of 4-1BB was evaluated by flow cytometry after gating for lymphocytes → living cells (PI negative) → CD3+ (T cells). The results are shown in Figure 12.

TIL fragments (F1-F24, n=24) from patient 4273 were co-cultured with autologous APCs pulsed with peptide vehicle (DMSO) or purified (>95% by HPLC) 25-amino acid peptides composed of WT p53-R248 sequence or mutated p53-R248W sequence. Co-cultures were performed overnight at 37 °C. Secretion of IFN-γ was evaluated using ELISPOT assay. The results are shown in Figure 13. Expression of 4-1BB was evaluated by flow cytometry after gating for lymphocytes → living cells (PI negative) → CD3+ (T cells). The results are shown in Figure 14.

For patient 4273, F15 was the most reactive fragment and the responses by F15 to the LP and TMG were comparable and primarily by CD4 T cells. So 4273-F15 was co-cultured with APCs pulsed with the R248W LP, and the following day we sorted CD4+41BB+ T cells as single cells into wells a 96 well PCR plate at one cell per well. The PCR plate has an RT-PCR solution in each well, which amplifies the TCR alpha and beta CDR3 regions in the same solution. The wells of the plate are then split into two 96 well PCR plates and a second PCR round is performed to amplify either the CDR3 alpha or the CDR3 beta as separate reactions. The PCR products from each well (total of 192 PCR products mapped to each well - alpha or beta) are sequenced by Sanger sequencing. The nucleotide sequence is inputted into IMGT/V-QUEST (imgt.org/IMGT_vquest/vquest?livret=0&Option=humanTcR), IgBlast (ncbi.nlm.nih.gov/igblast/igblast.cgi?CMD=Web&SEARCH_TYPE=TCR&LINK_LOC=igt ab) and translated by Expasy (web.expasy.org/translate/). The variable family is determined and fused to the CDR3 and junction (J or DJ) from the translated sequence. The variable sequence is fused to the murine constant sequence and the reconstructed TCRalpha and TCRbeta are linked by furin-felxible-P2A (RAKR-SGSG-ATNFSLLKQAGDVEENPGP) (SEQ ID NO: 26). Then the sequence is synthesized into DNA de novo and cloned into an expression vector (gamma-retrovirus or SLEEPING BEAUTY (SB) transposon (University of Minnesota, Minneapolis, MN)). T cells are then made to express the TCR using the standard viral transduction or non-viral transposition protocols and T cells expressing murinized TCRs (as detected by mouse TCR beta constant chain) are tested against the putative peptide.

Autologous APCs were transfected with TMG encoding irrelevant mutations, WT p53 sequences or mutated p53 sequences including p53-R248W. Media alone and PMA and Ionomycin were negative and positive controls, respectively. TIL from patient 4273 (fragment cultures 8 and 15) were co-cultured overnight at 37 °C with TMG transfected APCs. Expression of 4-1BB was evaluated by flow cytometry after gating for lymphocytes → living cells (PI negative) → CD3+ (T cells). The results are shown in Figure 33.

Autologous APCs were pulsed with 25 amino acid peptides corresponding to the WT or mutated p53-R248W neoepitope for 2 hours at 37 °C. TIL from patient 4273 (fragment culture 15) with specificity to p53-R248W were co-cultured overnight at 37 °C with peptide-pulsed APCs. DMSO was peptide vehicle. Expression of 4-1BB was evaluated by flow cytometry after gating for lymphocytes → living cells (PI negative) → CD3+ (T cells). The results are shown in Figure 34.

Autologous APCs were pulsed with 15 amino acid peptides from the p53-R248W neoepitope overlapping 14 amino acids. TIL from patient 4273 (fragment culture 15) with specificity to p53-R248W were co-cultured overnight at 37 °C with peptide-pulsed APCs. DMSO was peptide vehicle, media alone (T cells only) and PMA and ionomycin were controls. The 25 amino acid peptides (wt p53-R248 and mutated p53-R248W) were additional controls for the 15 amino acid peptides. Expression of 4-1BB was evaluated by flow cytometry after gating for lymphocytes → living cells (PI negative) → CD3+ (T cells) → CD4+CD8-. The results are shown in Figure 35.

Cos7 cells (2.5×10⁴ per well) were plated on wells of flat-bottom 96 well plates. The following day, cells were co-transfected with individual HLA alleles from patient 4273 and either WT or mutated TP53 TMG with or without the p53-R248W neoantigen, respectively. The following day, TIL with specificity to p53-R248W from Patient 4273 (fragment culture 15) were co-cultured with transfected Cos7 cells overnight at 37 °C. Secretion of IFN-γ was evaluated by ELISA. The results are shown in Figure 36.

T cells expressing mock (no TCR) or 4273-TCR1a2 were co-cultured with autologous APCs which were pulsed with peptide vehicle (DMSO) or purified (>95% by HPLC) 25 amino acid peptides composed of WT p53-R248 sequence or mutated p53-R248W sequence. Media alone and PMA and Ionomycin were negative and positive controls, respectively. Co-cultures were performed overnight at 37 °C. Secretion of IFN-γ was evaluated by ELISPOT. The results are shown in Figure 37.

The sequence of TCR 4273-TP53-R248W-TCR1a1, which was isolated from Patient 4273, is set forth below. Starting from the amino terminus, the first underlined region is the CDR1alpha (SEQ ID NO: 437), the second underlined region is the CDR2alpha (SEQ ID NO: 438), the third underlined region is the CDR3alpha (SEQ ID NO: 439), the fourth underlined region is the CDR1beta (SEQ ID NO: 440), the fifth underlined region is the CDR2beta (SEQ ID NO: 441), and the sixth underlined region is the CDR3beta (SEQ ID NO: 442). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 443) includes the sequence starting from the amino terminus and ending immediately prior to the start of the alpha chain constant region. The beta chain variable region (SEQ ID NO: 444) includes the sequence starting immediately after the linker and ending immediately prior to the start of the beta chain constant region. The full-length alpha chain (SEQ ID NO: 445) includes the sequence starting from the amino terminus and ending immediately prior to the start of the linker. The full-length beta chain (SEQ ID NO: 446) includes the sequence starting immediately after the linker and ending with the carboxyl terminus.
**TCR name: 4273-TP53-R248W-TCR1a1**
**Recognition of p53 mutation: R248W**
**Method: p53 "hotspot" mutation universal screening**

MASLRVLLVILWLQLSWVWSQQKEVEQNSGPLSVPEGAIASLNCTYSDRGSQSFFW
YRQYSGKSPELIMFIYSNGDKEDGRFTAQLNKASQYVSLLIRDSQPSDSATYLCAVTL
CGGYNKLIFGAGTRLAVHPNIQNPEPAVYQLKDPRSQDSTLCLFTDFDSQINVPKTMES
GTFITDKCVLDMKAMDSKSNGAIAWSNQTSFTCQDIFKETNATYPSSDVPCDATLTEKSFE
TDMNLNFQNLLVIVLRILLLKVAGFNLLMTLRLWSSRAKRSGSGATNFSLLKQAGDVE
ENPGPMHNQVLCCVVLCFLGANTVDGGITQSPKYLFRKEGQNVTLSCEQNLNHDA
MYWYRQDPGQGLRLIYYSQIVNDFQKGDIVEGYSVSREKKESFPLTVTSAQKNPTAF
YLCASSSRDYEQYFGPGTRLTVTEDLRNVTPPKVSLFEPSKAEIANKQKATLVCLARGFF
PDHVELSWWVNGKEVHSGVCTDPQAYKESNYSYCLSSRLRVSATFWHNPRNHFRCQVQF
HGLSEEDKWPEGSPKPVTQNISAEAWGRADCGITSASYQQGVLSATILYEILLGKATLYAVL
VSTLVVMAMVKRKNS (SEQ ID NO: 563)

The sequence of TCR 4273-TP53-R248W-TCR1a2, which was isolated from Patient 4273, is set forth below. Starting from the amino terminus, the first underlined region is the CDR1alpha (SEQ ID NO: 447), the second underlined region is the CDR2alpha (SEQ ID NO: 448), the third underlined region is the CDR3alpha (SEQ ID NO: 449), the fourth underlined region is the CDR1beta (SEQ ID NO: 450), the fifth underlined region is the CDR2beta (SEQ ID NO: 451), and the sixth underlined region is the CDR3beta (SEQ ID NO: 452). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 453) includes the sequence starting from the amino terminus and ending immediately prior to the start of the alpha chain constant region. The beta chain variable region (SEQ ID NO: 454) includes the sequence starting immediately after the linker and ending immediately prior to the start of the beta chain constant region. The full-length alpha chain (SEQ ID NO: 455) includes the sequence starting from the amino terminus and ending immediately prior to the start of the linker. The full-length beta chain (SEQ ID NO: 456) includes the sequence starting immediately after the linker and ending with the carboxyl terminus.
**TCR name: 4273-TP53-R248W-TCR1a2**
**Recognition of p53 mutation: R248W**
**Method: p53 "hotspot" mutation universal screening**

MASLRVLLVILWLQLSWVWSQQKEVEQNSGPLSVPEGAIASLNCTYSDRGSQSFFW
YRQYSGKSPELIMFIYSNGDKEDGRFTAQLNKASQYVSLLIRDSQPSDSATYLCAVTL
SGGYNKLIFGAGTRLAVHPNIQNPEPAVYQLKDPRSQDSTLCLFTDFDSQINVPKTMES
GTFITDKCVLDMKAMDSKSNGAIAWSNQTSFTCQDIFKETNATYPSSDVPCDATLTEKSFE
TDMNLNFQNLLVIVLRILLLKVAGFNLLMTLRLWSSRAKRSGSGATNFSLLKQAGDVE
ENPGPMHNQVLCCVVLCFLGANTVDGGITQSPKYLFRKEGQNVTLSCEQNLNHDA
MYWYRQDPGQGLRLIYYSQIVNDFQKGDIVEGYSVSREKKESFPLTVTSAQKNPTAF
YLCASSSRDYEQYFGPGTRLTVTEDLRNVTPPKVSLFEPSKAEIANKQKATLVCLARGFF
PDHVELSWWVNGKEVHSGVCTDPQAYKESNYSYCLSSRLRVSATFWHNPRNHFRCQVQF
HGLSEEDKWPEGSPKPVTQNISAEAWGRADCGITSASYQQGVLSATILYEILLGKATLYAVL
VSTLVVMAMVKRKNS (SEQ ID NO: 564)

The statistics for the TCRs of Patient 4273 are set forth in Table 8 below. In Table 8, 96 total wells sorted with 41BB+ T cells after co-culture with mutated p53 protein (TMG or peptide). 77 wells had productive pairs (meaning had (1) a sequence and (2) no stop codons in the sequence) for a pairing frequency of 80.2%. 43 of those pairs were the CDR3A/CDR3B combination to make 4273-TP53-R248W-TCR1a1 (55.8% of the productive pairs). 30 of those pairs were the CDR3A/CDR3B combination to make 4273-TP53-R248W-TCR1a2 (39% of the productive pairs). Overall, the CDR3A and CDR3B for 4273-TP53-R248W-TCR1a1 were found 50 and 83 times, respectively, out of 96 wells. Overall, the CDR3A and CDR3B for 4273-TP53-R248W-TCR1a2 were found 33 and 83 times, respectively, out of 96 wells.

**TABLE 8**

| **TCR name** | **4273-TP53-R248W-TCR1a1** | **4273-TP53-R248W-TCR1a2** |
|---|---|---|
| CDR3a | CAVTLCGGYNKLIF (SEQ ID NO: 439) | CAVTLSGGYNKLIF (SEQ ID NO: 449) |
| CDR3b | CASSSRDYEQYF (SEQ ID NO: 442) | CASSSRDYEQYF (SEQ ID NO: 452) |
| total wells | 96 | |
| total CDR3a/CDR3b pairs | 77 | |
| % paired | 80.21% | |
| # times CDR3a | 50 | 33 |
| # times CDR3b | 83 | 83 |
| paired CDR3a/CDR3b | 43 | 30 |
| % of paired CDR3a/CDR3b pairs | 55.84% | 38.96% |

### EXAMPLE 7

This example demonstrates the identification of anti-mutated p53 T cells in Patient 4149. This example also demonstrates the isolation of one anti-mutated p53 TCR from patient 4149.

Experiments were carried out as described for Figures 15-18 for Patient 4149. The TCR was found using the Tran method. The TCR was then used to validate the "p53 hotspot mutation universal screening" method.

A TCR (4149-TCRa2b1 or 4149-TCRa2b2) was transposed into autologous PBL from patient 4149 and co-cultured with autologous APCs which were (1) electroporated with TMG composed of irrelevant, WT p53, or mutated p53 sequence or (2) pulsed with peptide vehicle (DMSO) or purified (>95% by HPLC) 25-amino acid peptides composed of WT p53-Y220 sequence or mutated p53-Y220C sequence. Combination of PMA and ionomycin was positive control. Co-cultures were performed overnight at 37 °C. Secretion of IFN-γ was evaluated using ELISPOT assay. The results are shown in Figure 15. Expression of 4-1BB was evaluated by flow cytometry after gating for lymphocytes → living cells (PI negative) → CD3+ (T cells) → CD4+ mTCR+ (TCR transposed T cells). The results are shown in Figure 16.

The percentage of CD4+4-1BB+ cells by TCRAD deep sequencing and TCRB deep sequencing was also performed. The results are shown in Table 9.

**TABLE 9**

| **TCR name** | **CDR3α (% of CD4+41BB+ by TCRAD deep sequencing)** | **CDR3β (% of CD4+41BB+ by TCRB deep sequencing)** |
|---|---|---|
| **4149-TCR-a2b1** | 20% | 66% |
| **4149-TCR-a2b2** | 20% | 18% |

Mapping of putative p53^{Y220C} minimal epitope recognized by 4149-F11: Autologous DC cells were peptide pulsed (10 µg/mL) and rested overnight in granulocyte-macrophage colony-stimulating factor (GM-CSF) and IL-4. TIL were rested for 2-3 days in 500 CU/mL IL-2. 2×10⁴ TIL and 10⁵ target cells were co-cultured overnight at 37 °C. IFN-γ was measured by ELISPOT. The results are shown in Table 10. 4-1BB expression was measured by FACS with the gate lymphocytes\PI(neg)CD3+\CD3+CD4+. The results are shown in Figure 17.

**TABLE 10**

| **Peptide No.** | **ELISPOT Result Positive (+) or negative (-) for IFN-γ production** | **Peptide** | **SEQ ID NO:** |
|---|---|---|---|
| 0 | - | Vehicle | Not applicable |
| 1 | + | DRNTFRHSVVVP**C**EP | 508 |
| 2 | + | RNTFRHSVVVP**C**EPP | 509 |
| 3 | + | NTFRHSVVVP**C**EPPE | 510 |
| 4 | + | TFRHSVVVP**C**EPPEV | 511 |
| 5 | + (weak) | FRHSVVVP**C**EPPEVG | 512 |
| 6 | - | RHSVVVP**C**EPPEVGS | 513 |
| 7 | - | HSVVVP**C**EPPEVGSD | 514 |
| 8 | - | SVVVP**C**EPPEVGSDC | 515 |
| 9 | - | VVVP**C**EPPEVGSDCT | 516 |
| 10 | - | VVP**C**EPPEVGSDCTT | 517 |
| 11 | - | VP**C**EPPEVGSDCTTI | 518 |

Cos7 cells (2.5 × 10⁴ per well) were plated on wells of flat-bottom 96 well plates. After 20 hours, cells were co-transfected with individual HLA alleles with or without TMGs. After 20 hours, autologous DC cells were transfected with TMG in parallel. All HLA Class-II alleles were co-transfected into one set of wells with or without TMG. Cells not transfected with TMG were pulsed with p53-Y220C 15-mer peptide for 2-3 hours at 37 °C at 10 µg/mL. After washing, 4149-TCRa2b2-transposed T cells (10⁵) at day+14 of second REP were added to wells and co-cultured overnight at 37 °C. IFN-γ secretion was measured by ELISA. The results are shown in Figure 18. Prediction (Table 11) by NetMHCIIpan: cbs.dtu.dk/services/NetMHCIIpan/.

**TABLE 11**

| **HLA** | **Peptide** | **Affinity, nM** | **Rank** |
|---|---|---|---|
| DRB3*02:02 | NTFRHSVVVP**C**EPPE (SEQ ID NO: 510) | 433.8 | 17 |

DRB3*02 expression was detected in 1367 of 3719 (37%) of DRB_ typed patients in the NCI HLA database and in 5 of 9 (56%) endometrial and ovarian cancer patients at NCI-SB. The reported frequency of the DRB3*02 allele is very high.

The sequence of TCR **4149TCRa2b2,** which was isolated from Patient 4149, is set forth below. Starting from the amino terminus, the first underlined region is the CDR1alpha (SEQ ID NO: 57), the second underlined region is the CDR2alpha (SEQ ID NO: 58), the third underlined region is the CDR3alpha (SEQ ID NO: 59), the fourth underlined region is the CDR1beta (SEQ ID NO: 60), the fifth underlined region is the CDR2beta (SEQ ID NO: 61), and the sixth underlined region is the CDR3beta (SEQ ID NO: 62). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 63) includes the sequence starting from the amino terminus and ending immediately prior to the start of the alpha chain constant region. The beta chain variable region (SEQ ID NO: 64) includes the sequence starting immediately after the linker and ending immediately prior to the start of the beta chain constant region. The full-length alpha chain (SEQ ID NO: 65) includes the sequence starting from the amino terminus and ending immediately prior to the start of the linker. The full-length beta chain (SEQ ID NO: 66) includes the sequence starting immediately after the linker and ending with the carboxyl terminus.

Cancer reactive T cells were identified using the screening method set forth below. The p53 reactive cells for this patient were identified by the method described in U.S. Application No. 2017/0224800 ("Tran method").
**TCR name: 4149TCRa2b2**
**Recognition of p53 mutation: Y220C**
**Screening method: Used to validate the p53 "hotspot" mutation universal screening (the latter is given)**
**Co-culture to identify TCR: Co-culture 4149-F11 TIL fragment with p53-Y220C long peptide, sorted CD4+41BB+ T cells**
**Method to identify TCR: Frequency pairing. CD4+41BB+ sorted T cells were expanded in REP and the resulting T cell culture was subjected to TCRAD (alpha) and TCRB (beta) deep sequencing by Adaptive Biotechnologies. I paired the top two TCR alphas with the top two TCR betas in a matrix of 4 total TCRs. The second TCR alpha and second TCR beta was the reactive TCR hence TCRa2b2 nomenclature.**
**Abundance of TCR amongst all TCRs: as below**
**TCR orientation: alpha-beta**
**Expression vector: SB transposon**

MASAPISMLAMLFTLSGLRAQSVAQPEDQVNVAEGNPLTVKCTYSVSGNPYLFWYV
QYPNRGLQFLLKYITGDNLVKGSYGFEAEFNKSQTSFHLKKPSALVSDSALYFCAVR
VWDYKLSFGAGTTVTVRANIQNPEPAVYQLKDPRSQDSTLCLFTDFDSQINVPKTMESG
TFITDKCVLDMKAMDSKSNGAIAWSNQTSFTCQDIFKETNATYPSSDVPCDATLTEKSFET
DMNLNFQNLLVIVLRILLLKVAGFNLLMTLRLWSSRAKRSGSGATNFSLLKQAGDVEE
NPGPMHNQVLCCVVLCFLGANTVDGGITQSPKYLFRKEGQNVTLSCEQNLNHDAM
YWYRQDPGQGLRLIYYSQIVNDFQKGDIAEGYSVSREKKESFPLTVTSAQKNPTAFY
LCASSISAGGDGYTFGSGTRLTVVEDLRNVTPPKVSLFEPSKAEIANKQKATLVCLARGF
FPDHVELSWWVNGKEVHSGVCTDPQAYKESNYSYCLSSRLRVSATFWHNPRNHFRCQVQ
FHGLSEEDKWPEGSPKPVTQNISAEAWGRADCGITSASYQQGVLSATILYEILLGKATLYAV
LVSTLVVMAMVKRKNS (SEQ ID NO: 565)

The statistics for TCR **4149TCRa2b2** of Patient 4149 are set forth in Table 12 below.

**TABLE 12**

| **CDR3** | **Rank of unique, productive CDR3s** | **CDR3 Counts** | **CDR3 Frequency** |
|---|---|---|---|
| Alpha: CAVRVWDYKLSF (SEQ ID NO: 59) | 2 of 4,308 | 648,707 of 3,309,400 | 19.6 % |
| Beta: CASSISAGGDGYTF (SEQ ID NO: 62) | 2 of 3,176 | 104,325 of 578,948 | 18.0 % |

### EXAMPLE 8

This example demonstrates the identification of anti-mutated p53 T cells in Patient 4213 by co-culturing autologous APCs induced to express mutated p53 within autologous T cells ("p53 hotspot mutation universal screening"). This example also demonstrates the isolation of twelve anti-mutated p53 TCRs from patient 4213.

Experiments were carried out as described for Figures 19-20 for Patient 4213.

TIL fragments (F2 and F24) from patient 4213 were co-cultured with autologous APCs pulsed with peptide vehicle (DMSO) or purified (>95% by HPLC) 25-amino acid peptides composed of the mutated p53-R248Q sequence. Co-cultures were performed overnight at 37 °C. Expression of 4-1BB was evaluated by flow cytometry after gating for lymphocytes → living cells (PI negative) → CD3+ (T cells). The results are shown in Figure 19. CD8+4-1BB+ T cells were sorted into wells of 96 wells plates. TCRs were identified using single-cell RT-PCR.

CD4+ T cells came from patient 4213's peripheral blood lymphocytes. The CD4+ T cell culture was co-cultured with autologous APCs pulsed with peptide vehicle (DMSO) or purified (>95% by HPLC) 25-amino acid peptides composed of the mutated p53-R248Q sequence. Co-cultures were performed overnight at 37 °C. Secretion of IFN-γ was evaluated by ELISPOT. Expression of 4-1BB was evaluated by flow cytometry after gating for lymphocytes → living cells (PI negative) → CD3+ (T cells). The results are shown in Figure 20. CD4+41BB+ T cells were sorted into wells of 96 wells plates. TCRs were identified using single-cell RT-PCR.

The sequence of **4213-F2-TCR1,** which was isolated from Patient 4213, is set forth below. Starting from the amino terminus, the first underlined region is the CDR1alpha (SEQ ID NO: 317), the second underlined region is the CDR2alpha (SEQ ID NO: 318), the third underlined region is the CDR3alpha (SEQ ID NO: 319), the fourth underlined region is the CDR1beta (SEQ ID NO: 320), the fifth underlined region is the CDR2beta (SEQ ID NO: 321), and the sixth underlined region is the CDR3beta (SEQ ID NO: 322). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 323) includes the sequence starting from the amino terminus and ending immediately prior to the start of the alpha chain constant region. The beta chain variable region (SEQ ID NO: 324) includes the sequence starting immediately after the linker and ending immediately prior to the start of the beta chain constant region. The full-length alpha chain (SEQ ID NO: 325) includes the sequence starting from the amino terminus and ending immediately prior to the start of the linker. The full-length beta chain (SEQ ID NO: 326) includes the sequence starting immediately after the linker and ending with the carboxyl terminus.

Cancer reactive T cells were identified using the screening method set forth below. The method used to isolate the TCR is set forth below.
**TCR name: 4213-F2-TCR1**
**Recognition of p53 mutation: R248Q**
**Screening method: p53 "hotspot" mutation universal screening**
**Co-culture to identify TCR: Co-culture 4213-F2 with R248Q long peptide, sorted CD8+41BB+ T cells**
**Method to identify TCR: single-cell RT-PCR**
**Abundance of TCR amongst all paired TCRs: 8.3% (observed 2 times of 24 pairs)**
**TCR orientation: alpha-beta**
**Expression vector: SB transposon**

MAMLLGASVLILWLQPDWVNSQQKNDDQQVKQNSPSLSVQEGRISILNCDYTNSMF
DYFLWYKKYPAEGPTFLISISSIKDKNEDGRFTVFLNKSAKHLSLHIVPSQPGDSAVYF
CAANTGNQFYFGTGTSLTVIPNIQNPEPAVYQLKDPRSQDSTLCLFTDFDSQINVPKTME
SGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWSSRAKRSGSGATNFSLLKQAGDV
EENPGPMHTRLLCWAALCLLGAELTEAGVAQSPRYKIIEKRQSVAFWCNPISGHATL
YWYQQILGQGPKLLIQFQNNGVVDDSQLPKDRFSAERLKGVDSTLKIQPAKLEDSAV
YLCASSHLAGEFYNEQFFGPGTRLTVLEDLRNVTPPKVSLFEPSKAEIANKQKATLVCLA
RGFFPDHVELSWWVNGKEVHSGVCTDPQAYKESNYSYCLSSRLRVSATFWHNPRNHFRC
QVQFHGLSEEDKWPEGSPKPVTQNISAEAWGRADCGITSASYQQGVLSATILYEILLGKAT
LYAVLVSTLVVMAMVKRKNS (SEQ ID NO: 566)

The statistics for TCR **4213-F2-TCR1** of Patient 4213 are set forth in Table 13 below.

**TABLE 13**

| **Parameter** | # | **Frequency** |
|---|---|---|
| Total wells | 96 | 100% |
| CDR3alpha | 3 | 3.1% |
| CDR3beta | 8 | 8.3% |
| 4213-F2-TCR1 pairs | 2 | 2.1% |
| Total paired TCRs | 24 | 25.0% |

The sequence of **4213-F2-TCR2,** which was isolated from Patient 4213, is set forth below. Starting from the amino terminus, the first underlined region is the CDR1alpha (SEQ ID NO: 327), the second underlined region is the CDR2alpha (SEQ ID NO: 328), the third underlined region is the CDR3alpha (SEQ ID NO: 329), the fourth underlined region is the CDR1beta (SEQ ID NO: 330), the fifth underlined region is the CDR2beta (SEQ ID NO: 331), and the sixth underlined region is the CDR3beta (SEQ ID NO: 332). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 333) includes the sequence starting from the amino terminus and ending immediately prior to the start of the alpha chain constant region. The beta chain variable region (SEQ ID NO: 334) includes the sequence starting immediately after the linker and ending immediately prior to the start of the beta chain constant region. The full-length alpha chain (SEQ ID NO: 335) includes the sequence starting from the amino terminus and ending immediately prior to the start of the linker. The full-length beta chain (SEQ ID NO: 336) includes the sequence starting immediately after the linker and ending with the carboxyl terminus.

Cancer reactive T cells were identified using the screening method set forth below. The method used to isolate the TCR is set forth below.
**TCR name: 4213-F2-TCR2**
**Recognition of p53 mutation: R248Q**
**Screening method: p53 "hotspot" mutation universal screening**
**Co-culture to identify TCR: Co-culture 4213-F2 with R248Q long peptide, sorted CD8+41BB+ T cells**
**Method to identify TCR: single-cell RT-PCR**
**Abundance of TCR amongst all paired TCRs: 12.5% (observed 3 times of 24 pairs)**
**TCR orientation: alpha-beta**
**Expression vector: SB transposon**

MAGAFLLYVSMKMGGTAGQSLEQPSEVTAVEGAIVQINCTYQTSGFYGLSWYQQH
DGGAPTFLSYNALDGLEETGRFSSFLSRSDSYGYLLLQELQMKDSASYFCAFAYGQN
FVFGPGTRLSVLPNIQNPEPAVYQLKDPRSQDSTLCLFTDFDSQINVPKTMESGTFITDKC
VLDMKAMDSKSNGAIAWSNQTSFTCQDIFKETNATYPSSDVPCDATLTEKSFETDMNLNF
QNLLVIVLRILLLKVAGFNLLMTLRLWSSRAKRSGSGATNFSLLKQAGDVEENPGPMH
FRLLCCVAFCLLGAGPVDSGVTQTPKHLITATGQRVTLRCSPRSGDLSVYWYQQSLD
QGLQFLIQYYNGEERAKGNILERFSAQQFPDLHSELNLSSLELGDSALYFCASSPLGD
SGNTIYFGEGSWLTVVEDLRNVTPPKVSLFEPSKAEIANKQKATLVCLARGFFPDHVELS
WWVNGKEVHSGVCTDPQAYKESNYSYCLSSRLRVSATFWHNPRNHFRCQVQFHGLSEED
KWPEGSPKPVTQNISAEAWGRADCGITSASYQQGVLSATILYEILLGKATLYAVLVSTLVVM
AMVKRKNS (SEQ ID NO: 567)

The statistics for TCR **4213-F2-TCR2** of Patient 4213 are set forth in Table 14 below.

**TABLE 14**

| **Parameter** | # | **Frequency** |
|---|---|---|
| Total wells | 96 | 100% |
| CDR3alpha | 4 | 4.2% |
| CDR3beta | 4 | 4.2% |
| 4213-F2-TCR2 pairs | 3 | 3.1% |
| Total paired TCRs | 24 | 25.0% |

The sequence of **4213-F2-TCR3,** which was isolated from Patient 4213, is set forth below. Starting from the amino terminus, the first underlined region is the CDR1alpha (SEQ ID NO: 337), the second underlined region is the CDR2alpha (SEQ ID NO: 338), the third underlined region is the CDR3alpha (SEQ ID NO: 339), the fourth underlined region is the CDR1beta (SEQ ID NO: 340), the fifth underlined region is the CDR2beta (SEQ ID NO: 341), and the sixth underlined region is the CDR3beta (SEQ ID NO: 342). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 343) includes the sequence starting from the amino terminus and ending immediately prior to the start of the alpha chain constant region. The beta chain variable region (SEQ ID NO: 344) includes the sequence starting immediately after the linker and ending immediately prior to the start of the beta chain constant region. The full-length alpha chain (SEQ ID NO: 345) includes the sequence starting from the amino terminus and ending immediately prior to the start of the linker. The full-length beta chain (SEQ ID NO: 346) includes the sequence starting immediately after the linker and ending with the carboxyl terminus.

Cancer reactive T cells were identified using the screening method set forth below. The method used to isolate the TCR is set forth below.
**TCR name: 4213-F2-TCR3**
**Recognition of p53 mutation: R248Q**
**Screening method: p53 "hotspot" mutation universal screening**
**Co-culture to identify TCR: Co-culture 4213-F2 with R248Q long peptide, sorted CD8+41BB+ T cells**
**Method to identify TCR: single-cell RT-PCR**
**Abundance of TCR amongst all paired TCRs: 70.8% (observed 17 times of 24 pairs)**
**TCR orientation: alpha-beta**
**Expression vector: SB transposon**

MATLLGVSLVILWLQLARVNSQQGEEDPQALSIQEGENATMNCSYKTSINNLQWYR
QNSGRGLVHLILIRSNEREKHSGRLRVTLDTSKKSSSLLITASRAADTASYFCATDAW
NNDMRFGAGTRLTVKPNIQNPEPAVYQLKDPRSQDSTLCLFTDFDSQINVPKTMESGTF
ITDKCVLDMKAMDSKSNGAIAWSNQTSFTCQDIFKETNATYPSSDVPCDATLTEKSFETD
MNLNFQNLLVIVLRILLLKVAGFNLLMTLRLWSSRAKRSGSGATNFSLLKQAGDVEEN
PGPMHIGLLCCVAFSLLWASPVNAGVTQTPKFQVLKTGQSMTLQCAQDMNHNSMY
WYRQDPGMGLRLIYYSASEGTTDKGEVPNGYNVSRLNKREFSLRLESAAPSQTSVYF
CASSESQGNTEAFFGQGTRLTVVEDLRNVTPPKVSLFEPSKAEIANKQKATLVCLARGFF
PDHVELSWWVNGKEVHSGVCTDPQAYKESNYSYCLSSRLRVSATFWHNPRNHFRCQVQF
HGLSEEDKWPEGSPKPVTQNISAEAWGRADCGITSASYQQGVLSATILYEILLGKATLYAVL
VSTLVVMAMVKRKNS (SEQ ID NO: 568)

The statistics for TCR **4213-F2-TCR3** of Patient 4213 are set forth in Table 15 below.

**TABLE 15**

| **Parameter** | # | **Frequency** |
|---|---|---|
| Total wells | 96 | 100% |
| CDR3alpha | 19 | 19.8% |
| CDR3beta | 42 | 43.8% |
| 4213-F2-TCR3 pairs | 17 | 17.7% |
| Total paired TCRs | 24 | 25.0% |

The sequence of **4213-F24-TCRa1**, which was isolated from Patient 4213, is set forth below. Starting from the amino terminus, the first underlined region is the CDR1alpha (SEQ ID NO: 347), the second underlined region is the CDR2alpha (SEQ ID NO: 348), the third underlined region is the CDR3alpha (SEQ ID NO: 349), the fourth underlined region is the CDR1beta (SEQ ID NO: 350), the fifth underlined region is the CDR2beta (SEQ ID NO: 351), and the sixth underlined region is the CDR3beta (SEQ ID NO: 352). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 353) includes the sequence starting from the amino terminus and ending immediately prior to the start of the alpha chain constant region. The beta chain variable region (SEQ ID NO: 354) includes the sequence starting immediately after the linker and ending immediately prior to the start of the beta chain constant region. The full-length alpha chain (SEQ ID NO: 355) includes the sequence starting from the amino terminus and ending immediately prior to the start of the linker. The full-length beta chain (SEQ ID NO: 356) includes the sequence starting immediately after the linker and ending with the carboxyl terminus.

Cancer reactive T cells were identified using the screening method set forth below. The method used to isolate the TCR is set forth below.
**TCR name: 4213-F24-TCRa1**
**Recognition of p53 mutation: R248Q**
**Screening method: p53 "hotspot" mutation universal screening**
**Co-culture to identify TCR: Co-culture 4213-F24 with R248Q long peptide, sorted CD8+41BB+ T cells**
**Method to identify TCR: single-cell RT-PCR**
**Abundance of TCR amongst all paired TCRs: 15.9% (observed 7 times of 44 pairs)**
**TCR orientation: alpha-beta**
**Expression vector: SB transposon**

MAKHLTTFLVILWLYFYRGNGKNQVEQSPQSLIILEGKNCTLQCNYTVSPFSNLRWY
KQDTGRGPVSLTIMTFSENTKSNGRYTATLDADTKQSSLHITASQLSDSASYICVVSS
YKIIFGTGTRLHVFPNIQNPEPAVYQLKDPRSQDSTLCLFTDFDSQINVPKTMESGTFITD
KCVLDMKAMDSKSNGAIAWSNQTSFTCQDIFKETNATYPSSDVPCDATLTEKSFETDMNL
NFQNLLVIVLRILLLKVAGFNLLMTLRLWSSRAKRSGSGATNFSLLKQAGDVEENPGP
MHTRLLFWVAFCLLGAYHTGAGVSQSPSNKVTEKGKDVELRCDPISGHTALYWYR
QRLGQGLEFLIYFQGNSAPDKSGLPSDRFSAERTGESVSTLTIQRTQQEDSAVYLCAS
SPIQGENSPLHFGNGTRLTVTEDLRNVTPPKVSLFEPSKAEIANKQKATLVCLARGFFPD
HVELSWWVNGKEVHSGVCTDPQAYKESNYSYCLSSRLRVSATFWHNPRNHFRCQVQFHG
LSEEDKWPEGSPKPVTQNISAEAWGRADCGITSASYQQGVLSATILYEILLGKATLYAVLVST
LVVMAMVKRKNS (SEQ ID NO: 569)

The statistics for TCR **4213-F24-TCRa1** of Patient 4213 are set forth in Table 16 below.

**TABLE 16**

| **Parameter** | # | **Frequency** |
|---|---|---|
| Total wells | 96 | 100% |
| CDR3alpha | 7 | 7.3% |
| CDR3beta | 80 | 83.3% |
| 4213-F24-TCRa1 pairs | 7 | 7.3% |
| Total paired TCRs | 44 | 45.8% |

The sequence of **4213-F24-TCRa2,** which was isolated from Patient 4213, is set forth below. Starting from the amino terminus, the first underlined region is the CDR1alpha (SEQ ID NO: 357), the second underlined region is the CDR2alpha (SEQ ID NO: 358), the third underlined region is the CDR3alpha (SEQ ID NO: 359), the fourth underlined region is the CDR1beta (SEQ ID NO: 360), the fifth underlined region is the CDR2beta (SEQ ID NO: 361), and the sixth underlined region is the CDR3beta (SEQ ID NO: 362). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 363) includes the sequence starting from the amino terminus and ending immediately prior to the start of the alpha chain constant region. The beta chain variable region (SEQ ID NO: 364) includes the sequence starting immediately after the linker and ending immediately prior to the start of the beta chain constant region. The full-length alpha chain (SEQ ID NO: 365) includes the sequence starting from the amino terminus and ending immediately prior to the start of the linker. The full-length beta chain (SEQ ID NO: 366) includes the sequence starting immediately after the linker and ending with the carboxyl terminus.

Cancer reactive T cells were identified using the screening method set forth below. The method used to isolate the TCR is set forth below.
**TCR name: 4213-F24-TCRa2**
**Recognition of p53 mutation: R248Q**
**Screening method: p53 "hotspot" mutation universal screening**
**Co-culture to identify TCR: Co-culture 4213-F24 with R248Q long peptide, sorted CD8+41BB+ T cells**
**Method to identify TCR: single-cell RT-PCR**
**Abundance of TCR amongst all paired TCRs: 84.1% (observed 37 times of 44 pairs)**
**TCR orientation: alpha-beta**
**Expression vector: SB transposon**

MAKHLTTFLVILWLYFYRGNGKNQVEQSPQSLIILEGKNCTLQCNYTVSPFSNLRWY
KQDTGRGPVSLTIMTFSENTKSNGRYTATLDADTKQSSLHITASQLSDSASYICVVSS
YKLIFGTGTRLQVFPNIQNPEPAVYQLKDPRSQDSTLCLFTDFDSQINVPKTMESGTFITD
KCVLDMKAMDSKSNGAIAWSNQTSFTCQDIFKETNATYPSSDVPCDATLTEKSFETDMNL
NFQNLLVIVLRILLLKVAGFNLLMTLRLWSSRAKRSGSGATNFSLLKQAGDVEENPGP
MHTRLLFWVAFCLLGAYHTGAGVSQSPSNKVTEKGKDVELRCDPISGHTALYWYR
QRLGQGLEFLIYFQGNSAPDKSGLPSDRFSAERTGESVSTLTIQRTQQEDSAVYLCAS
SPIQGENSPLHFGNGTRLTVTEDLRNVTPPKVSLFEPSKAEIANKQKATLVCLARGFFPD
HVELSWWVNGKEVHSGVCTDPQAYKESNYSYCLSSRLRVSATFWHNPRNHFRCQVQFHG
LSEEDKWPEGSPKPVTQNISAEAWGRADCGITSASYQQGVLSATILYEILLGKATLYAVLVST
LVVMAMVKRKNS (SEQ ID NO: 570)

The statistics for TCR **4213-F24-TCRa2** of Patient 4213 are set forth in Table 17 below.

**TABLE 17**

| **Parameter** | # | **Frequency** |
|---|---|---|
| Total wells | 96 | 100% |
| CDR3alpha | 39 | 40.6% |
| CDR3beta | 80 | 83.3% |
| 4213-F24-TCRa2 pairs | 37 | 38.5% |
| Total paired TCRs | 44 | 45.8% |

The sequence of **4213-PBL-TCR1,** which was isolated from Patient 4213, is set forth below. Starting from the amino terminus, the first underlined region is the CDR1alpha (SEQ ID NO: 367), the second underlined region is the CDR2alpha (SEQ ID NO: 368), the third underlined region is the CDR3alpha (SEQ ID NO: 369), the fourth underlined region is the CDR1beta (SEQ ID NO: 370), the fifth underlined region is the CDR2beta (SEQ ID NO: 371), and the sixth underlined region is the CDR3beta (SEQ ID NO: 372). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 373) includes the sequence starting from the amino terminus and ending immediately prior to the start of the alpha chain constant region. The beta chain variable region (SEQ ID NO: 374) includes the sequence starting immediately after the linker and ending immediately prior to the start of the beta chain constant region. The full-length alpha chain (SEQ ID NO: 375) includes the sequence starting from the amino terminus and ending immediately prior to the start of the linker. The full-length beta chain (SEQ ID NO: 376) includes the sequence starting immediately after the linker and ending with the carboxyl terminus.

Cancer reactive T cells were identified using the screening method set forth below. The method used to isolate the TCR is set forth below.
**TCR name: 4213-PBL-TCR1**
**Recognition of p53 mutation: R248Q**
**Screening method: p53 "hotspot" mutation universal screening**
**Co-culture to identify TCR: CD4+ Memory T cells after in vitro sensitization with R248Q long peptide were co-cultured with R248Q long peptide and CD4+41BB+ T cells were sorted**
**Method to identify TCR: single-cell RT-PCR**
**Abundance of TCR amongst all paired TCRs: 9.5% (observed 6 times of 63 pairs)**
**TCR orientation: alpha-beta**
**Expression vector: SB transposon**

MALLLVPAFQVIFTLGGTRAQSVTQLDSQVPVFEEAPVELRCNYSSSVSVYLFWYVQ
YPNQGLQLLLKYLSGSTLVESINGFEAEFNKSQTSFHLRKPSVHISDTAEYFCAVSKG
TGAQKLVFGQGTRLTINPNIQNPEPAVYQLKDPRSQDSTLCLFTDFDSQINVPKTMESGT
FITDKCVLDMKAMDSKSNGAIAWSNQTSFTCQDIFKETNATYPSSDVPCDATLTEKSFETD
MNLNFQNLLVIVLRILLLKVAGFNLLMTLRLWSSRAKRSGSGATNFSLLKQAGDVEEN
PGPMHNQVLCCVVLCFLGANTVDGGITQSPKYLFRKEGQNVTLSCEQNLNHDAMY
WYRQDPGQGLRLIYYSQIVNDFQKGDIAEGYSVSREKKESFPLIVTSAQKNPTASYLC
ASEAFFGQGTRLTVVEDLRNVTPPKVSLFEPSKAEIANKQKATLVCLARGFFPDHVELSW
WVNGKEVHSGVCTDPQAYKESNYSYCLSSRLRVSATFWHNPRNHFRCQVQFHGLSEEDK
WPEGSPKPVTQNISAEAWGRADCGITSASYQQGVLSATILYEILLGKATLYAVLVSTLVVMA
MVKRKNS (SEQ ID NO: 571)

The statistics for TCR **4213-PBL-TCR1** of Patient 4213 are set forth in Table 18 below.

**TABLE 18**

| **Parameter** | # | **Frequency** |
|---|---|---|
| Total wells | 192 | 100% |
| CDR3alpha | 9 | 4.7% |
| CDR3beta | 6 | 3.1% |
| 4213-PBL-TCR1 pairs | 6 | 3.1% |
| Total paired TCRs | 63 | 32.8% |

The sequence of **4213-PBL-TCR2,** which was isolated from Patient 4213, is set forth below. Starting from the amino terminus, the first underlined region is the CDR1alpha (SEQ ID NO: 377), the second underlined region is the CDR2alpha (SEQ ID NO: 378), the third underlined region is the CDR3alpha (SEQ ID NO: 379), the fourth underlined region is the CDR1beta (SEQ ID NO: 380), the fifth underlined region is the CDR2beta (SEQ ID NO: 381), and the sixth underlined region is the CDR3beta (SEQ ID NO: 382). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 383) includes the sequence starting from the amino terminus and ending immediately prior to the start of the alpha chain constant region. The beta chain variable region (SEQ ID NO: 384) includes the sequence starting immediately after the linker and ending immediately prior to the start of the beta chain constant region. The full-length alpha chain (SEQ ID NO: 385) includes the sequence starting from the amino terminus and ending immediately prior to the start of the linker. The full-length beta chain (SEQ ID NO: 386) includes the sequence starting immediately after the linker and ending with the carboxyl terminus.

Cancer reactive T cells were identified using the screening method set forth below. The method used to isolate the TCR is set forth below.
**TCR name: 4213-PBL-TCR2**
**Recognition of p53 mutation: R248Q**
**Screening method: p53 "hotspot" mutation universal screening**
**Co-culture to identify TCR: CD4+ Memory T cells after in vitro sensitization with R248Q long peptide were co-cultured with R248Q long peptide and CD4+41BB+ T cells were sorted**
**Method to identify TCR: single-cell RT-PCR**
**Abundance of TCR amongst all paired TCRs: 7.9% (observed 5 times of 63 pairs)**
**TCR orientation: alpha-beta**
**Expression vector: SB transposon**

MALVTSITVLLSLGIMGDAKTTQPNSMESNEEEPVHLPCNHSTISGTDYIHWYRQLPS
QGPEYVIHGLTSNVNNRMASLAIAEDRKSSTLILHRATLRDAAVYYCILASGAGSYQ
LTFGKGTKLSVIPNIQNPEPAVYQLKDPRSQDSTLCLFTDFDSQINVPKTMESGTFITDKC
VLDMKAMDSKSNGAIAWSNQTSFTCQDIFKETNATYPSSDVPCDATLTEKSFETDMNLNF
QNLLVIVLRILLLKVAGFNLLMTLRLWSSRAKRSGSGATNFSLLKQAGDVEENPGPMH
CRLLCCVVFCLLQAGPLDTAVSQTPKYLVTQMGNDKSIKCEQNLGHDTMYWYKQD
SKKFLKIMFSYNNKELIINETVPNRFSPKSPDKAHLNLHINSLELGDSAVYFCASRTIG
YNTEAFFGQGTRLTVVEDLRNVTPPKVSLFEPSKAEIANKQKATLVCLARGFFPDHVELS
WWVNGKEVHSGVCTDPQAYKESNYSYCLSSRLRVSATFWHNPRNHFRCQVQFHGLSEED
KWPEGSPKPVTQNISAEAWGRADCGITSASYQQGVLSATILYEILLGKATLYAVLVSTLVVM
AMVKRKNS (SEQ ID NO: 572)

The statistics for TCR **4213-PBL-TCR2** of Patient 4213 are set forth in Table 19 below.

**TABLE 19**

| **Parameter** | # | **Frequency** |
|---|---|---|
| Total wells | 192 | 100% |
| CDR3alpha | 8 | 4.2% |
| CDR3beta | 8 | 4.2% |
| 4213-PBL-TCR2 pairs | 5 | 2.6% |
| Total paired TCRs | 63 | 32.8% |

The sequence of **4213-PBL-TCR3,** which was isolated from Patient 4213, is set forth below. Starting from the amino terminus, the first underlined region is the CDR1alpha (SEQ ID NO: 387), the second underlined region is the CDR2alpha (SEQ ID NO: 388), the third underlined region is the CDR3alpha (SEQ ID NO: 389), the fourth underlined region is the CDR1beta (SEQ ID NO: 390), the fifth underlined region is the CDR2beta (SEQ ID NO: 391), and the sixth underlined region is the CDR3beta (SEQ ID NO: 392). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 393) includes the sequence starting from the amino terminus and ending immediately prior to the start of the alpha chain constant region. The beta chain variable region (SEQ ID NO: 394) includes the sequence starting immediately after the linker and ending immediately prior to the start of the beta chain constant region. The full-length alpha chain (SEQ ID NO: 395) includes the sequence starting from the amino terminus and ending immediately prior to the start of the linker. The full-length beta chain (SEQ ID NO: 396) includes the sequence starting immediately after the linker and ending with the carboxyl terminus.

Cancer reactive T cells were identified using the screening method set forth below. The method used to isolate the TCR is set forth below.
**TCR name: 4213-PBL-TCR3**
**Recognition of p53 mutation: R248Q**
**Screening method: p53 "hotspot" mutation universal screening**
**Co-culture to identify TCR: CD4+ Memory T cells after in vitro sensitization with R248Q long peptide were co-cultured with R248Q long peptide and CD4+41BB+ T cells were sorted**
**Method to identify TCR: single-cell RT-PCR**
**Abundance of TCR amongst all paired TCRs: 6.3% (observed 4 times of 63 pairs)**
**TCR orientation: alpha-beta**
**Expression vector: SB transposon**

MAKIRQFLLAILWLQLSCVSAAKNEVEQSPQNLTAQEGEFITINCSYSVGISALHWLQ
QHPGGGIVSLFMLSSGKKKHGRLIATINIQEKHSSLHITASHPRDSAVYICAALSYNTD
KLIFGTGTRLQVFPNIQNPEPAVYQLKDPRSQDSTLCLFTDFDSQINVPKTMESGTFITDK
CVLDMKAMDSKSNGAIAWSNQTSFTCQDIFKETNATYPSSDVPCDATLTEKSFETDMNLN
FQNLLVIVLRILLLKVAGFNLLMTLRLWSSRAKRSGSGATNFSLLKQAGDVEENPGPM
HTRLLCWAALCLLGADHTGAGVSQTPSNKVTEKGKYVELRCDPISGHTALYWYRQS
LGQGPEFLIYFQGTGAADDSGLPNDRFFAVRPEGSVSTLKIQRTERGDSAVYLCASSL
SGLLQETQYFGPGTRLLVLEDLRNVTPPKVSLFEPSKAEIANKQKATLVCLARGFFPDHV
ELSWWVNGKEVHSGVCTDPQAYKESNYSYCLSSRLRVSATFWHNPRNHFRCQVQFHGLS
EEDKWPEGSPKPVTQNISAEAWGRADCGITSASYQQGVLSATILYEILLGKATLYAVLVSTL
VVMAMVKRKNS (SEQ ID NO: 573)

The statistics for TCR **4213-PBL-TCR3** of Patient 4213 are set forth in Table 20 below.

**TABLE 20**

| **Parameter** | # | **Frequency** |
|---|---|---|
| Total wells | 192 | 100% |
| CDR3alpha | 4 | 2.1% |
| CDR3beta | 7 | 3.6% |
| 4213-PBL-TCR3 pairs | 4 | 2.1% |
| Total paired TCRs | 63 | 32.8% |

The sequence of **4213-PBL-TCR4a1**, which was isolated from Patient 4213, is set forth below. Starting from the amino terminus, the first underlined region is the CDR1alpha (SEQ ID NO: 397), the second underlined region is the CDR2alpha (SEQ ID NO: 398), the third underlined region is the CDR3alpha (SEQ ID NO: 399), the fourth underlined region is the CDR1beta (SEQ ID NO: 400), the fifth underlined region is the CDR2beta (SEQ ID NO: 401), and the sixth underlined region is the CDR3beta (SEQ ID NO: 402). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 403) includes the sequence starting from the amino terminus and ending immediately prior to the start of the alpha chain constant region. The beta chain variable region (SEQ ID NO: 404) includes the sequence starting immediately after the linker and ending immediately prior to the start of the beta chain constant region. The full-length alpha chain (SEQ ID NO: 405) includes the sequence starting from the amino terminus and ending immediately prior to the start of the linker. The full-length beta chain (SEQ ID NO: 406) includes the sequence starting immediately after the linker and ending with the carboxyl terminus.

Cancer reactive T cells were identified using the screening method set forth below. The method used to isolate the TCR is set forth below.
**TCR name: 4213-PBL-TCR4a1**
**Recognition of p53 mutation: R248Q**
**Screening method: p53 "hotspot" mutation universal screening**
**Co-culture to identify TCR: CD4+ Memory T cells after in vitro sensitization with R248Q long peptide were co-cultured with R248Q long peptide and CD4+41BB+ T cells were sorted**
**Method to identify TCR: single-cell RT-PCR**
**Abundance of TCR amongst all paired TCRs: 3.2% (observed 2 times of 63 pairs)**
**TCR orientation: alpha-beta**
**Expression vector: SB transposon**

MAYSPGLVSLILLLLGRTRGNSVTQMEGPVTLSEEAFLTINCTYTATGYPSLFWYVQ
YPGEGLQLLLKATKADDKGSNKGFEATYRKETTSFHLEKGSVQVSDSAVYFCALSH
TGSSNTGKLIFGQGTRLQVKPNIQNPEPAVYQLKDPRSQDSTLCLFTDFDSQINVPKTME
SGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWSSRAKRSGSGATNFSLLKQAGDV
EENPGPMHTRLLCWAALCLLGAELTEAGVAQSPRYKIIEKRQSVAFWCNPISGHATL
YWYQQILGQGPKLLIQFQNNGVVDDSQLPKDRFSAERLKGVDSTLKIQPAKLEDSAV
YLCASSTGGGRHQPQHFGDGTRLSILEDLRNVTPPKVSLFEPSKAEIANKQKATLVCLAR
GFFPDHVELSWWVNGKEVHSGVCTDPQAYKESNYSYCLSSRLRVSATFWHNPRNHFRCQ
VQFHGLSEEDKWPEGSPKPVTQNISAEAWGRADCGITSASYQQGVLSATILYEILLGKATLY
AVLVSTLVVMAMVKRKNS (SEQ ID NO: 574)

The statistics for TCR **4213-PBL-TCR4a1** of Patient 4213 are set forth in Table 21 below.

**TABLE 21**

| **Parameter** | # | **Frequency** |
|---|---|---|
| Total wells | 192 | 100% |
| CDR3alpha | 2 | 1.0% |
| CDR3beta | 36 | 18.8% |
| 4213-PBL-TCR4a1 pairs | 2 | 1.0% |
| Total paired TCRs | 63 | 32.8% |

The sequence of **4213-PBL-TCR4a2,** which was isolated from Patient 4213, is set forth below. Starting from the amino terminus, the first underlined region is the CDR1alpha (SEQ ID NO: 407), the second underlined region is the CDR2alpha (SEQ ID NO: 408), the third underlined region is the CDR3alpha (SEQ ID NO: 409), the fourth underlined region is the CDR1beta (SEQ ID NO: 410), the fifth underlined region is the CDR2beta (SEQ ID NO: 411), and the sixth underlined region is the CDR3beta (SEQ ID NO: 412). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 413) includes the sequence starting from the amino terminus and ending immediately prior to the start of the alpha chain constant region. The beta chain variable region (SEQ ID NO: 414) includes the sequence starting immediately after the linker and ending immediately prior to the start of the beta chain constant region. The full-length alpha chain (SEQ ID NO: 415) includes the sequence starting from the amino terminus and ending immediately prior to the start of the linker. The full-length beta chain (SEQ ID NO: 416) includes the sequence starting immediately after the linker and ending with the carboxyl terminus.

Cancer reactive T cells were identified using the screening method set forth below. The method used to isolate the TCR is set forth below.
**TCR name: 4213-PBL-TCR4a2**
**Recognition of p53 mutation: R248Q**
**Screening method: p53 "hotspot" mutation universal screening**
**Co-culture to identify TCR: CD4+ Memory T cells after in vitro sensitization with R248Q long peptide were co-cultured with R248Q long peptide and CD4+41BB+ T cells were sorted**
**Method to identify TCR: single-cell RT-PCR**
**Abundance of TCR amongst all paired TCRs: 3.2% (observed 2 times of 63 pairs)**
**TCR orientation: alpha-beta**
**Expression vector: SB transposon**

MAYSPGLVSLILLLLGRTRGNSVTQMEGPVTLSEEAFLTINCTYTATGYPSLFWYVQ
YPGEGLQLLLKATKADDKGSNKGFEATYRKETTSFHLEKGSVQVSDSAVYFCALSQ
TGSSKTGKLIFGQGTRLQVKPNIQNPEPAVYQLKDPRSQDSTLCLFTDFDSQINVPKTME
SGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWSSRAKRSGSGATNFSLLKQAGDV
EENPGPMHTRLLCWAALCLLGAELTEAGVAQSPRYKIIEKRQSVAFWCNPISGHATL
YWYQQILGQGPKLLIQFQNNGVVDDSQLPKDRFSAERLKGVDSTLKIQPAKLEDSAV
YLCASSTGGGRHQPQHFGDGTRLSILEDLRNVTPPKVSLFEPSKAEIANKQKATLVCLAR
GFFPDHVELSWWVNGKEVHSGVCTDPQAYKESNYSYCLSSRLRVSATFWHNPRNHFRCQ

VQFHGLSEEDKWPEGSPKPVTQNISAEAWGRADCGITSASYQQGVLSATILYEILLGKATLY
AVLVSTLVVMAMVKRKNS (SEQ ID NO: 575)

The statistics for TCR **4213-PBL-TCR4a2** of Patient 4213 are set forth in Table 22 below.

**TABLE 22**

| **Parameter** | # | **Frequency** |
|---|---|---|
| Total wells | 192 | 100% |
| CDR3alpha | 2 | 1.0% |
| CDR3beta | 36 | 18.8% |
| 4213-PBL-TCR4a2 pairs | 2 | 1.0% |
| Total paired TCRs | 63 | 32.8% |

The sequence of **4213-PBL-TCR4a3,** which was isolated from Patient 4213, is set forth below. Starting from the amino terminus, the first underlined region is the CDR1alpha (SEQ ID NO: 417), the second underlined region is the CDR2alpha (SEQ ID NO: 418), the third underlined region is the CDR3alpha (SEQ ID NO: 419), the fourth underlined region is the CDR1beta (SEQ ID NO: 420), the fifth underlined region is the CDR2beta (SEQ ID NO: 421), and the sixth underlined region is the CDR3beta (SEQ ID NO: 422). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 423) includes the sequence starting from the amino terminus and ending immediately prior to the start of the alpha chain constant region. The beta chain variable region (SEQ ID NO: 424) includes the sequence starting immediately after the linker and ending immediately prior to the start of the beta chain constant region. The full-length alpha chain (SEQ ID NO: 425) includes the sequence starting from the amino terminus and ending immediately prior to the start of the linker. The full-length beta chain (SEQ ID NO: 426) includes the sequence starting immediately after the linker and ending with the carboxyl terminus.

Cancer reactive T cells were identified using the screening method set forth below. The method used to isolate the TCR is set forth below.
**TCR name: 4213-PBL-TCR4a3**
**Recognition of p53 mutation: R248Q**
**Screening method: p53 "hotspot" mutation universal screening**
**Co-culture to identify TCR: CD4+ Memory T cells after in vitro sensitization with R248Q long peptide were co-cultured with R248Q long peptide and CD4+41BB+ T cells were sorted**
**Method to identify TCR: single-cell RT-PCR**
**Abundance of TCR amongst all paired TCRs: 4.8% (observed 3 times of 63 pairs)**
**TCR orientation: alpha-beta**
**Expression vector: SB transposon**

MAYSPGLVSLILLLLGRTRGNSVTQMEGPVTLSEEAFLTINCTYTATGYPSLFWYVQ
YPGEGLQLLLKATKADDKGSNKGFEATYRKETTSFHLEKGSVQVSDSAVYFCALSQ
TGSSNTGKLIFGQGTRLQVKPNIQNPEPAVYQLKDPRSQDSTLCLFTDFDSQINVPKTME
SGTFITDKCVLDMKAMDSKSNGAIAWSNQTSFTCQDIFKETNATYPSSDVPCDATLTEKSF
ETDMNLNFQNLLVIVLRILLLKVAGFNLLMTLRLWSSRAKRSGSGATNFSLLKQAGDV
EENPGPMHTRLLCWAALCLLGAELTEAGVAQSPRYKIIEKRQSVAFWCNPISGHATL
YWYQQILGQGPKLLIQFQNNGVVDDSQLPKDRFSAERLKGVDSTLKIQPAKLEDSAV
YLCASSTGGGRHQPQHFGDGTRLSILEDLRNVTPPKVSLFEPSKAEIANKQKATLVCLAR
GFFPDHVELSWWVNGKEVHSGVCTDPQAYKESNYSYCLSSRLRVSATFWHNPRNHFRCQ
VQFHGLSEEDKWPEGSPKPVTQNISAEAWGRADCGITSASYQQGVLSATILYEILLGKATLY
AVLVSTLVVMAMVKRKNS (SEQ ID NO: 576)

The statistics for TCR **4213-PBL-TCR4a3** of Patient 4213 are set forth in Table 23 below.

**TABLE 23**

| **Parameter** | # | **Frequency** |
|---|---|---|
| Total wells | 192 | 100% |
| CDR3alpha | 4 | 2.1% |
| CDR3beta | 36 | 18.8% |
| 4213-PBL-TCR4a3 pairs | 3 | 1.6% |
| Total paired TCRs | 63 | 32.8% |

The sequence of **4213-PBL-TCR4a4,** which was isolated from Patient 4213, is set forth below. Starting from the amino terminus, the first underlined region is the CDR1alpha (SEQ ID NO: 427), the second underlined region is the CDR2alpha (SEQ ID NO: 428), the third underlined region is the CDR3alpha (SEQ ID NO: 429), the fourth underlined region is the CDR1beta (SEQ ID NO: 430), the fifth underlined region is the CDR2beta (SEQ ID NO: 431), and the sixth underlined region is the CDR3beta (SEQ ID NO: 432). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 433) includes the sequence starting from the amino terminus and ending immediately prior to the start of the alpha chain constant region. The beta chain variable region (SEQ ID NO: 434) includes the sequence starting immediately after the linker and ending immediately prior to the start of the beta chain constant region. The full-length alpha chain (SEQ ID NO: 435) includes the sequence starting from the amino terminus and ending immediately prior to the start of the linker. The full-length beta chain (SEQ ID NO: 436) includes the sequence starting immediately after the linker and ending with the carboxyl terminus.

Cancer reactive T cells were identified using the screening method set forth below. The method used to isolate the TCR is set forth below.
**TCR name: 4213-PBL-TCR4a4**
**Recognition of p53 mutation: R248Q**
**Screening method: p53 "hotspot" mutation universal screening**
**Co-culture to identify TCR: CD4+ Memory T cells after in vitro sensitization with R248Q long peptide were co-cultured with R248Q long peptide and CD4+41BB+ T cells were sorted**
**Method to identify TCR: single-cell RT-PCR**
**Abundance of TCR amongst all paired TCRs: 3.2% (observed 2 times of 63 pairs)**
**TCR orientation: alpha-beta**
**Expression vector: SB transposon**

MAYSPGLVSLILLLLGRTRGNSVTQMEGPVTLSEEAFLTINCTYTATGYPSLFWYVQ
YPGEGLQLLLKATKADDKGSNKGFEATYRKETTSFHLEKGSVQVSDSAVYFCALSTT
GSSNTGKLIFGQGTTLQVKPNIQNPEPAVYQLKDPRSQDSTLCLFTDFDSQINVPKTMES
GTFITDKCVLDMKAMDSKSNGAIAWSNQTSFTCQDIFKETNATYPSSDVPCDATLTEKSFE

TDMNLNFQNLLVIVLRILLLKVAGFNLLMTLRLWSSRAKRSGSGATNFSLLKQAGDVE
ENPGPMHTRLLCWAALCLLGAELTEAGVAQSPRYKIIEKRQSVAFWCNPISGHATLY
WYQQILGQGPKLLIQFQNNGVVDDSQLPKDRFSAERLKGVDSTLKIQPAKLEDSAVY
LCASSTGGGRHQPQHFGDGTRLSILEDLRNVTPPKVSLFEPSKAEIANKQKATLVCLARG
FFPDHVELSWWVNGKEVHSGVCTDPQAYKESNYSYCLSSRLRVSATFWHNPRNHFRCQV
QFHGLSEEDKWPEGSPKPVTQNISAEAWGRADCGITSASYQQGVLSATILYEILLGKATLYA
VLVSTLVVMAMVKRKNS (SEQ ID NO: 577)

The statistics for TCR **4213-PBL-TCR4a4** of Patient 4213 are set forth in Table 24 below.

**TABLE 24**

| **Parameter** | # | **Frequency** |
|---|---|---|
| Total wells | 192 | 100% |
| CDR3alpha | 2 | 1.0% |
| CDR3beta | 36 | 18.8% |
| 4213-PBL-TCR4a3 pairs | 2 | 1.0% |
| Total paired TCRs | 63 | 32.8% |

### EXAMPLE 9

This example demonstrates the identification of anti-mutated p53 T cells in Patient 4268 by co-culturing autologous APCs induced to express mutated p53 within autologous T cells ("p53 hotspot mutation universal screening"). This example also demonstrates the isolation of five anti-mutated p53 TCRs from patient 4268.

Experiments were carried out as described for Figures 21-24 for Patient 4268.

TIL fragments (F1-F24, n=24) from patient 4268 were co-cultured with autologous APCs electroporated with TMG composed of irrelevant, WT p53, or mutated p53 sequence. Co-cultures were performed overnight at 37 °C. Secretion of IFN-γ was evaluated using ELISPOT assay. The results are shown in Figure 21.

TIL fragments (F1-F24, n=24) from patient 4268 were co-cultured with autologous APCs pulsed with peptide vehicle (DMSO) or purified (>95% by HPLC) 25-amino acid peptides composed of WT p53-R248 sequence or mutated p53-R248Q sequence. Co-cultures were performed overnight at 37 °C. Secretion of IFN-γ was evaluated using ELISPOT assay. The results are shown in Figure 22.

TIL fragments (F1-F24, n=24) from patient 4268 were co-cultured with autologous APCs pulsed with peptide vehicle (DMSO) or purified (>95% by HPLC) 25-amino acid peptides composed of wt p53-R248 sequence or mutated p53-R248Q sequence. Co-cultures were performed overnight at 37 °C. Expression of 4-1BB was evaluated by flow cytometry after gating for lymphocytes → living cells (PI negative) → CD3+ (T cells). The results are shown in Figures 23-24. TIL fragments F18 and F19 were sources of TCRs after sorting CD4+41BB+ T cells. TIL fragments F7, F8, and F15 were sources of TCRs after sorting CD8+4-1BB+ T cells.

The sequence of **4268-TCR1,** which was isolated from Patient 4268, is set forth below. Starting from the amino terminus, the first underlined region is the CDR1alpha (SEQ ID NO: 137), the second underlined region is the CDR2alpha (SEQ ID NO: 138), the third underlined region is the CDR3alpha (SEQ ID NO: 139), the fourth underlined region is the CDR1beta (SEQ ID NO: 140), the fifth underlined region is the CDR2beta (SEQ ID NO: 141), and the sixth underlined region is the CDR3beta (SEQ ID NO: 142). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 143) includes the sequence starting from the amino terminus and ending immediately prior to the start of the alpha chain constant region. The beta chain variable region (SEQ ID NO: 144) includes the sequence starting immediately after the linker and ending immediately prior to the start of the beta chain constant region. The full-length alpha chain (SEQ ID NO: 145) includes the sequence starting from the amino terminus and ending immediately prior to the start of the linker. The full-length beta chain (SEQ ID NO: 146) includes the sequence starting immediately after the linker and ending with the carboxyl terminus.

Cancer reactive T cells were identified using the screening method set forth below. The method used to isolate the TCR is set forth below.
**TCR name: 4268-TCR1**
**Recognition of p53 mutation: R248Q**
**Method: p53 "hotspot" mutation universal screening**
**Co-culture to identify TCR: Co-culture 4268-F7 and 4268-F8 with p53-R248Q long peptide, sorted CD8+41BB+ T cells**
**Method to identify TCR: single-cell RT-PCR**
**Abundance of TCR amongst all paired TCRs: 78.7% (observed 48 times of 61 pairs)**
**TCR orientation: alpha-beta**
**Expression vector: SB transposon**

MASLRVLLVILWLQLSWVWSQQKEVEQNSGPLSVPEGAIASLNCTYSDRGSQSFFW
YRQYSGKSPELIMFIYSNGDKEDGRFTAQLNKASQYVSLLIRDSQPSDSATYLCAVS
WYSTLTFGKGTMLLVSPNIQNPEPAVYQLKDPRSQDSTLCLFTDFDSQINVPKTMESGT
FITDKCVLDMKAMDSKSNGAIAWSNQTSFTCQDIFKETNATYPSSDVPCDATLTEKSFETD
MNLNFQNLLVIVLRILLLKVAGFNLLMTLRLWSSRAKRSGSGATNFSLLKQAGDVEEN
PGPMHTRLFFYVALCLLWAGHRDAGITQSPRYKITETGRQVTLMCHQTWSHSYMF
WYRQDLGHGLRLIYYSAAADITDKGEVPDGYVVSRSKTENFPLTLESATRSQTSVYF
CASSGSRTDTQYFGPGTRLTVLEDLRNVTPPKVSLFEPSKAEIANKQKATLVCLARGFFP
DHVELSWWVNGKEVHSGVCTDPQAYKESNYSYCLSSRLRVSATFWHNPRNHFRCQVQFH
GLSEEDKWPEGSPKPVTQNISAEAWGRADCGITSASYQQGVLSATILYEILLGKATLYAVLV
STLVVMAMVKRKNS (SEQ ID NO: 578)

The statistics for TCR **4268-TCR1** of Patient 4268 are set forth in Table 25 below.

**TABLE 25**

| **Parameter** | # | **Frequency** |
|---|---|---|
| Total wells | 96 | 100% |
| CDR3alpha | 54 | 56.3% |
| CDR3beta | 71 | 74.0% |
| 4268-TCR1 pairs | 48 | 50.0% |
| Total paired TCRs | 61 | 63.5% |

The sequence of **4268-TCR2,** which was isolated from Patient 4268, is set forth below. Starting from the amino terminus, the first underlined region is the CDR1alpha (SEQ ID NO: 147), the second underlined region is the CDR2alpha (SEQ ID NO: 148), the third underlined region is the CDR3alpha (SEQ ID NO: 149), the fourth underlined region is the CDR1beta (SEQ ID NO: 150), the fifth underlined region is the CDR2beta (SEQ ID NO: 151), and the sixth underlined region is the CDR3beta (SEQ ID NO: 152). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 153) includes the sequence starting from the amino terminus and ending immediately prior to the start of the alpha chain constant region. The beta chain variable region (SEQ ID NO: 154) includes the sequence starting immediately after the linker and ending immediately prior to the start of the beta chain constant region. The full-length alpha chain (SEQ ID NO: 155) includes the sequence starting from the amino terminus and ending immediately prior to the start of the linker. The full-length beta chain (SEQ ID NO: 156) includes the sequence starting immediately after the linker and ending with the carboxyl terminus.

Cancer reactive T cells were identified using the screening method set forth below. The method used to isolate the TCR is set forth below.
**TCR name: 4268-TCR2**
**Recognition of p53 mutation: R248Q**
**Method: p53 "hotspot" mutation universal screening**
**Co-culture to identify TCR: Co-culture 4268-F7 and 4268-F8 with p53-R248Q long peptide, sorted CD8+41BB+ T cells**
**Method to identify TCR: single-cell RT-PCR**
**Abundance of TCR amongst all paired TCRs: 6.6% (observed 4 times of 61 pairs)**
**TCR orientation: alpha-beta**
**Expression vector: SB transposon**

MALKFSVSILWIQLAWVSTQLLEQSPQFLSIQEGENLTVYCNSSSVFSSLQWYRQEPG
EGPVLLVTVVTGGEVKKLKRLTFQFGDARKDSSLHITAAQPGDTGLYLCAGEFAGN
QFYFGTGTSLTVIPNIQNPEPAVYQLKDPRSQDSTLCLFTDFDSQINVPKTMESGTFITDK
CVLDMKAMDSKSNGAIAWSNQTSFTCQDIFKETNATYPSSDVPCDATLTEKSFETDMNLN
FQNLLVIVLRILLLKVAGFNLLMTLRLWSSRAKRSGSGATNFSLLKQAGDVEENPGPM
HCRLLCCAVLCLLGAVPIDTEVTQTPKHLVMGMTNKKSLKCEQHMGHRAMYWYK
QKAKKPPELMFVYSYEKLSINESVPSRFSPECPNSSLLNLHLHALQPEDSALYLCASS
QVGLTYEQYFGPGTRLTVTEDLRNVTPPKVSLFEPSKAEIANKQKATLVCLARGFFPDH

VELSWWVNGKEVHSGVCTDPQAYKESNYSYCLSSRLRVSATFWHNPRNHFRCQVQFHGL
SEEDKWPEGSPKPVTQNISAEAWGRADCGITSASYQQGVLSATILYEILLGKATLYAVLVSTL
VVMAMVKRKNS (SEQ ID NO: 579)

The statistics for TCR **4268-TCR2** of Patient 4268 are set forth in Table 26 below.

**TABLE 26**

| **Parameter** | # | **Frequency** |
|---|---|---|
| Total wells | 96 | 100% |
| CDR3alpha | 6 | 6.3% |
| CDR3beta | 4 | 4.2% |
| 4268-TCR2 pairs | 4 | 4.2% |
| Total paired TCRs | 61 | 63.5% |

The sequence of **4268-TCR3,** which was isolated from Patient 4268, is set forth below. Starting from the amino terminus, the first underlined region is the CDR1alpha (SEQ ID NO: 157), the second underlined region is the CDR2alpha (SEQ ID NO: 158), the third underlined region is the CDR3alpha (SEQ ID NO: 159), the fourth underlined region is the CDR1beta (SEQ ID NO: 160), the fifth underlined region is the CDR2beta (SEQ ID NO: 161), and the sixth underlined region is the CDR3beta (SEQ ID NO: 162). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 163) includes the sequence starting from the amino terminus and ending immediately prior to the start of the alpha chain constant region. The beta chain variable region (SEQ ID NO: 164) includes the sequence starting immediately after the linker and ending immediately prior to the start of the beta chain constant region. The full-length alpha chain (SEQ ID NO: 165) includes the sequence starting from the amino terminus and ending immediately prior to the start of the linker. The full-length beta chain (SEQ ID NO: 166) includes the sequence starting immediately after the linker and ending with the carboxyl terminus.

Cancer reactive T cells were identified using the screening method set forth below. The method used to isolate the TCR is set forth below.
**TCR name: 4268-TCR3**
**Recognition of p53 mutation: R248Q**
**Method: p53 "hotspot" mutation universal screening**
**Co-culture to identify TCR: Co-culture 4268-F15 with p53-R248Q long peptide, sorted CD8+41BB+ T cells**
**Method to identify TCR: single-cell RT-PCR**
**Abundance of TCR amongst all paired TCRs: 88.6% (observed 31 times of 35 pairs)**
**TCR orientation: alpha-beta**
**Expression vector: SB transposon**

MASAPISMLAMLFTLSGLRAQSVAQPEDQVNVAEGNPLTVKCTYSVSGNPYLFWYV
QYPNRGLQFLLKYITGDNLVKGSYGFEAEFNKSQTSFHLKKPSALVSDSALYFCAVR
DNSGGSNYKLTFGKGTLLTVNPNIQNPEPAVYQLKDPRSQDSTLCLFTDFDSQINVPKT
MESGTFITDKCVLDMKAMDSKSNGAIAWSNQTSFTCQDIFKETNATYPSSDVPCDATLTE
KSFETDMNLNFQNLLVIVLRILLLKVAGFNLLMTLRLWSSRAKRSGSGATNFSLLKQAG
DVEENPGPMHTRLLCWAALCLLGAELTEAGVAQSPRYKIIEKRQSVAFWCNPISGH
ATLYWYQQILGQGPKLLIQFQNNGVVDDSQLPKDRFSAERLKGVDSTLKIQPAKLED
SAVYLCASSLGQGQTQYFGPGTRLLVLEDLRNVTPPKVSLFEPSKAEIANKQKATLVCL
ARGFFPDHVELSWWVNGKEVHSGVCTDPQAYKESNYSYCLSSRLRVSATFWHNPRNHFR
CQVQFHGLSEEDKWPEGSPKPVTQNISAEAWGRADCGITSASYQQGVLSATILYEILLGKA
TLYAVLVSTLVVMAMVKRKNS (SEQ ID NO: 580)

The statistics for TCR **4268-TCR3** of Patient 4268 are set forth in Table 27 below.

**TABLE 27**

| **Parameter** | # | **Frequency** |
|---|---|---|
| Total wells | 96 | 100% |
| CDR3alpha | 42 | 43.8% |
| CDR3beta | 37 | 38.5% |
| 4268-TCR3 pairs | 31 | 32.3% |
| Total paired TCRs | 35 | 36.5% |

The sequence of **4268-TCR4,** which was isolated from Patient 4268, is set forth below. Starting from the amino terminus, the first underlined region is the CDR1alpha (SEQ ID NO: 167), the second underlined region is the CDR2alpha (SEQ ID NO: 168), the third underlined region is the CDR3alpha (SEQ ID NO: 169), the fourth underlined region is the CDR1beta (SEQ ID NO: 170), the fifth underlined region is the CDR2beta (SEQ ID NO: 171), and the sixth underlined region is the CDR3beta (SEQ ID NO: 172). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 173) includes the sequence starting from the amino terminus and ending immediately prior to the start of the alpha chain constant region. The beta chain variable region (SEQ ID NO: 174) includes the sequence starting immediately after the linker and ending immediately prior to the start of the beta chain constant region. The full-length alpha chain (SEQ ID NO: 175) includes the sequence starting from the amino terminus and ending immediately prior to the start of the linker. The full-length beta chain (SEQ ID NO: 176) includes the sequence starting immediately after the linker and ending with the carboxyl terminus.

Cancer reactive T cells were identified using the screening method set forth below. The method used to isolate the TCR is set forth below.
**TCR name: 4268-TCR4**
**Recognition of p53 mutation: R248Q**
**Method: p53 "hotspot" mutation universal screening**
**Co-culture to identify TCR: Co-culture 4268-F18 with p53-R248Q long peptide, sorted CD4+41BB+ T cells**
**Method to identify TCR: single-cell RT-PCR**
**Abundance of TCR amongst all paired TCRs: 95.2% (observed 40 times of 42 pairs)**
**TCR orientation: alpha-beta**
**Expression vector: SB transposon**

MALLLVPAFQVIFTLGGTRAQSVTQLDSQVPVFEEAPVELRCNYSSSVSVYLFWYVQ
YPNQGLQLLLKYLSGSTLVESINGFEAEFNKSQTSFHLRKPSVHISDTAEYFCAVRGSS
GTYKYIFGTGTRLKVLANIQNPEPAVYQLKDPRSQDSTLCLFTDFDSQINVPKTMESGTF
ITDKCVLDMKAMDSKSNGAIAWSNQTSFTCQDIFKETNATYPSSDVPCDATLTEKSFETD
MNLNFQNLLVIVLRILLLKVAGFNLLMTLRLWSSRAKRSGSGATNFSLLKQAGDVEEN
PGPMHIRLLCRVAFCFLAVGLVDVKVTQSSRYLVKRTGEKVFLECVQDMDHENMF
WYRQDPGLGLRLIYFSYDVKMKEKGDIPEGYSVSREKKERFSLILESASTNQTSMYL
CASKGDQNTEAFFGQGTRLTVVEDLRNVTPPKVSLFEPSKAEIANKQKATLVCLARGFF

PDHVELSWWVNGKEVHSGVCTDPQAYKESNYSYCLSSRLRVSATFWHNPRNHFRCQVQF
HGLSEEDKWPEGSPKPVTQNISAEAWGRADCGITSASYQQGVLSATILYEILLGKATLYAVL
VSTLVVMAMVKRKNS (SEQ ID NO: 581)

The statistics for TCR **4268-TCR4** of Patient 4268 are set forth in Table 28 below.

**TABLE 28**

| **Parameter** | # | **Frequency** |
|---|---|---|
| Total wells | 96 | 100% |
| CDR3alpha | 43 | 44.8% |
| CDR3beta | 53 | 55.2% |
| 4268-TCR4 pairs | 40 | 41.7% |
| Total paired TCRs | 42 | 43.8% |

The sequence of **4268-TCR5,** which was isolated from Patient 4268, is set forth below. Starting from the amino terminus, the first underlined region is the CDR1alpha (SEQ ID NO: 177), the second underlined region is the CDR2alpha (SEQ ID NO: 178), the third underlined region is the CDR3alpha (SEQ ID NO: 179), the fourth underlined region is the CDR1beta (SEQ ID NO: 180), the fifth underlined region is the CDR2beta (SEQ ID NO: 181), and the sixth underlined region is the CDR3beta (SEQ ID NO: 182). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 183) includes the sequence starting from the amino terminus and ending immediately prior to the start of the alpha chain constant region. The beta chain variable region (SEQ ID NO: 184) includes the sequence starting immediately after the linker and ending immediately prior to the start of the beta chain constant region. The full-length alpha chain (SEQ ID NO: 185) includes the sequence starting from the amino terminus and ending immediately prior to the start of the linker. The full-length beta chain (SEQ ID NO: 186) includes the sequence starting immediately after the linker and ending with the carboxyl terminus.

Cancer reactive T cells were identified using the screening method set forth below. The method used to isolate the TCR is set forth below.
**TCR name: 4268-TCR5**
**Recognition of p53 mutation: R248Q**
**Method: p53 "hotspot" mutation universal screening**
**Co-culture to identify TCR: Co-culture 4268-F19 with p53-mut-TMG, sorted CD4+41BB+ T cells**
**Method to identify TCR: single-cell RT-PCR**
**Abundance of TCR amongst all paired TCRs: 11.8% (observed 2 times of 17 pairs)**
**TCR orientation: alpha-beta**
**Expression vector: SB transposon**

MAGVFLLYVSMKMGGTTGQNIDQPTEMTATEGAIVQINCTYQTSGFNGLFWYQQH
AGEAPTFLSYNVLDGLEEKGRFSSFLSRSKGYSYLLLKELQMKDSASYLCAVRDLQT
GANNLFFGTGTRLTVIPNIQNPEPAVYQLKDPRSQDSTLCLFTDFDSQINVPKTMESGTFI
TDKCVLDMKAMDSKSNGAIAWSNQTSFTCQDIFKETNATYPSSDVPCDATLTEKSFETDM
NLNFQNLLVIVLRILLLKVAGFNLLMTLRLWSSRAKRSGSGATNFSLLKQAGDVEENP
GPMHIRLLCRVAFCFLAVGLVDVKVTQSSRYLVKRTGEKVFLECVQDMDHENMFW
YRQDPGLGLRLIYFSYDVKMKEKGDIPEGYSVSREKKERFSLILESASTNQTSMYLCA
SSLTFGTTEAFFGQGTRLTVVEDLRNVTPPKVSLFEPSKAEIANKQKATLVCLARGFFPD
HVELSWWVNGKEVHSGVCTDPQAYKESNYSYCLSSRLRVSATFWHNPRNHFRCQVQFHG
LSEEDKWPEGSPKPVTQNISAEAWGRADCGITSASYQQGVLSATILYEILLGKATLYAVLVST
LVVMAMVKRKNS (SEQ ID NO: 582)

The statistics for TCR **4268-TCR5** of Patient 4268 are set forth in Table 29 below.

**TABLE 29**

| Parameter | # | Frequency |
|---|---|---|
| Total wells | 96 | 100% |
| CDR3alpha | 2 | 2.1% |
| CDR3beta | 87 | 90.6% |
| 4268-TCR5 pairs | 2 | 2.1% |
| Total paired TCRs | 17 | 17.7% |

### EXAMPLE 10

This example demonstrates the identification of anti-mutated p53 T cells in Patient 4266 by co-culturing autologous APCs induced to express mutated p53 within autologous T cells ("p53 hotspot mutation universal screening"). This example also demonstrates the isolation of four anti-mutated p53 TCRs from patient 4266.

Experiments were carried out as described for Figures 25-31 for Patient 4266.

TIL fragments (F1-F24, n=24) from patient 4266 were co-cultured with autologous APCs electroporated with TMG composed of irrelevant, WT p53, or mutated p53 sequence. Co-cultures were performed overnight at 37 °C. Secretion of IFN-γ was evaluated using ELISPOT assay. The results are shown in Figure 25. Expression of 4-1BB was evaluated by flow cytometry after gating for lymphocytes → living cells (PI negative) → CD3+ (T cells). The results are shown in Figure 26.

TIL fragments (F1-F24, n=24) from patient 4266 were co-cultured with autologous APCs pulsed with peptide vehicle (DMSO) or purified (>95% by HPLC) 25-amino acid peptides composed of WT p53-R248 sequence or mutated p53-R248W sequence. Co-cultures were performed overnight at 37 °C. Secretion of IFN-γ was evaluated using ELISPOT assay. The results are shown in Figure 27. Expression of 4-1BB was evaluated by flow cytometry after gating for lymphocytes → living cells (PI negative) → CD3+ (T cells). The results are shown in Figure 28.

Cos7 cells (2.5×10⁴ per well) were plated on wells of flat-bottom 96 well plates. The following day, cells were co-transfected with individual HLA alleles from patient 4266. The next day cells were pulsed with no peptide, DMSO, WT p53-R248 peptide SSCMGGMNRR (SEQ ID NO: 590) or mutated p53-R248W peptide SSCMGGMNWR (SEQ ID NO: 591) for 2 hours at 37 °C at 1 µg/mL. TIL cultures from from patient 4266 (10⁵) were added to wells and co-cultured overnight at 37°C. Expression of 4-1BB was evaluated by flow cytometry after gating for lymphocytes → living cells (PI negative) → CD3+ (T cells) → CD4-CD8+. The results are shown in Fig. 29.

T cells expressing mock (no TCR), 4266-TCR1, 4266-TCR2, 4266-TCR3 or 4266-TCR4 with putative specificity to p53-R248W identified from 4266-TIL were co-cultured with autologous APCs which were pulsed with peptide vehicle (DMSO) or purified (>95% by HPLC) 25 amino acid peptides composed of WT p53-R248 sequence or mutated p53-R248W sequence. Media alone and PMA and Ionomycin were negative and positive controls, respectively. Co-cultures were performed overnight at 37 °C. Expression of 4-1BB was evaluated by flow cytometry after gating for lymphocytes → living cells (PI negative) → CD3+ (T cells) → CD4-CD8+. The results are shown in Fig. 30.

A tumor cell (TC) line was established from a xenografted tumor fragment resected from Patient 4266 then serially passaged through immunocompromised mice (TC#4266). The TC#4266 was co-cultured with T cells (10⁵) expressing mock (no TCR) or p53-R248W-specific TCRs (4266-TCR2, 4266-TCR3 or 4266-TCR4) overnight at 37°C. The TC#4266 cells were either incubated with nothing, W6/32 pan-HLA Class-I specific blocking antibody, IVA12 pan-HLA Class-II specific blocking antibody or mutated p53-R248W peptide SSCMGGMNWR (SEQ ID NO: 591) for 2 hours at 37 °C. The antibodies were kept in the co-culture at 5 µg/mL final concentration. The peptide was incubated at 1 µg/mL and excess peptide was washed after incubation. Media alone (no TC) and PMA and Ionomycin were negative and positive controls, respectively. Expression of 4-1BB was evaluated by flow cytometry after gating for lymphocytes → living cells (PI negative) → CD3+ (T cells) → CD4-CD8+. The results are shown in Fig. 31.

The sequence of **4266-TCR1,** which was isolated from Patient 4266, is set forth below. Starting from the amino terminus, the first underlined region is the CDR1alpha (SEQ ID NO: 97), the second underlined region is the CDR2alpha (SEQ ID NO: 98), the third underlined region is the CDR3alpha (SEQ ID NO: 99), the fourth underlined region is the CDR1beta (SEQ ID NO: 100), the fifth underlined region is the CDR2beta (SEQ ID NO: 101), and the sixth underlined region is the CDR3beta (SEQ ID NO: 102). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 103) includes the sequence starting from the amino terminus and ending immediately prior to the start of the alpha chain constant region. The beta chain variable region (SEQ ID NO: 104) includes the sequence starting immediately after the linker and ending immediately prior to the start of the beta chain constant region. The full-length alpha chain (SEQ ID NO: 105) includes the sequence starting from the amino terminus and ending immediately prior to the start of the linker. The full-length beta chain (SEQ ID NO: 106) includes the sequence starting immediately after the linker and ending with the carboxyl terminus.

Cancer reactive T cells were identified using the screening method set forth below. The method used to isolate the TCR is set forth below.
**TCR name: 4266-TCR1**
**Recognition of p53 mutation: R248W**
**Screening method: p53 "hotspot" mutation universal screening**
**Co-culture to identify TCR: Co-culture 4266-F1, 4266-F3, 4266-F5 and 4266-F6 with p53mutTMG or R248W long peptide (both co-cultures detected the same TCR), sorted CD8+41BB+ T cells**
**Method to identify TCR: single-cell RT-PCR**
**Abundance of TCR amongst all paired TCRs: 17.0% (observed 9 times of 53 pairs)**
**TCR orientation: alpha-beta**
**Expression vector: SB transposon**

MALLLVPAFQVIFTLGGTRAQSVTQLDSQVPVFEEAPVELRCNYSSSVSVYLFWYVQ
YPNQGLQLLLKYLSGSTLVESINGFEAEFNKSQTSFHLRKPSVHISDTAEYFCAVSDL
VRDDKIIFGKGTRLHILPNIQNPEPAVYQLKDPRSQDSTLCLFTDFDSQINVPKTMESGTF
ITDKCVLDMKAMDSKSNGAIAWSNQTSFTCQDIFKETNATYPSSDVPCDATLTEKSFETD
MNLNFQNLLVIVLRILLLKVAGFNLLMTLRLWSSRAKRSGSGATNFSLLKQAGDVEEN
PGPMHIGLLCCVAFSLLWASPVNAGVTQTPKFQVLKTGQSMTLQCAQDMNHNSMY
WYRQDPGMGLRLIYYSASEGTTDKGEVPNGYNVSRLNKREFSLRLESAAPSQTSVYF
CASIGGFEAFFGQGTRLTVVEDLRNVTPPKVSLFEPSKAEIANKQKATLVCLARGFFPDH
VELSWWVNGKEVHSGVCTDPQAYKESNYSYCLSSRLRVSATFWHNPRNHFRCQVQFHGL
SEEDKWPEGSPKPVTQNISAEAWGRADCGITSASYQQGVLSATILYEILLGKATLYAVLVSTL
VVMAMVKRKNS (SEQ ID NO: 583)

The statistics for TCR **4266-TCR1** of Patient 4266 are set forth in Table 30 below.

**TABLE 30**

| **Parameter** | **#** | **Frequency** |
|---|---|---|
| Total wells | 96 | 100% |
| CDR3alpha | 9 | 9.4% |
| CDR3beta | 10 | 10.4% |
| 4266-TCR1 pairs | 9 | 9.4% |
| Total paired TCRs | 53 | 55.2% |

The sequence of **4266-TCR2,** which was isolated from Patient 4266, is set forth below. Starting from the amino terminus, the first underlined region is the CDR1alpha (SEQ ID NO: 107), the second underlined region is the CDR2alpha (SEQ ID NO: 108), the third underlined region is the CDR3alpha (SEQ ID NO: 109), the fourth underlined region is the CDR1beta (SEQ ID NO: 110), the fifth underlined region is the CDR2beta (SEQ ID NO: 111), and the sixth underlined region is the CDR3beta (SEQ ID NO: 112). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 113) includes the sequence starting from the amino terminus and ending immediately prior to the start of the alpha chain constant region. The beta chain variable region (SEQ ID NO: 114) includes the sequence starting immediately after the linker and ending immediately prior to the start of the beta chain constant region. The full-length alpha chain (SEQ ID NO: 115) includes the sequence starting from the amino terminus and ending immediately prior to the start of the linker. The full-length beta chain (SEQ ID NO: 116) includes the sequence starting immediately after the linker and ending with the carboxyl terminus.

Cancer reactive T cells were identified using the screening method set forth below. The method used to isolate the TCR is set forth below.
**TCR name: 4266-TCR2**
**Recognition of p53 mutation: R248W**
**Screening method: p53 "hotspot" mutation universal screening**
**Co-culture to identify TCR: Co-culture 4266-F1, 4266-F3, 4266-F5 and 4266-F6 with p53mutTMG or R248W long peptide (both co-cultures detected the same TCR), sorted CD8+41BB+ T cells**
**Method to identify TCR: single-cell RT-PCR**
**Abundance of TCR amongst all paired TCRs: 24.5% (observed 13 times of 53 pairs)**
**TCR orientation: alpha-beta**
**Expression vector: SB transposon**

MAGVFLLYVSMKMGGTTGQNIDQPTEMTATEGAIVQINCTYQTSGFNGLFWYQQH
AGEAPTFLSYNVLDGLEEKGRFSSFLSRSKGYSYLLLKELQMKDSASYLCAVYTGGF
KTIFGAGTRLFVKANIQNPEPAVYQLKDPRSQDSTLCLFTDFDSQINVPKTMESGTFITD
KCVLDMKAMDSKSNGAIAWSNQTSFTCQDIFKETNATYPSSDVPCDATLTEKSFETDMNL
NFQNLLVIVLRILLLKVAGFNLLMTLRLWSSRAKRSGSGATNFSLLKQAGDVEENPGP
MHTRLLCWAALCLLGAELTEAGVAQSPRYKIIEKRQSVAFWCNPISGHATLYWYQQ
ILGQGPKLLIQFQNNGVVDDSQLPKDRFSAERLKGVDSTLKIQPAKLEDSAVYLCAS
NLGGGSTDTQYFGPGTRLTVLEDLRNVTPPKVSLFEPSKAEIANKQKATLVCLARGFFP
DHVELSWWVNGKEVHSGVCTDPQAYKESNYSYCLSSRLRVSATFWHNPRNHFRCQVQFH
GLSEEDKWPEGSPKPVTQNISAEAWGRADCGITSASYQQGVLSATILYEILLGKATLYAVLV
STLVVMAMVKRKNS (SEQ ID NO: 584)

The statistics for TCR **4266-TCR2** of Patient 4266 are set forth in Table 31 below.

**TABLE 31**

| **Parameter** | **#** | **Frequency** |
|---|---|---|
| Total wells | 96 | 100% |
| CDR3alpha | 21 | 21.9% |
| CDR3beta | 18 | 18.8% |
| 4266-TCR2 pairs | 13 | 13.5% |
| Total paired TCRs | 53 | 55.2% |

The sequence of **4266-TCR3,** which was isolated from Patient 4266, is set forth below. Starting from the amino terminus, the first underlined region is the CDR1alpha (SEQ ID NO: 117), the second underlined region is the CDR2alpha (SEQ ID NO: 118), the third underlined region is the CDR3alpha (SEQ ID NO: 119), the fourth underlined region is the CDR1beta (SEQ ID NO: 120), the fifth underlined region is the CDR2beta (SEQ ID NO: 121), and the sixth underlined region is the CDR3beta (SEQ ID NO: 122). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 123) includes the sequence starting from the amino terminus and ending immediately prior to the start of the alpha chain constant region. The beta chain variable region (SEQ ID NO: 124) includes the sequence starting immediately after the linker and ending immediately prior to the start of the beta chain constant region. The full-length alpha chain (SEQ ID NO: 125) includes the sequence starting from the amino terminus and ending immediately prior to the start of the linker. The full-length beta chain (SEQ ID NO: 126) includes the sequence starting immediately after the linker and ending with the carboxyl terminus.

Cancer reactive T cells were identified using the screening method set forth below. The method used to isolate the TCR is set forth below.
**TCR name: 4266-TCR3**
**Recognition of p53 mutation: R248W**
**Screening method: p53 "hotspot" mutation universal screening**
**Co-culture to identify TCR: Co-culture 4266-F1, 4266-F3, 4266-F5 and 4266-F6 with p53mutTMG or R248W long peptide (both co-cultures detected the same TCR), sorted CD8+41BB+ T cells**
**Method to identify TCR: single-cell RT-PCR**
**Abundance of TCR amongst all paired TCRs: 34.0% (observed 18 times of 53 pairs)**
**TCR orientation: alpha-beta**
**Expression vector: SB transposon**

MAGVFLLYVSMKMGGTTGQNIDQPTEMTATEGAIVQINCTYQTSGFNGLFWYQQH
AGEAPTFLSYNVLDGLEEKGRFSSFLSRSKGYSYLLLKELQMKDSASYLCAFYYGGS
QGNLIFGKGTKLSVKPNIQNPEPAVYQLKDPRSQDSTLCLFTDFDSQINVPKTMESGTFIT
DKCVLDMKAMDSKSNGAIAWSNQTSFTCQDIFKETNATYPSSDVPCDATLTEKSFETDMN
LNFQNLLVIVLRILLLKVAGFNLLMTLRLWSSRAKRSGSGATNFSLLKQAGDVEENPG
PMHTRLLCWVVLGFLGTDHTGAGVSQSPRYKVAKRGQDVALRCDPISGHVSLFWY
QQALGQGPEFLTYFQNEAQLDKSGLPSDRFFAERPEGSVSTLKIQRTQQEDSAVYLC

ASSFGSGSTDTQYFGPGTRLTVLEDLRNVTPPKVSLFEPSKAEIANKQKATLVCLARGFF
PDHVELSWWVNGKEVHSGVCTDPQAYKESNYSYCLSSRLRVSATFWHNPRNHFRCQVQF
HGLSEEDKWPEGSPKPVTQNISAEAWGRADCGITSASYQQGVLSATILYEILLGKATLYAVL
VSTLVVMAMVKRKNS (SEQ ID NO: 585)

The statistics for TCR **4266-TCR3** of Patient 4266 are set forth in Table 32 below.

**TABLE 32**

| **Parameter** | **#** | **Frequency** |
|---|---|---|
| Total wells | 96 | 100% |
| CDR3alpha | 19 | 19.8% |
| CDR3beta | 26 | 27.1 |
| 4266-TCR3 pairs | 18 | 18.8% |
| Total paired TCRs | 53 | 55.2% |

The sequence of **4266-TCR4,** which was isolated from Patient 4266, is set forth below. Starting from the amino terminus, the first underlined region is the CDR1alpha (SEQ ID NO: 127), the second underlined region is the CDR2alpha (SEQ ID NO: 128), the third underlined region is the CDR3alpha (SEQ ID NO: 129), the fourth underlined region is the CDR1beta (SEQ ID NO: 130), the fifth underlined region is the CDR2beta (SEQ ID NO: 131), and the sixth underlined region is the CDR3beta (SEQ ID NO: 132). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 133) includes the sequence starting from the amino terminus and ending immediately prior to the start of the alpha chain constant region. The beta chain variable region (SEQ ID NO: 134) includes the sequence starting immediately after the linker and ending immediately prior to the start of the beta chain constant region. The full-length alpha chain (SEQ ID NO: 135) includes the sequence starting from the amino terminus and ending immediately prior to the start of the linker. The full-length beta chain (SEQ ID NO: 136) includes the sequence starting immediately after the linker and ending with the carboxyl terminus.

Cancer reactive T cells were identified using the screening method set forth below. The method used to isolate the TCR is set forth below.
**TCR name: 4266-TCR4**
**Recognition of p53 mutation: R248W**
**Screening method: p53 "hotspot" mutation universal screening**
**Co-culture to identify TCR: Co-culture 4266-F1, 4266-F3, 4266-F5 and 4266-F6 with p53mutTMG or R248W long peptide (both co-cultures detected the same TCR), sorted CD8+41BB+ T cells**
**Method to identify TCR: single-cell RT-PCR**
**Abundance of TCR amongst all paired TCRs: 9.4% (observed 5 times of 53 pairs)**
**TCR orientation: alpha-beta**
**Expression vector: SB transposon**

MAGVFLLYVSMKMGGTTGQNIDQPTEMTATEGAIVQINCTYQTSGFNGLFWYQQH
AGEAPTFLSYNVLDGLEEKGRFSSFLSRSKGYSYLLLKELQMKDSASYLCAVYPGGS
QGNLIFGKGTKLSVKPNIQNPEPAVYQLKDPRSQDSTLCLFTDFDSQINVPKTMESGTFIT
DKCVLDMKAMDSKSNGAIAWSNQTSFTCQDIFKETNATYPSSDVPCDATLTEKSFETDMN
LNFQNLLVIVLRILLLKVAGFNLLMTLRLWSSRAKRSGSGATNFSLLKQAGDVEENPG
PMHTRLLCWAALCLLGAELTEAGVAQSPRYKIIEKRQSVAFWCNPISGHATLYWYQ
QILGQGPKLLIQFQNNGVVDDSQLPKDRFSAERLKGVDSTLKIQPAKLEDSAVYLCA
SSLGTGSTDTQYFGPGTRLTVLEDLRNVTPPKVSLFEPSKAEIANKQKATLVCLARGFFP
DHVELSWWVNGKEVHSGVCTDPQAYKESNYSYCLSSRLRVSATFWHNPRNHFRCQVQFH
GLSEEDKWPEGSPKPVTQNISAEAWGRADCGITSASYQQGVLSATILYEILLGKATLYAVLV
STLVVMAMVKRKNS (SEQ ID NO: 586)

The statistics for TCR **4266-TCR4** of Patient 4266 are set forth in Table 33 below.

**TABLE 33**

| **Parameter** | **#** | **Frequency** |
|---|---|---|
| Total wells | 96 | 100% |
| CDR3alpha | 5 | 5.2% |
| CDR3beta | 13 | 13.5 |
| 4266-TCR3 pairs | 5 | 5.2% |
| Total paired TCRs | 53 | 55.2% |

### EXAMPLE 11

This example demonstrates the identification of anti-mutated p53 T cells in Patient 4252 by co-culturing autologous APCs induced to express mutated p53 within autologous T cells ("p53 hotspot mutation universal screening").

TIL fragments F1, F3, F7 and F19 patient 4252 were used as effectors. Co-cultures with T cell effectors and autologous APCs (immature DCs) were either (1) electroporated with TMGs composed of irrelevant, WT p53 or mutated p53 sequences or (2) pulsed with peptide vehicle (DMSO) or purified (>95% by HPLC) 25 amino acid peptides composed of WT p53-R175 sequence or mutated p53-R175H sequence. T cells only (no target) was negative control and PMA and Iono was positive control. Co-cultures were performed overnight at 37 °C. Expression of 4-1BB on CD3+CD8-CD4+ T cells was evaluated by flow cytometry. The results are shown in Figure 38.

### EXAMPLE 12

This example demonstrates the identification of anti-mutated p53 T cells in Patient 4270 by co-culturing autologous APCs induced to express mutated p53 within autologous T cells ("p53 hotspot mutation universal screening").

TIL fragments F13 and F16 patient 4270 were used as effectors. Co-cultures with T cell effectors and autologous APCs (immature DCs) were either (1) electroporated with TMGs composed of irrelevant, WT p53 or mutated p53 sequences or (2) pulsed with peptide vehicle (DMSO) or purified (>95% by HPLC) 25 amino acid peptides composed of WT p53-R282 sequence or mutated p53-R282W sequence. T cells only was negative control and PMA and Iono was positive control. Co-cultures were performed overnight at 37 °C. Secretion of IFN-γ was evaluated using ELISPOT assay. The results are shown in Figure 39.

### EXAMPLE 13

This example demonstrates the identification of anti-mutated p53 T cells in Patient 4285 by co-culturing autologous APCs induced to express mutated p53 within autologous T cells ("p53 hotspot mutation universal screening").

TIL fragments (F1-F22 and F24, n=18) from patient 4285 were co-cultured with autologous APCs electroporated with TMGs composed of irrelevant, WT p53 or mutated p53 sequences. Co-cultures were performed overnight at 37 °C. Secretion of IFN-γ was evaluated using ELISPOT assay. The results are shown in Figure 40. Expression of 4-1BB was evaluated by flow cytometry after gating for lymphocytes → living cells (PI negative) → CD3+ (T cells). The results are shown in Figure 42.

TIL fragments (F1-F22 and F24, n=18) from patient 4285 were co-cultured with autologous APCs pulsed with peptide vehicle (DMSO) or purified (>95% by HPLC) 25 amino acid peptides composed of WT p53-R175 sequence or mutated p53-R175H sequence. Co-cultures were performed overnight at 37 °C. Secretion of IFN-γ was evaluated using ELISPOT assay. The results are shown in Figure 41. Expression of 4-1BB was evaluated by flow cytometry after gating for lymphocytes → living cells (PI negative) → CD3+ (T cells). The results are shown Figure 43.

Autologous APCs were pulsed with 15-amino acid peptides from the p53-R175H sequence (amino acid substitution underlined in Table 34) overlapping 14 amino acids. TIL from patient 4285 (fragment cultures 10, 6 and 9) with specificity to p53-R175H were co-cultured overnight at 37 °C with peptide-pulsed APCs. DMSO was peptide vehicle. Secretion of IFN-γ was evaluated using ELISPOT assay. The results are shown in Table 34.

**TABLE 34**

| | | **ELISPOT Result Positive (+) or negative (-) for IFN-γ production** | | |
|---|---|---|---|---|
| **Peptide** | **SEQ ID NO:** | **4285-F10** | **4285-F6** | **4285-F9** |
| None (vehicle) | Not applicable | - | - | - |
| YKQSQHMTEVVR**H**CP | 519 | - | - | - |
| KQSQHMTEVVR**H**CPH | 520 | - | - | + |
| QSQHMTEVVR**H**CPHH | 521 | - | + | + |
| SQHMTEVVR**H**CPHHE | 522 | + | + | + |
| QHMTEVVR**H**CPHHER | 523 | + | + | + |
| HMTEVVR**H**CPHHERC | 524 | + | + | + |
| MTEVVR**H**CPHHERCS | 525 | + | + | + |
| TEVVR**H**CPHHERCSD | 526 | + | + | + |
| EVVR**H**CPHHERCSDS | 527 | + | + | + |
| VVR**H**CPHHERCSDSD | 528 | + | + | - |
| VR**H**CPHHERCSDSDG | 529 | + | + | - |

Cos7 cells (2.5×10⁴ per well) were plated on wells of flat-bottom 96 well plates. The following day, cells were co-transfected with individual HLA alleles from patient 4285. The next day cells were pulsed with DMSO or mutated p53-R175H peptide YKQSQHMTEVVRHCPHHERCSDSDG (SEQ ID NO: 2) for 2 hours at 37 °C at 10 µg/mL. Selected TIL fragment cultures with specificity to p53-R175H from Patient 4285 (4285-F6, 4285-F9 and 4285-F10) were co-cultured with transfected Cos7 cells overnight at 37 °C. Expression of 4-1BB was assayed by flow cytometry after gating lymphocytes → live → CD3+ (T cells) → CD8-CD4+. The results are shown in Figure 54.

Autologous APCs were pulsed with decreasing concentrations of 25- or 15-amino acid peptides corresponding to the WT or mutated p53-R175H sequence for 2 hours at 37 °C. T cells transposed with 4285-TCR1 from patient 4285 were co-cultured overnight at 37 °C with peptide-pulsed APCs. Expression of 4-1BB was assayed by flow cytometry after gating lymphocytes → live → CD3+ (T cells) → CD8-CD4+. The results are shown in Figure 55.

The sequence of **4285-TCR1,** which was isolated from Patient 4285, is set forth below. Starting from the amino terminus, the first underlined region is the CDR1alpha (SEQ ID NO: 487), the second underlined region is the CDR2alpha (SEQ ID NO: 488), the third underlined region is the CDR3alpha (SEQ ID NO: 489), the fourth underlined region is the CDR1beta (SEQ ID NO: 490), the fifth underlined region is the CDR2beta (SEQ ID NO: 491), and the sixth underlined region is the CDR3beta (SEQ ID NO: 492). The bold region is the linker (SEQ ID NO: 26). Starting from the amino terminus, the first italicized region is the alpha chain constant region (SEQ ID NO: 23) and the second italicized region is the beta chain constant region (SEQ ID NO: 25). The alpha chain variable region (SEQ ID NO: 493) includes the sequence starting from the amino terminus and ending immediately prior to the start of the alpha chain constant region. The beta chain variable region (SEQ ID NO: 494) includes the sequence starting immediately after the linker and ending immediately prior to the start of the beta chain constant region. The full-length alpha chain (SEQ ID NO: 495) includes the sequence starting from the amino terminus and ending immediately prior to the start of the linker. The full-length beta chain (SEQ ID NO: 496) includes the sequence starting immediately after the linker and ending with the carboxyl terminus.

Cancer reactive T cells were identified as described below. The TCR was isolated as described below.
**TCR name: 4285-TCR1**
**Recognition of p53 mutation: R175H**
**Screening method: p53 "hotspot" mutation universal screening**
**Co-culture to identify TCR: Co-culture 4285-F6 with p53-R175H peptide and sorted CD4+41BB+ T cells**
**Method to identify TCR: single-cell RT-PCR**
**Abundance of TCR amongst all paired TCRs: 81.8% (observed 36 times of 44 pairs)**
**TCR orientation: alpha-beta**
**Expression vector: SB transposon**

MAKNPLAAPLLILWFHLDCVSSILNVEQSPQSLHVQEGDSTNFTCSFPSSNFYALHWY
RWETAKSPEALFVMTLNGDEKKKGRISATLNTKEGYSYLYIKGSQPEDSATYLCALI
TGGGNKLTFGTGTQLKVELNIQNPEPAVYQLKDPRSQDSTLCLFTDFDSQINVPKTMES
GTFITDKCVLDMKAMDSKSNGAIAWSNQTSFTCQDIFKETNATYPSSDVPCDATLTEKSFE
TDMNLNFQNLLVIVLRILLLKVAGFNLLMTLRLWSSRAKRSGSGATNFSLLKQAGDVE
ENPGPMHLGLLCCGAFSLLWAGPVNAGVTQTPKFRVLKTGQSMTLLCAQDMNHEY
MYWYRQDPGMGLRLIHYSVGEGTTAKGEVPDGYNVSRLKKQNFLLGLESAAPSQTS
VYFCASRLQGWNSPLHFGNGTRLTVTEDLRNVTPPKVSLFEPSKAEIANKQKATLVCLA
RGFFPDHVELSWWVNGKEVHSGVCTDPQAYKESNYSYCLSSRLRVSATFWHNPRNHFRC
QVQFHGLSEEDKWPEGSPKPVTQNISAEAWGRADCGITSASYQQGVLSATILYEILLGKAT
LYAVLVSTLVVMAMVKRKNS (SEQ ID NO: 589)

The statistics for 4285-TCR1 from patient 4285 are set forth in Table 35.

**TABLE 35**

| **Parameter** | **#** | **Frequency** |
|---|---|---|
| Total wells | 96 | 100% |
| CDR3alpha | 37 | 39.8% |
| CDR3beta | 39 | 40.6% |
| 4285-TCR1 pairs | 36 | 37.5% |
| Total paired TCRs | 44 | 45.8% |

### EXAMPLE 14

This example demonstrates a summary of responses to p53 "hotspot" mutations by T cells.

A summary of responses to p53 "hotspot" mutations by T cells is provided in Table 36. Numbers 1-15 of Table 36 were a retrospective study. Numbers 16-33 of Table 36 were a prospective study.

**TABLE 36**

| # | **Tumor type** | **Patient** | **p53 mut** | **T cell screen** | **HLA** |
|---|---|---|---|---|---|
| 1 | Gastric | 3446 | G245S | n/a | n/a |
| 2 | Gastroesophageal | 3788 | Y220C | N | n/a |
| 3 | Rectal | 3942 | R273C | n/a | n/a |
| 4 | Colon | 4023 | R282W | Y | Class-II |
| 5 | Colon | 4095 | R282W | n/a | n/a |
| 6 | Ovarian | 4127 | G245S | Y | DRB3*02:02 |
| 7 | Breast | 4130 | R273H | Y | Class-II |
| 8 | Colon | 4141 | R175H | Y | A*02:01 |
| 9 | Ovarian | 4149 | Y220C | Y | DRB3*02:02 |
| 10 | Colon | 4160 | R273H | N | n/a |
| 11 | Melanoma | 4165 | G245D | N | n/a |
| 12 | Colon | 4166 | R248W | n/a | n/a |
| 13 | Rectal | 4171 | R248Q | N | n/a |
| 14 | Melanoma | 4187 | R273H | N | n/a |
| 15 | Colon | 4196 | R175H | Y | A*02:01 |
| 16 | Colon | 4213 | R248Q | Y | both |
| 17 | Colon | 4217 | R175H | N | n/a |
| 18 | Cholangiocarcinoma | 4220 | R248Q | N | n/a |
| 19 | Rectal | 4235 | R273C | N | n/a |
| 20 | Colon | 4238 | R248Q | Y | Class-I |
| 21 | Colon | 4244 | R282W | N | n/a |
| 22 | Colon | 4245 | R248Q | N | n/a |
| 23 | Colon | 4252 | R175H | N | n/a |
| 24 | Melanoma | 4253 | R248W | Y | unknown |
| 25 | Colon | 4254 | R273H | N | n/a |
| 26 | Colon | 4257 | R248W | N | n/a |
| 27 | Endometrial | 4258 | R273H | N | n/a |
| 28 | Colon | 4259 | Y220C | Y | A*02:01, DRB1*04 |
| 29 | Colon | 4266 | R248W | Y | A*68:01 |
| 30 | Colon | 4268 | R248Q | Y | both |
| 31 | Pancreatic | 4270 | R282W | Y | unknown |
| 32 | Rectal | 4273 | R248W | Y | DPB1*02:01 |
| 33 | Rectal | 4274 | R175H | n/a | |
| 34 | Colon | 4283 | R175H | N | n/a |
| 35 | Colon | 4285 | R175H | Y | DRB1*13:01 |
| 36 | Colon | 4287 | R248W | N | n/a |
| 37 | Colon | 4312 | R175H | N | n/a |

| | | | | | |
|---|---|---|---|---|---|
| n/a=not applicable; N=negative; Y=confirmed reactive; TBS=to be screened. | | | | | |

### EXAMPLE 15

This example demonstrates the treatment of patients with p53 mutation-reactive TIL.

A summary of the treatment of patients with p53 mutation-reactive TIL is provided in Table 37.

**TABLE 37**

| # | **Tumor Type** | **Patient** | **p53 mut** | **Infusion bag screening** | **Total # of Cells (x10⁹)** | **% of p53 reactive TIL** | **# of p53 reactive TIL (x10⁹)** | **Response** | **Duration (months)** |
|---|---|---|---|---|---|---|---|---|---|
| 1 | Ovarian | 4127 | G245S | Y | 143 | 2.8 | 4.0 | P.R. | 4 |
| 2 | Colon | 4141 | R175H | Y | 69 | 0.8 | 0.6 | N.R. | - |
| 3 | Colon | 4196 | R175H | Y | 92 | 3.3 | 3.0 | N.R. | - |
| 4 | Ovarian | 4149 | Y220C | Y | 37 | 11.1 | 4.1 | N.R. | - |
| 5 | Colon | 4213 | R248Q | TBS | 33 | | | P.R. | 4 |
| 6 | Colon | 4238 | R248Q | TBS | 57 | | | N.R. | |
| 7 | Colon | 4266 | R248W | Y | 104 | 50.8 | 52.8 | | |
| 8 | Colon | 4268 | R248Q | **TBS | | | | | |
| 9 | Rectal | 4273 | R248W | **Y | 117 | 6.8 | 8.0 | N.R. | - |
| 10 | Colon | 4285 | R175H | Y | 69.6 | 2.4 | 1.7 | N.R. | - |

| | | | | | | | | | |
|---|---|---|---|---|---|---|---|---|---|
| NT=not treated; TBS=to be screened; N.R.=no response; P.R.=partial response; **patient not yet treated. | | | | | | | | | |

### EXAMPLE 16

This example demonstrates the overall frequencies of each missense p53 mutation in all of the tumors sequenced.

Samples were acquired as follows: Tumor and PBL were collected from 141 cancer patients. TIL were grown from tumors. Tumor samples underwent exome and transcriptome sequencing. Tp53 mutations were identified. Out of 150 tumors, 30% expressed WT p53, and 70% expressed mutated p53. Out of 122 tumor samples which expressed mutated p53, 73% expressed a missense mutation, 13% expressed a stop-gain mutation, 11% expressed a frameshift mutation, and 3% expressed an indel mutation.

The overall frequencies of each missense p53 mutation in all of the tumors sequenced are shown in Figure 44.

### EXAMPLE 17

This example demonstrates a summary of the reactivity of TCRs of Examples 1-15.

The TCRs of Table 38 were isolated, expressed in T cells and tested against the relevant antigen. A summary of the results is shown in Table 38.

**TABLE 38**

| **TCR name** | **p53 a.a. substitution** | **TCR tested?** | **p53 mutation reactive?** |
|---|---|---|---|
| 4127-TP53-G245S-TCR1 | G245S | yes | yes |
| 4149TCRa2b2 | Y220C | yes | yes |
| 4266-TCR1 | R248W | yes | no |
| 4266-TCR2 | R248W | yes | yes |
| 4266-TCR3 | R248W | yes | yes |
| 4266-TCR4 | R248W | yes | yes |
| 4268-TCR1 | R248Q | yes | no |
| 4268-TCR2 | R248Q | yes | no |
| 4268-TCR3 | R248Q | yes | no |
| 4268-TCR4 | R248Q | yes | no |
| 4268-TCR5 | R248Q | yes | no |
| 4273-TP53-R248W-TCR1a1 | R248W | yes | no |
| 4273-TP53-R248W-TCR1a2 | R248W | yes | Yes |
| 4141-TCR1a2 | R175H | yes | yes |
| 4285-TCR1 | R175H | yes | yes |
| 4259-F6-TCR | Y220C | yes | yes |
| 4127-O37-TCR | G245S | yes | yes |

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A method of isolating T cells having antigenic specificity for a mutated human p53 amino acid sequence encoded by a cancer-specific p53 mutation, the method comprising:
inducing autologous antigen presenting cells (APCs) of a patient to present at least one mutated human p53 amino acid sequence, wherein inducing APCs of the patient to present the at least one mutated human p53 amino acid sequence comprises (i) or (ii):
(i) pulsing the APCs with a peptide comprising the mutated human p53 amino acid sequence or a pool of peptides, each peptide in the pool comprising a different mutated human p53 amino acid sequence; or
(ii) introducing a nucleotide sequence encoding the mutated human p53 amino acid sequence into the APCs;
co-culturing autologous T cells of the patient with the autologous APCs that present the mutated human p53 amino acid sequence; and
selecting the autologous T cells that (a) were co-cultured with the autologous APCs that present the mutated human p53 amino acid sequence and (b) have antigenic specificity for the mutated human p53 amino acid sequence presented in the context of a major histocompatibility complex (MHC) molecule expressed by the patient to provide isolated T cells having antigenic specificity for the mutated human p53 amino acid sequence encoded by the cancer-specific p53 mutation.

2. The method of claim 1, wherein the peptide or pool of peptides comprise(s) one or more mutated p53 peptides of SEQ ID NOs: 2-13.

3. The method of claim 1, wherein the nucleotide sequence introduced into the autologous APCs is a tandem minigene (TMG) construct comprising at least one minigene comprising a p53 gene, wherein, for each minigene comprising a p53 gene, each p53 gene includes a cancer-specific p53 mutation that encodes a mutated human p53 amino acid sequence, wherein each p53 gene in the TMG construct encodes a different mutated human p53 amino acid sequence.

4. The method of claim 3, wherein the TMG construct encodes one or more mutated p53 peptides of SEQ ID NOs: 2-13.

5. The method of claim 3, wherein the TMG construct encodes the amino acid sequence of SEQ ID NO: 14.

6. The method of any one of claims 1-5, wherein selecting the autologous T cells that have antigenic specificity for the mutated human p53 amino acid sequence comprises selectively growing the autologous T cells that have antigenic specificity for the mutated human p53 amino acid sequence.

7. The method of any one of claims 1-6, wherein selecting the autologous T cells that have antigenic specificity for the mutated human p53 amino acid sequence comprises selecting the T cells that express any one or more of programmed cell death 1 (PD-1), lymphocyte-activation gene 3 (LAG-3), T cell immunoglobulin and mucin domain 3 (TIM-3), 4-1BB, OX40, and CD107a.

8. The method of any one of claims 1-7, wherein selecting the autologous T cells that have antigenic specificity for the mutated human p53 amino acid sequence comprises selecting the T cells (i) that secrete a greater amount of one or more cytokines upon co-culture with APCs that present the mutated human p53 amino acid sequence as compared to the amount of the one or more cytokines secreted by a negative control or (ii) in which at least twice as many of the numbers of T cells secrete one or more cytokines upon co-culture with APCs that present the mutated human p53 amino acid sequence as compared to the numbers of negative control T cells that secrete the one or more cytokines.

9. The method of claim 8, wherein the one or more cytokines comprise interferon (IFN)-γ, interleukin (IL)-2, tumor necrosis factor alpha (TNF-α), granulocyte/monocyte colony stimulating factor (GM-CSF), IL-4, IL-5, IL-9, IL-10, IL-17, and IL-22.

10. A method of isolating a T cell receptor (TCR), or an antigen-binding portion thereof, having antigenic specificity for a mutated human p53 amino acid sequence encoded by a cancer-specific p53 mutation, the method comprising:
isolating T cells having antigenic specificity for a mutated human p53 amino acid sequence encoded by a cancer-specific p53 mutation according to the method of any one of claims 1-9; and
isolating a nucleotide sequence that encodes the TCR, or the antigen-binding portion thereof, from the selected autologous T cells, wherein the TCR, or the antigen-binding portion thereof, has antigenic specificity for the mutated human p53 amino acid sequence encoded by the cancer-specific p53 mutation.

11. A method of preparing a population of cells that express a TCR, or an antigen-binding portion thereof, having antigenic specificity for a mutated human p53 amino acid sequence encoded by a cancer-specific p53 mutation, the method comprising:
isolating a TCR, or an antigen-binding portion thereof, according to the method of claim 10, and
introducing *in vitro* the nucleotide sequence encoding the isolated TCR, or the antigen-binding portion thereof, into peripheral blood mononuclear cells (PBMC) to obtain cells that express the TCR, or the antigen-binding portion thereof.

12. A method of preparing a population of T cells that have antigenic specificity for a mutated human p53 amino acid sequence encoded by a cancer-specific p53 mutation, the method comprising:
isolating T cells according to the method of any one of claims 1-9, and
expanding the numbers of selected autologous T cells to obtain a population of T cells that have antigenic specificity for the mutated human p53 amino acid sequence encoded by the cancer-specific p53 mutation.

13. The method of claim 12, wherein expanding the numbers of cells comprises culturing the selected cells with feeder PBMC, interleukin (IL)-2, and OKT3 antibody.

## Patentansprüche

1. Verfahren zum Isolieren von T-Zellen mit antigener Spezifität für eine mutierte menschliche p53-Aminosäuresequenz, die durch eine krebsspezifische p53-Mutation kodiert wird, wobei das Verfahren umfasst:
Induzieren von autologen Antigen-präsentierenden Zellen (APCs) eines Patienten, um mindestens eine mutierte menschliche p53-Aminosäuresequenz zu präsentieren, wobei das Induzieren von APCs des Patienten, um die mindestens eine mutierte menschliche p53-Aminosäuresequenz zu präsentieren, (i) oder (ii) umfasst:
(i) Pulsieren der APCs mit einem Peptid, das die mutierte menschliche p53-Aminosäuresequenz umfasst, oder mit einem Pool von Peptiden, wobei jedes Peptid im Pool eine andere mutierte menschliche p53-Aminosäuresequenz umfasst; oder
(ii) Einführen einer Nukleotidsequenz, die die mutierte menschliche p53-Aminosäuresequenz kodiert, in die APCs;
Ko-kultivieren von autologen T-Zellen des Patienten mit den autologen APCs, die die mutierte menschliche p53-Aminosäuresequenz präsentieren; und
Auswählen der autologen T-Zellen, die (a) mit den autologen APCs, die die mutierte menschliche p53-Aminosäuresequenz präsentieren, ko-kultiviert wurden und (b) eine antigene Spezifität für die mutierte menschliche p53-Aminosäuresequenz, die im Kontext eines vom Patienten exprimierten Haupthistokompatibilitätskomplex (MHC) -Moleküls präsentiert wird, aufweisen, um isolierte T-Zellen bereitzustellen, die eine antigene Spezifität für die mutierte menschliche p53-Aminosäuresequenz, die durch die krebsspezifische p53-Mutation kodiert wird, aufweisen.

2. Verfahren nach Anspruch 1, wobei das Peptid oder der Pool von Peptiden ein oder mehrere mutierte p53-Peptide der SEQ ID NOs: 2-13 umfasst.

3. Verfahren gemäß Anspruch 1, wobei die in die autologen APCs eingeführte Nukleotidsequenz ein Tandem-Minigen-Konstrukt (TMG) ist, das mindestens ein Minigen umfasst, das ein p53-Gen umfasst, wobei für jedes Minigen, das ein p53-Gen umfasst, jedes p53-Gen eine krebsspezifische p53-Mutation umfasst, die eine mutierte menschliche p53-Aminosäuresequenz kodiert, wobei jedes p53-Gen im TMG-Konstrukt eine andere mutierte menschliche p53-Aminosäuresequenz kodiert.

4. Verfahren nach Anspruch 3, wobei das TMG-Konstrukt ein oder mehrere mutierte p53-Peptide der SEQ ID NOs: 2-13 kodiert.

5. Verfahren nach Anspruch 3, wobei das TMG-Konstrukt die Aminosäuresequenz von SEQ ID NO: 14 kodiert.

6. Verfahren nach einem der Ansprüche 1 bis 5, wobei das Auswählen der autologen T-Zellen, die eine antigene Spezifität für die mutierte menschliche p53-Aminosäuresequenz aufweisen, das selektive Züchten der autologen T-Zellen umfasst, die eine antigene Spezifität für die mutierte menschliche p53-Aminosäuresequenz aufweisen.

7. Verfahren gemäß einem der Ansprüche 1 bis 6, wobei das Auswählen der autologen T-Zellen, die eine antigene Spezifität für die mutierte menschliche p53-Aminosäuresequenz aufweisen, das Auswählen der T-Zellen, die eines oder mehrere von programmierter Zelltod 1 (PD-1), Lymphozytenaktivierungsgen 3 (LAG-3), T-Zell-Immunglobulin und Mucindomäne 3 (TIM-3), 4-1BB, OX40 und CD107a exprimieren, umfasst.

8. Verfahren nach einem der Ansprüche 1 bis 7, wobei das Auswählen der autologen T-Zellen, die eine antigene Spezifität für die mutierte menschliche p53-Aminosäuresequenz aufweisen, das Auswählen der T-Zellen umfasst, (i) die bei Ko-Kultur mit APCs, die die mutierte menschliche p53-Aminosäuresequenz präsentieren, eine größere Menge eines oder mehrerer Zytokine absondern als die Menge des einen oder der mehreren Zytokine, die von einer Negativkontrolle abgesondert werden, oder (ii) bei denen mindestens doppelt so viele T-Zellen bei Ko-Kultur mit APCs, die die mutierte menschliche p53-Aminosäuresequenz präsentieren, ein oder mehrere Zytokine absondern als die Anzahl von Negativkontroll-T-Zellen, die das eine oder die mehreren Zytokine absondern.

9. Verfahren nach Anspruch 8, wobei die ein oder mehreren Zytokine Interferon (IFN)-γ, Interleukin (IL)-2, Tumornekrosefaktor Alpha (TNF-α), Granulozyten-/Monozytenkoloniestimulierender Faktor (GM-CSF), IL-4, IL-5, IL-9, IL-10, IL-17 und IL-22 umfassen.

10. Verfahren zum Isolieren eines T-Zell-Rezeptors (TCR) oder eines Antigen-bindenden Teils davon, der eine antigene Spezifität für eine mutierte menschliche p53-Aminosäuresequenz aufweist, die durch eine krebsspezifische p53-Mutation kodiert wird, wobei das Verfahren umfasst:
Isolieren von T-Zellen mit antigener Spezifität für eine mutierte menschliche p53-Aminosäuresequenz, die durch eine krebsspezifische p53-Mutation kodiert wird, gemäß dem Verfahren eines der Ansprüche 1 bis 9; und
Isolieren einer Nukleotidsequenz, die den TCR oder den Antigen-bindenden Teil davon kodiert, aus den ausgewählten autologen T-Zellen, wobei der TCR oder der Antigen-bindende Teil davon eine antigene Spezifität für die mutierte menschliche p53-Aminosäuresequenz aufweist, die durch die krebsspezifische p53-Mutation kodiert wird.

11. Verfahren zur Herstellung einer Population von Zellen, die einen TCR oder einen Antigen-bindenden Teil davon, der eine antigene Spezifität für eine mutierte menschliche p53-Aminosäuresequenz aufweist, die durch eine krebsspezifische p53-Mutation kodiert wird, exprimieren, wobei das Verfahren umfasst:
Isolieren eines TCR oder eines Antigen-bindenden Teils davon gemäß dem Verfahren nach Anspruch 10 und
Einführen der Nukleotidsequenz, die den isolierten TCR oder den Antigen-bindenden Teil davon kodiert, in vitro in periphere mononukleäre Blutzellen (PBMC), um Zellen zu erhalten, die den TCR oder den Antigen-bindenden Teil davon exprimieren.

12. Verfahren zur Herstellung einer Population von T-Zellen, die eine antigene Spezifität für eine mutierte menschliche p53-Aminosäuresequenz aufweisen, die durch eine krebsspezifische p53-Mutation kodiert wird, wobei das Verfahren umfasst:
Isolieren von T-Zellen gemäß dem Verfahren eines der Ansprüche 1 bis 9 und
Expandieren der Anzahl ausgewählter autologer T-Zellen, um eine Population von T-Zellen zu erhalten, die eine antigene Spezifität für die mutierte menschliche p53-Aminosäuresequenz aufweisen, die durch die krebsspezifische p53-Mutation kodiert wird.

13. Verfahren nach Anspruch 12, wobei das Expandieren der Anzahl von Zellen die Kultivierung der ausgewählten Zellen mit Feeder-PBMC, Interleukin (IL)-2 und OKT3-Antikörper umfasst.

## Revendications

1. Procédé d'isolement de cellules T ayant une spécificité antigénique pour une séquence d'acides aminés de p53 humaine mutée codée par une mutation de p53 spécifique au cancer, le procédé comprenant :
l'induction de cellules autologues présentatrices d'antigène (APC) d'un patient pour présenter au moins une séquence d'acides aminés de p53 humaine mutée, sachant que l'induction d'APC du patient pour présenter l'au moins une séquence d'acides aminés de p53 humaine mutée comprend (i) ou (ii) :
(i) la stimulation pulsée des APC avec un peptide comprenant la séquence d'acides aminés de p53 humaine mutée ou un ensemble de peptides, chaque peptide de l'ensemble comprenant une séquence d'acides aminés de p53 humaine mutée différente ; ou
(ii) l'introduction d'une séquence de nucléotides codant la séquence d'acides aminés de p53 humaine mutée dans les APC ;
la mise en co-culture de cellules T autologues du patient avec les APC autologues qui présentent la séquence d'acides aminés de p53 humaine mutée ; et
la sélection des cellules T autologues qui (a) ont été mises en co-culture avec les APC autologues qui présentent la séquence d'acides aminés de p53 humaine mutée et (b) ont une spécificité antigénique pour la séquence d'acides aminés de p53 humaine mutée présentée dans le contexte d'une molécule du complexe majeur d'histocompatibilité (MHC) exprimée par le patient pour fournir des cellules T isolées ayant une spécificité antigénique pour la séquence d'acides aminés de p53 humaine mutée codée par la mutation de p53 spécifique au cancer.

2. Le procédé de la revendication 1, sachant que le peptide ou l'ensemble de peptides comprend un ou plusieurs peptides de p53 mutée de SEQ ID N° 2-13.

3. Le procédé de la revendication 1, sachant que la séquence de nucléotides introduite dans les APC autologues est une structure de minigène en tandem (TMG) comprenant au moins un minigène comprenant un gène de p53, sachant que, pour chaque minigène comprenant un gène de p53, chaque gène de p53 inclut une mutation de p53 spécifique au cancer qui code une séquence d'acides aminés de p53 humaine mutée, sachant que chaque gène de p53 dans la structure de TMG code une séquence d'acides aminés de p53 humaine mutée différente.

4. Le procédé de la revendication 3, sachant que la structure de TMG code un ou plusieurs peptides de p53 mutée de SEQ ID N° 2-13.

5. Le procédé de la revendication 3, sachant que la structure de TMG code la séquence d'acides aminés de SEQ ID N° 14.

6. Le procédé de l'une quelconque des revendications 1 à 5, sachant que la sélection des cellules T autologues qui ont une spécificité antigénique pour la séquence d'acides aminés de p53 humaine mutée comprend la croissance sélective des cellules T autologues qui ont une spécificité antigénique pour la séquence d'acides aminés de p53 humaine mutée.

7. Le procédé de l'une quelconque des revendications 1 à 6, sachant que la sélection des cellules T autologues qui ont une spécificité antigénique pour la séquence d'acides aminés de p53 humaine mutée comprend la sélection des cellules T qui expriment l'un quelconque ou plusieurs de la mort cellulaire programmée 1 (PD-1), du gène d'activation des lymphocytes 3 (LAG-3), du domaine d'immunoglobine et de mucine des cellules T 3 (TIM-3), de 4-1BB, d'OX40, et de CD107a.

8. Le procédé de l'une quelconque des revendications 1 à 7, sachant que la sélection des cellules T autologues qui ont une spécificité antigénique pour la séquence d'acides aminés de p53 humaine mutée comprend la sélection des cellules T (i) qui sécrètent une quantité plus élevée d'une ou de plusieurs cytokines lors de la mise en co-culture avec des APC qui présentent la séquence d'acides aminés de p53 humaine mutée en comparaison avec la quantité de l'une ou des plusieurs cytokines sécrétées par un témoin négatif ou (ii) où au moins deux fois autant des nombres de cellules T sécrètent une ou plusieurs cytokines lors de la mise en co-culture avec des APC qui présentent la séquence d'acides aminés de p53 humaine mutée en comparaison avec les nombres de cellules T de témoin négatif qui sécrètent l'une ou les plusieurs cytokines.

9. Le procédé de la revendication 8, sachant que l'une ou les plusieurs cytokines comprennent l'interféron (IFN)-γ, l'interleukine (IL)-2, le facteur de nécrose tumorale alpha (TNF-α), le facteur de stimulation des granulocytes et des colonies de monocytes (GM-CSF), IL-4, IL-5, IL-9, IL-10, IL-17, et IL-22.

10. Procédé d'isolement d'un récepteur de cellules T (TCR), ou d'une partie de liaison à l'antigène de celui-ci, ayant une spécificité antigénique pour une séquence d'acides aminés de p53 humaine mutée codée par une mutation de p53 spécifique au cancer, le procédé comprenant :
l'isolement de cellules T ayant une spécificité antigénique pour une séquence d'acides aminés de p53 humaine mutée codée par une mutation de p53 spécifique au cancer selon le procédé de l'une quelconque des revendications 1 à 9 ; et
l'isolement d'une séquence de nucléotides qui code le TCR, ou de la partie de liaison à l'antigène de celui-ci, par rapport aux cellules T autologues sélectionnées, sachant que le TCR, ou la partie de liaison à l'antigène de celui-ci, a une spécificité antigénique pour la séquence d'acides aminés de p53 humaine mutée codée par la mutation de p53 spécifique au cancer.

11. Procédé de préparation d'une population de cellules qui expriment un TCR, ou une partie de liaison à l'antigène de celui-ci, ayant une spécificité antigénique pour une séquence d'acides aminés de p53 humaine mutée codée par une mutation de p53 spécifique au cancer, le procédé comprenant :
l'isolement d'un TCR, ou d'une partie de liaison à l'antigène de celui-ci, selon le procédé de la revendication 10, et
l'introduction *in vitro* de la séquence de nucléotides codant le TCR isolé, ou de la partie de liaison à l'antigène de celui-ci, dans des cellules mononucléées du sang périphérique (PBMC) pour obtenir des cellules qui expriment le TCR, ou la partie de liaison à l'antigène de celui-ci.

12. Procédé de préparation d'une population de cellules T qui ont une spécificité antigénique pour une séquence d'acides aminés de p53 humaine mutée codée par une mutation de p53 spécifique au cancer, le procédé comprenant :
l'isolement de cellules T selon le procédé de l'une quelconque des revendications 1 à 9, et
l'expansion des nombres de cellules T autologues sélectionnées pour obtenir une population de cellules T qui ont une spécificité antigénique pour la séquence d'acides aminés de p53 humaine mutée codée par la mutation de p53 spécifique au cancer.

13. Le procédé de la revendication 12, sachant que l'expansion des nombres de cellules comprend la mise en culture des cellules sélectionnées avec des PBMC nourrissantes, l'interleukine (IL)-2, et l'anticorps OKT3.
